# EUROPEAN PATENT APPLICATION

(11) **EP 3 882 270 A2**
(43) Date of publication of application: **22.09.2021**
(21) Application number: 21164058.6
(22) Date of filing: 16.08.2018
(51) Int. Cl.: C07K 16/28, C07K 14/705, C07K 16/30, A61K 47/68

(54) **PROTEINS BINDING NKG2D, CD16, AND EGFR, CCR4, OR PD-L1**

(30) Priority: 16.08.2017 US 201762546297 P; 16.08.2017 US 201762546300 P; 30.08.2017 US 201762552152 P; 07.09.2017 US 201762555114 P
(62) Divisional of application: 18846836.7
(71) Applicant: Dragonfly Therapeutics, Inc., Waltham, MA 02451 (US)
(72) Inventor: CHANG, Gregory, P., Medford, MA 02155 (US); CHEUNG, Ann, F., Lincoln, MA 01773 (US); DU, Jinyan, Waltham, MA 02452 (US); GRINBERG, Asya, Lexington, MA 02421 (US); HANEY, William, Wayland, MA 01778 (US); SETHI, Dhruv, Kam, Belmont, MA 02478 (US); WAGTMANN, Nicolai, Concord, MA 01742 (US); LUNDE, Bradley, M., Lebanon, NH 03766 (US); PRINZ, Bianka, Lebanon, NH 03766 (US)
(74) Representative: Haseltine Lake Kempner LLP

(57) **Abstract**

Multi-specific binding proteins that bind NKG2D receptor, CD16, and a tumour-associated antigen selected from EGFR, CCR4, and PD-L1 are described, as well as pharmaceutical compositions and therapeutic methods useful for the treatment of cancer.

## Description

### CROSS-REFERENCE TO RELATED APPLICATIONS

This application claims the benefit of and priority to U.S. Provisional Patent Application No. 62/546,300, filed August 16, 2017; U.S. Provisional Patent Application No. 62/546,297, filed August 16, 2017; U.S. Provisional Patent Application No. 62/552,152, filed August 30, 2017; and U.S. Provisional Patent Application No. 62/555,114, filed September 7, 2017, the content of each of which is hereby incorporated by reference in its entirety for all purposes.

### SEQUENCE LISTING

The instant application contains a Sequence Listing which has been submitted electronically in ASCII format and is hereby incorporated by reference in its entirety. Said ASCII copy, created on August 15, 2018, is named DFY-033WO_SL.txt and is 214,413 bytes in size.

### FIELD OF THE INVENTION

The invention relates to multi-specific binding proteins that bind to NKG2D, CD16, and a tumor-associated antigen selected from EGFR, HLA-E, CCR4, and PD-L1.

### BACKGROUND

Cancer continues to be a significant health problem despite the substantial research efforts and scientific advances reported in the literature for treating this disease. Blood and bone marrow cancers are frequently diagnosed cancer types, including multiple myelomas, leukemia, and lymphomas. Current treatment options for these cancers are not effective for all patients and/or can have substantial adverse side effects. Other types of cancer also remain challenging to treat using existing therapeutic options.

Cancer immunotherapies are desirable because they are highly specific and can facilitate destruction of cancer cells using the patient's own immune system. Fusion proteins such as bi-specific T-cell engagers are cancer immunotherapies described in the literature that bind to tumor cells and T-cells to facilitate destruction of tumor cells. Antibodies that bind to certain tumor-associated antigens and to certain immune cells have been described in the literature. *See, e.g.,* WO 2016/134371 and WO 2015/095412.

Natural killer (NK) cells are a component of the innate immune system and make up approximately 15% of circulating lymphocytes. NK cells infiltrate virtually all tissues and were originally characterized by their ability to kill tumor cells effectively without the need for prior sensitization. Activated NK cells kill target cells by means similar to cytotoxic T cells - *i.e.,* via cytolytic granules that contain perforin and granzymes as well as via death receptor pathways. Activated NK cells also secrete inflammatory cytokines such as IFN-γ and chemokines that promote the recruitment of other leukocytes to the target tissue.

NK cells respond to signals through a variety of activating and inhibitory receptors on their surface. For example, when NK cells encounter healthy self-cells, their activity is inhibited through activation of the killer-cell immunoglobulin-like receptors (KIRs). Alternatively, when NK cells encounter foreign cells or cancer cells, they are activated via their activating receptors (*e.g*., NKG2D, NCRs, DNAM1). NK cells are also activated by the constant region of some immunoglobulins through CD16 receptors on their surface. The overall sensitivity of NK cells to activation depends on the sum of stimulatory and inhibitory signals.

The epidermal growth factor receptor (EGFR; ErbB-1; HER1) is a transmembrane protein that is a receptor for members of the epidermal growth factor family (EGF family) of extracellular protein ligands. Upon binding of its specific ligands, including epidermal growth factor and transforming growth factor α (TGFα), EGFR undergoes a transition from an inactive monomeric form to an active homodimer or heterodimer with other ErbB family receptors. The dimerization stimulates its intrinsic intracellular protein-tyrosine kinase activity, and elicits downstream signaling cascades, leading to DNA synthesis and cell proliferation. EGFR is involved in modulation of phenotypes such as cell migration, adhesion, and proliferation.

Mutations that lead to EGFR overexpression or overactivity have been associated with a number of cancers, including non-small cell lung cancer, anal cancers, glioblastoma and epithelial tumors of the head and neck. These somatic mutations involving EGFR lead to its constant activation, which produces uncontrolled cell division. In glioblastoma a more or less specific mutation of EGFR, called EGFRvIII is often observed. Mutations, amplifications or misregulations of EGFR or family members are implicated in other solid tumors, including colorectal cancer, renal cell carcinoma, bladder cancer, cervical cancer, ovarian cancer, pancreatic cancer, and liver cancer.

The immune system plays an important role in tumorigenesis, and evasion of immune surveillance has become one of the important hallmarks of cancer. HLA-E is a non-classical major histocompatibility complex (MHC) molecule. It belongs to non-classical HLA-class Ib family that also includes HLA-G, HLA-F and HLA-H. The function of HLA-E is to bind peptides derived from the leader sequence of HLA-class I molecules (HLA-A, -B, - C, and -G) and to present them to NK cells through the interaction with the inhibitory receptor CD94/NKG2A, thus inhibiting NK cell lysis against cells that express normal levels of HLA-class I molecules. This mechanism has been used by many cancers to escape immune surveillance, including lymphoma, head and neck cancer, bladder cancer, cervical cancer, lung cancer, renal cancer, melanoma, colorectal cancer, ovarian cancer, glioblastoma and sarcomas.

CCR4 is a C-C type chemokine receptor for CC chemokines, which includes CCL2, CCL4, CCL5, CCL17 and CCL22. Chemokines are a group of small structurally related proteins that regulate cell trafficking of various types of leukocytes, and play fundamental roles in the development, homeostasis, and function of the immune system. In addition, CCR4 has been shown to be expressed in several types of malignancies including adult T-cell lymphoma/leukemia (ATLL), peripheral T cell lymphoma, cutaneous T cell lymphoma, chronic lymphocytic leukemia, B cell malignancies, non-Hodgkin's lymphoma, Hodgkin's lymphoma, anaplastic large cell lymphoma, mature T/natural killer (NK) cell neoplasms, thymoma, gastric cancer, and renal cell carcinoma.

Programmed death-ligand 1 (PD-L1) plays an important role in maintaining immune homeostasis. It binds to PD-1 receptor on T cells, and downregulates cytotoxic T-cell, thereby protecting normal cells from collateral damage. Development and progression of tumor are accompanied by the formation of special tumor immune microenvironment. Tumor cells can escape the immune surveillance and disrupt immune checkpoint of host by overexpressing PD-L1. When PD-L1 binds to PD-1, an inhibitory signal is transmitted into the T cell, which reduces cytokine production and suppresses T-cell proliferation. Tumor cells exploit this immune-checkpoint pathway as a mechanism to evade detection and inhibit the immune response. PD-L1 is over-expressed in various types of cancers, especially in lymphoma, leukemia, multiple myeloma, head and neck cancer, bladder cancer, cervical cancer, lung cancer, renal cancer, melanoma, colorectal cancer, ovarian cancer, glioblastoma, sarcomas, and gastric cancer.

### SUMMARY

The invention provides multi-specific binding proteins that bind to the NKG2D receptor and CD16 receptor on natural killer cells, and a tumor-associated antigen selected from EGFR, HLA-E, CCR4, and PD-L1. Such proteins can engage more than one kind of NK-activating receptor, and may block the binding of natural ligands to NKG2D. In certain embodiments, the proteins can agonize NK cells in humans. In some embodiments, the proteins can agonize NK cells in humans and in other species such as rodents and cynomolgus monkeys. Various aspects and embodiments of the invention are described in further detail below.

Accordingly, one aspect of the invention provides a protein that incorporates a first antigen-binding site that binds NKG2D; a second antigen-binding site that binds a tumor-associated antigen selected from EGFR, HLA-E, CCR4, and PD-L1; and an antibody fragment crystallizable (Fc) domain, a portion thereof sufficient to bind CD 16, or a third antigen-binding site that binds CD16. In some embodiments, the first antigen-binding site binds to NKG2D in humans.

The antigen-binding sites may each incorporate an antibody heavy chain variable domain and an antibody light chain variable domain (*e.g*., arranged as in an antibody, or fused together to from a single-chain variable-fragment (scFv)), or one or more of the antigen-binding sites may be a single-domain antibody, such as a V_{H}H antibody like a camelid antibody or a V_{NAR} antibody like those found in cartilaginous fish. For example, the first antigen-binding site that binds NKG2D includes an antibody heavy chain variable domain and an antibody light chain variable domain. In some embodiments the second antigen-binding site that binds a tumor-associated antigen selected from EGFR, HLA-E, CCR4, and PD-L1 includes an antibody heavy chain variable domain and an antibody light chain variable domain. In some embodiments the third antigen-binding site that binds CD16 includes an antibody heavy chain variable domain and an antibody light chain variable domain. In some embodiments, two or more of the first antigen-binding site, the second antigen-binding site, and the third antigen-binding site include an antibody heavy chain variable domain and an antibody light chain variable domain.

In some embodiments, the first antigen-binding site that binds NKG2D is a single-domain antibody, for example, a V_{H}H fragment or a V_{NAR} fragment. In some embodiments, the second antigen-binding site that binds a tumor-associated antigen selected from EGFR, HLA-E, CCR4, and PD-L1 is a single-domain antibody, for example, a V_{H}H fragment or a V_{NAR} fragment. In some embodiments the third antigen-binding site that binds CD16 is a single-domain antibody, for example, a V_{H}H fragment or a V_{NAR} fragment. In some embodiments, two or more of the first antigen-binding site, the second antigen-binding site, and the third antigen-binding site are a single-domain antibody, for example, a V_{H}H fragment or a V_{NAR} fragment.

In some embodiments an antibody heavy chain variable domain and an antibody light chain variable domain are present on the same polypeptide. For example, in some embodiments the first antigen-binding site that binds NKG2D includes an antibody heavy chain variable domain and an antibody light chain variable domain present on the same polypeptide. In some embodiments the second antigen-binding site that binds a tumor-associated antigen selected from EGFR, HLA-E, CCR4, and PD-L1 includes an antibody heavy chain variable domain and an antibody light chain variable domain present on the same polypeptide. In some embodiments the third antigen-binding site that binds CD16 includes an antibody heavy chain variable domain and an antibody light chain variable domain present on the same polypeptide. In some embodiments, two or more of the first antigen-binding site, the second antigen-binding site, and the third antigen-binding site include an antibody heavy chain variable domain and an antibody light chain variable domain present on the same polypeptide.

In one aspect, the invention provides a protein comprising (a) a first antigen-binding site comprising an Fab fragment that binds NKG2D; (b) a second antigen-binding site comprising a single-chain variable fragment (scFv) that binds EGFR; and (c) an antibody Fc domain or a portion thereof sufficient to bind CD16, or a third antigen-binding site that binds CD16. The present invention provides a protein in which the first antigen-binding site that binds NKG2D is an Fab fragment, and the second antigen-binding site that binds a tumor-associated antigen EGFR is an scFv.

Certain proteins described in the present disclosure include an scFv, comprising a heavy chain variable domain and a light chain variable domain, linked to an antibody Fc domain or a portion thereof sufficient to bind CD 16, or the third antigen-binding site that binds CD16, via a hinge comprising Ala-Ser. Some proteins of the present disclosure includes an scFv linked to an antibody Fc domain. Some proteins of the present disclosure includes a heavy chain variable domain of an scFv, which forms a disulfide bridge with the light chain variable domain of the scFv.

Some proteins of the present disclosure include an scFv fragment, in which a disulfide bridge is formed between C44 from the heavy chain variable domain and C100 from the light chain variable domain.

Some proteins of the present disclosure include an scFv linked to an antibody Fc domain, in which the light chain variable domain of the scFv is positioned at the N-terminus of the heavy chain variable domain of the scFv, and is linked to the heavy chain variable domain of the scFv via a flexible linker (GlyGlyGlyGlySer)₄ (G4S)₄) (SEQ ID NO:263), and the Fab is linked to the antibody Fc domain.

Some proteins of the present disclosure include a heavy chain variable domain of an scFv linked to the light chain variable domain of the scFv via a flexible linker, *e.g.,* (GlyGlyGlyGlySer)₄ ((G4S)₄) linker.

Some proteins of the present disclosure include an scFv in which the heavy chain variable domain is positioned at the N-terminus or the C-terminus of the light chain variable domain of the scFv.

Some proteins of the present disclosure include an scFv in which the light chain variable domain is positioned at the N-terminus of the heavy chain variable domain of the scFv.

Some proteins of the present disclosure include an Fab fragment linked to the antibody Fc domain or a portion thereof sufficient to bind CD 16, or the third antigen-binding site that binds CD16.

Some proteins of the present disclosure include an Fab fragment, wherein the heavy chain portion of the Fab fragment comprises a heavy chain variable domain and a CH1 domain, and wherein the heavy chain variable domain is linked to the CH1 domain.

Some proteins of the present disclosure include an Fab linked to the antibody Fc domain.

Some proteins of the present disclosure include a sequence selected from SEQ ID NO:264, SEQ ID NO:265, and SEQ ID NO:266.

Some proteins of the present disclosure include an scFv linked to an antibody Fc domain, wherein the scFv linked to the antibody Fc domain is represented by a sequence selected from SEQ ID NO:267, SEQ ID NO:268, and SEQ ID NO:269.

Some proteins of the present disclosure include a sequence of SEQ ID NO:270, SEQ and SEQ ID NO:271.

Some proteins of the present disclosure include a sequence at least 90% identical to an amino acid sequence selected from SEQ ID NO:264, SEQ ID NO:265, and SEQ ID NO:266.

Some proteins of the present disclosure include a sequence at least 95% identical to an amino acid sequence selected from SEQ ID NO:264, SEQ ID NO:265, and SEQ ID NO:266.

Some proteins of the present disclosure include a sequence at least 99% identical to an amino acid sequence selected from SEQ ID NO:264, SEQ ID NO:265, and SEQ ID NO:266.

Some proteins of the present disclosure include a sequence at least 90% identical to an amino acid sequence selected from SEQ ID NO:267, SEQ ID NO:268, and SEQ ID NO:269.

Some proteins of the present disclosure include a sequence at least 95% identical to an amino acid sequence selected from SEQ ID NO:267, SEQ ID NO:268, and SEQ ID NO:269.

Some proteins of the present disclosure include a sequence at least 99% identical to an amino acid sequence selected from SEQ ID NO:267, SEQ ID NO:268, and SEQ ID NO:269.

In one aspect, the present invention provides multi-specific binding proteins that bind to the NKG2D receptor and CD16 receptor on natural killer cells, and a tumor-associated antigen selected from EGFR, HLA-E, CCR4, and PD-L1. The first antigen-binding site that binds to NKG2D includes a heavy chain variable domain at least 90% identical to an amino acid sequence selected from the amino acid sequence of: SEQ ID NO:1, SEQ ID NO:41, SEQ ID NO:49, SEQ ID NO:57, SEQ ID NO:59, SEQ ID NO:61, SEQ ID NO:69, SEQ ID NO:77, SEQ ID NO:85, and SEQ ID NO:93.

The first antigen-binding site, which binds to NKG2D, in some embodiments, can incorporate a heavy chain variable domain related to SEQ ID NO:1, such as by having an amino acid sequence at least 90% *(e.g.,* 90%, 91%, 92%, 93%, 94%, 95%, 96%, 97%, 98%, 99%, or 100%) identical to SEQ ID NO:1, and/or incorporating amino acid sequences identical to the CDR1 (SEQ ID NO: 105), CDR2 (SEQ ID NO: 106), and CDR3 (SEQ ID NO: 107) sequences of SEQ ID NO:1. The heavy chain variable domain related to SEQ ID NO: 1 can be coupled with a variety of light chain variable domains to form an NKG2D binding site. For example, the first antigen-binding site that incorporates a heavy chain variable domain related to SEQ ID NO: 1 can further incorporate a light chain variable domain selected from any one of the sequences related to SEQ ID NOs:2, 4, 6, 8, 10, 12, 14, 16, 18, 20, 22, 24, 26, 28, 30, 32, 34, 36, 38, and 40. For example, the first antigen-binding site incorporates a heavy chain variable domain with amino acid sequences at least 90% (*e.g.,* 90%, 91%, 92%, 93%, 94%, 95%, 96%, 97%, 98%, 99%, or 100%) identical to SEQ ID NO: 1 and a light chain variable domain with amino acid sequences at least 90% (*e.g.,* 90%, 91%, 92%, 93%, 94%, 95%, 96%, 97%, 98%, 99%, or 100%) identical to any one of the sequences selected from SEQ ID NOs:2, 4, 6, 8, 10, 12, 14, 16, 18, 20, 22, 24, 26, 28, 30, 32, 34, 36, 38, and 40.

Alternatively, the first antigen-binding site can incorporate a heavy chain variable domain related to SEQ ID NO:41 and a light chain variable domain related to SEQ ID NO:42. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:41, and/or incorporate amino acid sequences identical to the CDR1 (SEQ ID NO:43), CDR2 (SEQ ID NO:44), and CDR3 (SEQ ID NO:45) sequences of SEQ ID NO:41. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:42, and/or incorporate amino acid sequences identical to the CDR1 (SEQ ID NO:46), CDR2 (SEQ ID NO:47), and CDR3 (SEQ ID NO:48) sequences of SEQ ID NO:42.

In other embodiments, the first antigen-binding site can incorporate a heavy chain variable domain related to SEQ ID NO:49 and a light chain variable domain related to SEQ ID NO:50. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:49, and/or incorporate amino acid sequences identical to the CDR1 (SEQ ID NO:51), CDR2 (SEQ ID NO:52), and CDR3 (SEQ ID NO:53) sequences of SEQ ID NO:49. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:50, and/or incorporate amino acid sequences identical to the CDR1 (SEQ ID NO:54), CDR2 (SEQ ID NO:55), and CDR3 (SEQ ID NO:56) sequences of SEQ ID NO:50.

Alternatively, the first antigen-binding site can incorporate a heavy chain variable domain related to SEQ ID NO:57 and a light chain variable domain related to SEQ ID NO:58, such as by having amino acid sequences at least 90% *(e.g.,* 90%, 91%, 92%, 93%, 94%, 95%, 96%, 97%, 98%, 99%, or 100%) identical to SEQ ID NO:57 and at least 90% (*e.g.,* 90%, 91%, 92%, 93%, 94%, 95%, 96%, 97%, 98%, 99%, or 100%) identical to SEQ ID NO:58, respectively.

In another embodiment, the first antigen-binding site can incorporate a heavy chain variable domain related to SEQ ID NO:59 and a light chain variable domain related to SEQ ID NO:60, For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:59, and/or incorporate amino acid sequences identical to the CDR1 (SEQ ID NO: 134), CDR2 (SEQ ID NO: 135), and CDR3 (SEQ ID NO:136) sequences of SEQ ID NO:59. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:60, and/or incorporate amino acid sequences identical to the CDR1 (SEQ ID NO: 137), CDR2 (SEQ ID NO: 138), and CDR3 (SEQ ID NO:139) sequences of SEQ ID NO:60.

The first antigen-binding site, which binds to NKG2D, in some embodiments, can incorporate a heavy chain variable domain related to SEQ ID NO:61 and a light chain variable domain related to SEQ ID NO:62. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:61, and/or incorporate amino acid sequences identical to the CDR1 (SEQ ID NO:63), CDR2 (SEQ ID NO:64), and CDR3 (SEQ ID NO:65) sequences of SEQ ID NO:61. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:62, and/or incorporate amino acid sequences identical to the CDR1 (SEQ ID NO:66), CDR2 (SEQ ID NO:67), and CDR3 (SEQ ID NO:68) sequences of SEQ ID NO:62.

In some embodiments, the first antigen-binding site can incorporate a heavy chain variable domain related to SEQ ID NO:69 and a light chain variable domain related to SEQ ID NO:70. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:69, and/or incorporate amino acid sequences identical to the CDR1 (SEQ ID NO:71), CDR2 (SEQ ID NO:72), and CDR3 (SEQ ID NO:73) sequences of SEQ ID NO:69. Similarly, the light chain variable domain of the second antigen-binding site can be at least 90% (*e.g*., 90%, 91%, 92%, 93%, 94%, 95%, 96%, 97%, 98%, 99%, or 100%) identical to SEQ ID NO:70, and/or incorporate amino acid sequences identical to the CDR1 (SEQ ID NO:74), CDR2 (SEQ ID NO:75), and CDR3 (SEQ ID NO:76) sequences of SEQ ID NO:70.

In some embodiments, the first antigen-binding site can incorporate a heavy chain variable domain related to SEQ ID NO:77 and a light chain variable domain related to SEQ ID NO:78. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:77, and/or incorporate amino acid sequences identical to the CDR1 (SEQ ID NO:79), CDR2 (SEQ ID NO:80), and CDR3 (SEQ ID NO:81) sequences of SEQ ID NO:77. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:78, and/or incorporate amino acid sequences identical to the CDR1 (SEQ ID NO:82), CDR2 (SEQ ID NO:83), and CDR3 (SEQ ID NO:84) sequences of SEQ ID NO:78.

In some embodiments, the first antigen-binding site can incorporate a heavy chain variable domain related to SEQ ID NO:85 and a light chain variable domain related to SEQ ID NO:86. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:85, and/or incorporate amino acid sequences identical to the CDR1 (SEQ ID NO:87), CDR2 (SEQ ID NO:88), and CDR3 (SEQ ID NO:89) sequences of SEQ ID NO:85. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:86, and/or incorporate amino acid sequences identical to the CDR1 (SEQ ID NO:90), CDR2 (SEQ ID NO:91), and CDR3 (SEQ ID NO:92) sequences of SEQ ID NO:86.

In some embodiments, the first antigen-binding site can incorporate a heavy chain variable domain related to SEQ ID NO:93 and a light chain variable domain related to SEQ ID NO:94. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:93, and/or incorporate amino acid sequences identical to the CDR1 (SEQ ID NO:95), CDR2 (SEQ ID NO:96), and CDR3 (SEQ ID NO:97) sequences of SEQ ID NO:93. Similarly, the light chain variable domain of the second antigen-binding site can be at least 90% (*e.g*., 90%, 91%, 92%, 93%, 94%, 95%, 96%, 97%, 98%, 99%, or 100%) identical to SEQ ID NO:94, and/or incorporate amino acid sequences identical to the CDR1 (SEQ ID NO:98), CDR2 (SEQ ID NO:99), and CDR3 (SEQ ID NO:100) sequences of SEQ ID NO:94.

In some embodiments, the first antigen-binding site can incorporate a heavy chain variable domain related to SEQ ID NO:101 and a light chain variable domain related to SEQ ID NO:102, such as by having amino acid sequences at least 90% *(e.g.,* 90%, 91%, 92%, 93%, 94%, 95%, 96%, 97%, 98%, 99%, or 100%) identical to SEQ ID NO:101 and at least 90% *(e.g.,* 90%, 91%, 92%, 93%, 94%, 95%, 96%, 97%, 98%, 99%, or 100%) identical to SEQ ID NO:102, respectively.

In some embodiments, the first antigen-binding site can incorporate a heavy chain variable domain related to SEQ ID NO:103 and a light chain variable domain related to SEQ ID NO:104, such as by having amino acid sequences at least 90% *(e.g.,* 90%, 91%, 92%, 93%, 94%, 95%, 96%, 97%, 98%, 99%, or 100%) identical to SEQ ID NO:103 and at least 90% *(e.g.,* 90%, 91%, 92%, 93%, 94%, 95%, 96%, 97%, 98%, 99%, or 100%) identical to SEQ ID NO:104, respectively.

In some embodiments, the second antigen-binding site can bind to EGFR and can incorporate a heavy chain variable domain related to SEQ ID NO:217 and a light chain variable domain related to SEQ ID NO:109. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:217, and/or incorporate amino acid sequences identical to the CDR1 (SEQ ID NO:218), CDR2 (SEQ ID NO:219), and CDR3 (SEQ ID NO:220) sequences of SEQ ID NO:217. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:109, and/or incorporate amino acid sequences identical to the CDR1 (SEQ ID NO:110), CDR2 (SEQ ID NO:111), and CDR3 (SEQ ID NO: 112) sequences of SEQ ID NO:109.

Alternatively, the second antigen-binding site can bind to EGFR and can incorporate a heavy chain variable domain related to SEQ ID NO:113 and a light chain variable domain related to SEQ ID NO:117. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:113, and/or incorporate amino acid sequences identical to the CDR1 (SEQ ID NO:114), CDR2 (SEQ ID NO:115), and CDR3 (SEQ ID NO:116) sequences of SEQ ID NO:113. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:117, and/or incorporate amino acid sequences identical to the CDR1 (SEQ ID NO:118), CDR2 (SEQ ID NO:119), and CDR3 (SEQ ID NO:120) sequences of SEQ ID NO:117.

Alternatively, the second antigen-binding site can bind to EGFR and can incorporate a heavy chain variable domain related to SEQ ID NO:121 and a light chain variable domain related to SEQ ID NO:125. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:121, and/or incorporate amino acid sequences identical to the CDR1 (SEQ ID NO:122), CDR2 (SEQ ID NO:123), and CDR3 (SEQ ID NO:124) sequences of SEQ ID NO:121. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:125, and/or incorporate amino acid sequences identical to the CDR1 (SEQ ID NO:126), CDR2 (SEQ ID NO:127), and CDR3 (SEQ ID NO:128) sequences of SEQ ID NO:125.

Alternatively, the second antigen-binding site can bind to EGFR and can incorporate a heavy chain variable domain related to SEQ ID NO:129 and a light chain variable domain related to SEQ ID NO:133. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:129, and/or incorporate amino acid sequences identical to the CDR1 (SEQ ID NO:130), CDR2 (SEQ ID NO:131), and CDR3 (SEQ ID NO:132) sequences of SEQ ID NO:129. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:133, and/or incorporate amino acid sequences identical to the CDR1 (SEQ ID NO:140), CDR2 (SEQ ID NO:141), and CDR3 (SEQ ID NO:142) sequences of SEQ ID NO:133.

Alternatively, the second antigen-binding site can bind to EGFR and can incorporate a heavy chain variable domain related to SEQ ID NO:143 and a light chain variable domain related to SEQ ID NO:147. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:143, and/or incorporate amino acid sequences identical to the CDR1 (SEQ ID NO: 144), CDR2 (SEQ ID NO:145), and CDR3 (SEQ ID NO: 146) sequences of SEQ ID NO: 143. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 147, and/or incorporate amino acid sequences identical to the CDR1 (SEQ ID NO: 148), CDR2 (SEQ ID NO: 149), and CDR3 (SEQ ID NO: 150) sequences of SEQ ID NO: 147.

Alternatively, the second antigen-binding site can bind to EGFR and can incorporate a heavy chain variable related to SEQ ID NO: 151 and a light chain variable domain related to SEQ ID NO:152. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:151, and the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 152.

Alternatively, the second antigen-binding site can bind to EGFR and can incorporate a heavy chain variable related to SEQ ID NO: 153 and a light chain variable domain related to SEQ ID NO:154. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 153, and/or incorporate amino acid sequences identical to the CDR1 (SEQ ID NO:227), CDR2 (SEQ ID NO:228), and CDR3 (SEQ ID NO:229) sequences of SEQ ID NO: 153. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 154, and/or incorporate amino acid sequences identical to the CDR1 (SEQ ID NO:230), CDR2 (SEQ ID NO:231), and CDR3 (SEQ ID NO:232) sequences of SEQ ID NO:154.

Alternatively, the second antigen-binding site can bind to EGFR and can incorporate a heavy chain variable related to SEQ ID NO: 155 and a light chain variable domain related to SEQ ID NO:156. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 155, and/or incorporate amino acid sequences identical to the CDR1 (SEQ ID NO:233), CDR2 (SEQ ID NO:234), and CDR3 (SEQ ID NO:235) sequences of SEQ ID NO:155. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 156, and/or incorporate amino acid sequences identical to the CDR1 (SEQ ID NO:236), CDR2 (SEQ ID NO:237), and CDR3 (SEQ ID NO:238) sequences of SEQ ID NO:156.

Alternatively, the second antigen-binding site can bind to EGFR and can incorporate a heavy chain variable related to SEQ ID NO: 157 and a light chain variable domain related to SEQ ID NO:158. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 157, and/or incorporate amino acid sequences identical to the CDR1 (SEQ ID NO:239), CDR2 (SEQ ID NO:240), and CDR3 (SEQ ID NO:241) sequences of SEQ ID NO:157. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 158, and/or incorporate amino acid sequences identical to the CDR1 (SEQ ID NO:242), CDR2 (SEQ ID NO:243), and CDR3 (SEQ ID NO:244) sequences of SEQ ID NO:158.

Alternatively, the second antigen-binding site can bind to EGFR and can incorporate a heavy chain variable related to SEQ ID NO: 159 and a light chain variable domain related to SEQ ID NO:160. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 159, and/or incorporate amino acid sequences identical to the CDR1 (SEQ ID NO:245), CDR2 (SEQ ID NO:246), and CDR3 (SEQ ID NO:247) sequences of SEQ ID NO: 159. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 160, and/or incorporate amino acid sequences identical to the CDR1 (SEQ ID NO:248), CDR2 (SEQ ID NO:249), and CDR3 (SEQ ID NO:250) sequences of SEQ ID NO:160.

Alternatively, the second antigen-binding site can bind to EGFR and can incorporate a heavy chain variable related to SEQ ID NO: 161 and a light chain variable domain related to SEQ ID NO:162. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 161, and/or incorporate amino acid sequences identical to the CDR1 (SEQ ID NO:251), CDR2 (SEQ ID NO:252), and CDR3 (SEQ ID NO:253) sequences of SEQ ID NO:161. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 162, and/or incorporate amino acid sequences identical to the CDR1 (SEQ ID NO:254), CDR2 (SEQ ID NO:255), and CDR3 (SEQ ID NO:256) sequences of SEQ ID NO:162.

Alternatively, the second antigen-binding site can bind to EGFR and can incorporate a heavy chain variable related to SEQ ID NO: 163 and a light chain variable domain related to SEQ ID NO:164. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 163, and/or incorporate amino acid sequences identical to the CDR1 (SEQ ID NO:257), CDR2 (SEQ ID NO:258), and CDR3 (SEQ ID NO:259) sequences of SEQ ID NO: 163. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 164, and/or incorporate amino acid sequences identical to the CDR1 (SEQ ID NO:260), CDR2 (SEQ ID NO:261), and CDR3 (SEQ ID NO:262) sequences of SEQ ID NO:164.

Alternatively, the second antigen-binding site can bind to PD-L1 and can incorporate a heavy chain variable related to SEQ ID NO: 167 and a light chain variable domain related to SEQ ID NO:171. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 167, and/or incorporate amino acid sequences identical to the CDR1 (SEQ ID NO: 168), CDR2 (SEQ ID NO: 169), and CDR3 (SEQ ID NO: 170) sequences of SEQ ID NO: 167. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 171, and/or incorporate amino acid sequences identical to the CDR1 (SEQ ID NO: 172), CDR2 (SEQ ID NO: 173), and CDR3 (SEQ ID NO: 174) sequences of SEQ ID NO:171.

Alternatively, the second antigen-binding site can bind to PD-L1 and can incorporate a heavy chain variable related to SEQ ID NO: 175 and a light chain variable domain related to SEQ ID NO:179. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 175, and/or incorporate amino acid sequences identical to the CDR1 (SEQ ID NO: 176), CDR2 (SEQ ID NO: 177), and CDR3 (SEQ ID NO: 178) sequences of SEQ ID NO: 175. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 179, and/or incorporate amino acid sequences identical to the CDR1 (SEQ ID NO: 180), CDR2 (SEQ ID NO: 181), and CDR3 (SEQ ID NO: 182) sequences of SEQ ID NO:179.

Alternatively, the second antigen-binding site can bind to PD-L1 and can incorporate a heavy chain variable related to SEQ ID NO: 183 and a light chain variable domain related to SEQ ID NO:187. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 183, and/or incorporate amino acid sequences identical to the CDR1 (SEQ ID NO: 184), CDR2 (SEQ ID NO: 185), and CDR3 (SEQ ID NO: 186) sequences of SEQ ID NO: 183. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 187, and/or incorporate amino acid sequences identical to the CDR1 (SEQ ID NO: 188), CDR2 (SEQ ID NO: 189), and CDR3 (SEQ ID NO: 190) sequences of SEQ ID NO:187.

Alternatively, the second antigen-binding site can bind to CCR4 and can incorporate a heavy chain variable related to SEQ ID NO: 192 and a light chain variable domain related to SEQ ID NO:196. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 192, and/or incorporate amino acid sequences identical to the CDR1 (SEQ ID NO: 193), CDR2 (SEQ ID NO: 194), and CDR3 (SEQ ID NO: 195) sequences of SEQ ID NO: 192. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 196, and/or incorporate amino acid sequences identical to the CDR1 (SEQ ID NO: 197), CDR2 (SEQ ID NO: 198), and CDR3 (SEQ ID NO: 199) sequences of SEQ ID NO:196.

Alternatively, the second antigen-binding site can bind to CCR4 and can incorporate a heavy chain variable related to SEQ ID NO:200 and a light chain variable domain related to SEQ ID NO:204. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:200, and/or incorporate amino acid sequences identical to the CDR1 (SEQ ID NO:201), CDR2 (SEQ ID NO:202), and CDR3 (SEQ ID NO:203) sequences of SEQ ID NO:200. Similarly, the light chain variable domain of the second antigen-binding site can be at least 90% (*e.g.,* 90%, 91%, 92%, 93%, 94%, 95%, 96%, 97%, 98%, 99%, or 100%) identical to SEQ ID NO:204, and/or incorporate amino acid sequences identical to the CDR1 (SEQ ID NO:205), CDR2 (SEQ ID NO:206), and CDR3 (SEQ ID NO:207) sequences of SEQ ID NO:204.

Alternatively, the second antigen-binding site can bind to CCR4 and can incorporate a heavy chain variable related to SEQ ID NO:208 and a light chain variable domain related to SEQ ID NO:212. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:208, and/or incorporate amino acid sequences identical to the CDR1 (SEQ ID NO:209), CDR2 (SEQ ID NO:210), and CDR3 (SEQ ID NO:211) sequences of SEQ ID NO:208. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:212, and/or incorporate amino acid sequences identical to the CDR1 (SEQ ID NO:213), CDR2 (SEQ ID NO:214), and CDR3 (SEQ ID NO:215) sequences of SEQ ID NO:212.

In some embodiments, the light chain variable domain of the first antigen-binding site includes an amino acid sequence identical to the amino acid sequence of the light chain variable domain of the second antigen-binding site. For example, in some embodiments, the light chain variable domain of the first antigen-binding site that binds NKGD2 includes an amino acid sequence identical to the amino acid sequence of the light chain variable domain of the second antigen-binding site that binds a tumor-associated antigen selected from EGFR, HLA-E, CCR4, and PD-L1.

In some embodiments, the protein incorporates a portion of an antibody Fc domain sufficient to bind CD16, wherein the antibody Fc domain comprises a hinge and a CH2 domain, for example, a hinge and a CH2 domain of a human IgG antibody. In some embodiments, the antibody Fc domain includes amino acid sequences at least 90% identical to amino acid sequence 234-332 of a human IgG antibody. In some embodiments, the antibody Fc domain includes an amino acid sequence at least 90% identical to the Fc domain of human IgG1 and the amino acid sequence of the antibody Fc domain differs at one or more positions selected from Q347, Y349, L351, S354, E356, E357, K360, Q362, S364, T366, L368, K370, N390, K392, T394, D399, S400, D401, F405, Y407, K409, T411, K439.

Formulations containing any one of the proteins described herein, cells containing one or more nucleic acids expressing the proteins, and methods of enhancing tumor cell death using the proteins are also provided. In some embodiments, the invention provides a formulation that includes a protein described herein and a pharmaceutically acceptable carrier. For example, in some embodiments, the formulation includes a protein that incorporates a first antigen-binding site that binds NKG2D; a second antigen-binding site that binds a tumor-associated antigen selected from EGFR, HLA-E, CCR4, and PD-L1; and an antibody Fc domain, a portion thereof sufficient to bind CD 16, or a third antigen-binding site that binds CD16, and a pharmaceutically acceptable carrier. In some embodiments, the invention provides a cell containing one or more nucleic acids that express a protein that incorporates a first antigen-binding site that binds NKG2D; a second antigen-binding site that binds a tumor-associated antigen selected from EGFR, HLA-E, CCR4, and PD-L1; and an antibody Fc domain, a portion thereof sufficient to bind CD 16, or a third antigen-binding site that binds CD16. In some embodiments, the invention provides a method of enhancing tumor cell death by exposing tumor cells and natural killer cells to an effective amount of a protein described herein, where the tumor cells express EGFR, HLA-E, CCR4, or PD-L1. For example, provided herein is a method of enhancing tumor cell death by exposing a tumor cell and a natural killer cell to an effective amount of a protein that incorporates a first antigen-binding site that binds NKG2D; a second antigen-binding site that binds a tumor-associated antigen selected from EGFR, HLA-E, CCR4, and PD-L1; and an antibody Fc domain, a portion thereof sufficient to bind CD16, or a third antigen-binding site that binds CD16, where the tumor cell expresses the tumor-associated antigen to which the second antigen-binding site of the protein binds *(e.g.,* EGFR, HLA-E, CCR4, or PD-L1).

Another aspect of the invention provides a method of treating cancer in a patient. The method comprises administering to a patient, for example, a patient in need thereof, a therapeutically effective amount of a multi-specific binding protein described herein or a formulation that includes a therapeutically effective amount of a multi-specific binding protein described herein. For example, in some embodiments, the method of treating cancer includes administering to a patient, for example, a patient in need of treatment, a formulation that includes a therapeutically effective amount of a multi-specific binding protein described herein and a pharmaceutically acceptable carrier.

In some embodiments, the method of treating cancer includes administering to a patient, for example, a patient in need of treatment, a therapeutically effective amount of a protein that incorporates a first antigen-binding site that binds NKG2D; a second antigen-binding site that binds a tumor-associated antigen selected from EGFR, HLA-E, CCR4, and PD-L1; and an antibody Fc domain, a portion thereof sufficient to bind CD 16, or a third antigen-binding site that binds CD16.

Exemplary cancers to be treated using the multi-specific binding proteins include adult T-cell lymphoma/leukemia, anaplastic large cell lymphoma, a B cell malignancy, bladder cancer, chronic lymphocytic leukemia, cervical cancer, colorectal cancer, cutaneous T cell lymphoma, gastric cancer, glioblastoma, glioma, head and neck cancer, Hodgkin's lymphoma, leukemia, liver cancer, lung cancer, lymphoma, a mature T/natural killer (NK) cell neoplasm, melanoma, multiple myeloma, non-Hodgkin's lymphoma, non-small cell lung cancer, ovarian cancer, pancreatic cancer, peripheral T cell lymphoma, renal cancer, renal cell carcinoma, a sarcoma, and thymoma. In some embodiments, the second antigen-binding site of the protein binds EGFR, and the cancer to be treated is head and neck cancer, colorectal cancer, non-small cell lung cancer, glioma, renal cell carcinoma, bladder cancer, cervical cancer, ovarian cancer, pancreatic cancer, or liver cancer. In some embodiments, the second antigen-binding site of the protein binds HLA-E, and the cancer to be treated is lymphoma, head and neck cancer, bladder cancer, cervical cancer, lung cancer, renal cancer, melanoma, colorectal cancer, ovarian cancer, glioblastoma, or a sarcoma. In some embodiments, the second antigen-binding site of the protein binds PD-L1, and the cancer to be treated is lymphoma, leukemia, multiple myeloma, head and neck cancer, bladder cancer, cervical cancer, lung cancer, renal cancer, melanoma, colorectal cancer, ovarian cancer, glioblastoma, a sarcoma, or gastric cancer. In some embodiments, the second antigen-binding site of the protein binds CCR4, and the cancer to be treated is adult T-cell lymphoma/leukemia, leukemia, peripheral T cell lymphoma, cutaneous T cell lymphoma, chronic lymphocytic leukemia, a B cell malignancy, non-Hodgkin's lymphoma, Hodgkin's lymphoma, anaplastic large cell lymphoma, a mature T/natural killer (NK) cell neoplasm, thymoma, gastric cancer, or renal cell carcinoma.

### BRIEF DESCRIPTION OF THE DRAWINGS

**FIG. 1** is a representation of a heterodimeric, multi-specific antibody. Each arm can represent either the NKG2D-binding domain, or the EGFR, HLA-E, CCR4, or PD-L1 binding domain. In some embodiments, the NKG2D- and the EGFR, HLA-E, CCR4, or PD-L1- binding domains can share a common light chain.
**FIG. 2A** is a representation of a heterodimeric, multi-specific antibody. Either the NKG2D-binding domain or the EGFR, HLA-E, CCR4, or PD-L1-binding domain can take the scFv format (right arm).

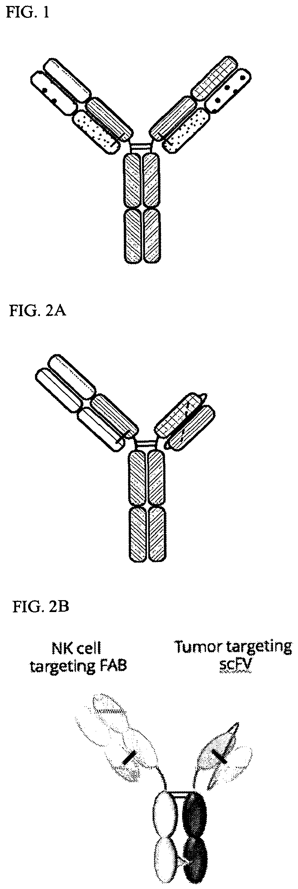

**FIG. 2B** illustrates a trispecific antibody (TriNKET) that contains an EGFR-binding scFv, a NKG2D-targeting Fab, and a heterodimerized antibody constant region/domain ("CD domain") that binds CD16 (scFv-Fab format). In an exemplary embodiment, the Fc domain linked to the Fab fragment comprises the mutations of K360E, K409W, and the Fc domain linked to the scFv comprises matching mutations Q347R, D399V, F405T for forming Fc heterodimer. The antibody format is referred herein as F3'-TriNKET. In another exemplary embodiment, the Fc domain linked to the Fab fragment comprises the mutations of Q347R, D399V, and F405T, and the Fc domain linked to the scFv comprises matching mutations K360E and K409W for forming a heterodimer.
**FIG. 3** are line graphs demonstrating the binding affinity of NKG2D-binding domains (listed as clones) to human recombinant NKG2D in an ELISA assay.
**FIG. 4** are line graphs demonstrating the binding affinity of NKG2D-binding domains (listed as clones) to cynomolgus recombinant NKG2D in an ELISA assay.
**FIG. 5** are line graphs demonstrating the binding affinity of NKG2D-binding domains (listed as clones) to mouse recombinant NKG2D in an ELISA assay.
**FIG. 6** are bar graphs demonstrating the binding of NKG2D-binding domains (listed as clones) to EL4 cells expressing human NKG2D by flow cytometry showing mean fluorescence intensity (MFI) fold over background (FOB).
**FIG. 7** are bar graphs demonstrating the binding of NKG2D-binding domains (listed as clones) to EL4 cells expressing mouse NKG2D by flow cytometry showing mean fluorescence intensity (MFI) fold over background (FOB).
**FIG. 8** are line graphs demonstrating specific binding affinity of NKG2D-binding domains (listed as clones) to recombinant human NKG2D-Fc by competing with natural ligand ULBP-6.
**FIG. 9** are line graphs demonstrating specific binding affinity of NKG2D-binding domains (listed as clones) to recombinant human NKG2D-Fc by competing with natural ligand MICA.
**FIG. 10** are line graphs demonstrating specific binding affinity of NKG2D-binding domains (listed as clones) to recombinant mouse NKG2D-Fc by competing with natural ligand Rae-1 delta.
**FIG. 11** are bar graphs showing activation of human NKG2D by NKG2D-binding domains (listed as clones) by quantifying the percentage of TNF-α positive cells, which express human NKG2D-CD3 zeta fusion proteins.
**FIG. 12** are bar graphs showing activation of mouse NKG2D by NKG2D-binding domains (listed as clones) by quantifying the percentage of TNF-α positive cells, which express mouse NKG2D-CD3 zeta fusion proteins.
**FIG. 13** are bar graphs showing activation of human NK cells by NKG2D-binding domains (listed as clones).
**FIG. 14** are bar graphs showing activation of human NK cells by NKG2D-binding domains (listed as clones).
**FIG. 15** are bar graphs showing activation of mouse NK cells by NKG2D-binding domains (listed as clones).
**FIG. 16** are bar graphs showing activation of mouse NK cells by NKG2D-binding domains (listed as clones).
**FIG. 17** are bar graphs showing the cytotoxic effect of NKG2D-binding domains (listed as clones) on tumor cells.
**FIG. 18** are bar graphs showing the melting temperature of NKG2D-binding domains (listed as clones) measured by differential scanning fluorimetry.
**FIGs. 19A-19C** are bar graphs of synergistic activation of NK cells using CD16 and NKG2D-binding. FIG. 19A demonstrates levels of CD107a; FIG. 19B demonstrates levels of IFN-γ; FIG. 19C demonstrates levels of CD107a and IFN-γ. Graphs indicate the mean (n = 2) ± SD. Data are representative of five independent experiments using five different healthy donors.

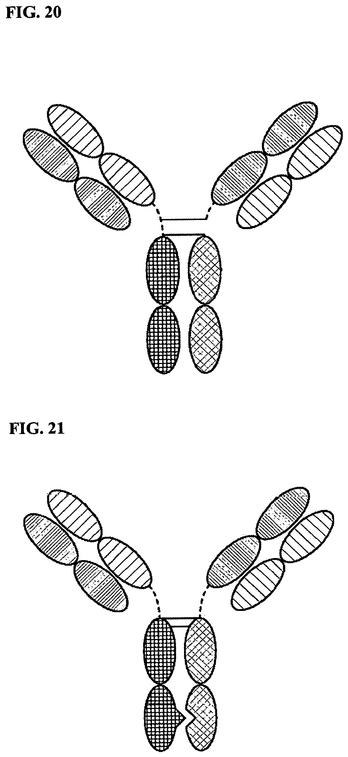

**FIG. 20** is a representation of a TriNKET in the Triomab form, which is a trifunctional, bispecific antibody that maintains an IgG-like shape. This chimera consists of two half antibodies, each with one light and one heavy chain, that originate from two parental antibodies. Triomab form may be a heterodimeric construct containing 1/2 of rat antibody and 1/2 of mouse antibody.
**FIG. 21** is a representation of a TriNKET in the KiH Common Light Chain form, which involves the knobs-into-holes (KIHs) technology. KiH is a heterodimer containing 2 Fab fragments binding to target 1 and 2, and an Fc stabilized by heterodimerization mutations. TriNKET in the KiH format may be a heterodimeric construct with 2 Fab fragments binding to target 1 and target 2, containing two different heavy chains and a common light chain that pairs with both heavy chains.

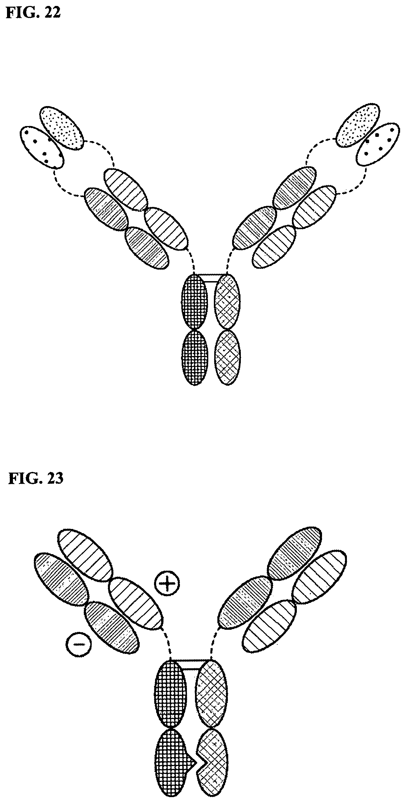

**FIG. 22** is a representation of a TriNKET in the dual-variable domain immunoglobulin (DVD-Ig™) form, which combines the target-binding domains of two monoclonal antibodies via flexible naturally occurring linkers, and yields a tetravalent IgG-like molecule. DVD-Ig™ is a homodimeric construct where variable domain targeting antigen 2 is fused to the N-terminus of a variable domain of Fab fragment targeting antigen 1. DVD-Ig™ form contains normal Fc.
**FIG. 23** is a representation of a TriNKET in the Orthogonal Fab interface (Ortho-Fab) form, which is a heterodimeric construct that contains 2 Fab fragments binding to target 1 and target 2 fused to Fc. Light chain (LC)-heavy chain (HC) pairing is ensured by orthogonal interface. Heterodimerization is ensured by mutations in the Fc.

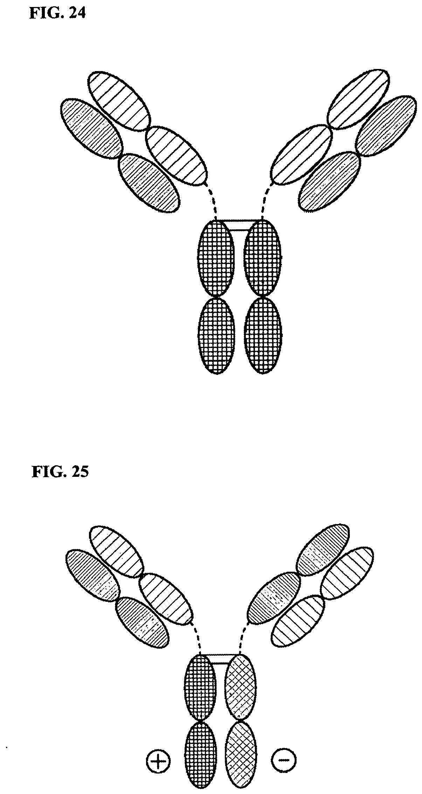

**FIG. 24** is a representation of a TriNKET in the 2-in-1 Ig format.
**FIG. 25** is a representation of a TriNKET in the ES form, which is a heterodimeric construct containing two different Fab fragments binding to target 1 and target 2 fused to the Fc. Heterodimerization is ensured by electrostatic steering mutations in the Fc.

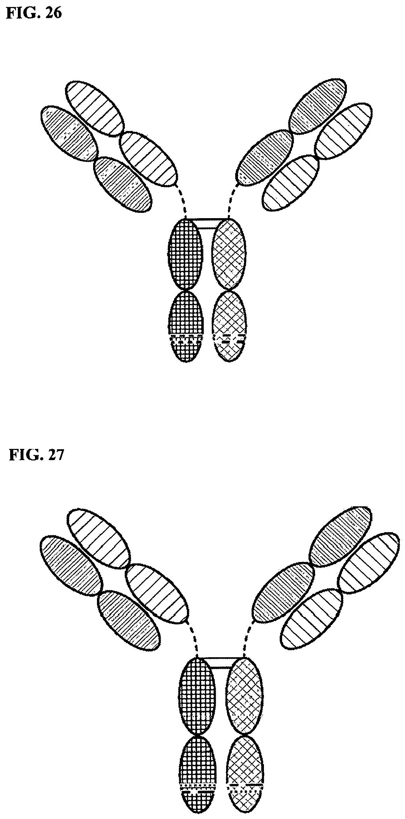

**FIG. 26** is a representation of a TriNKET in the Fab fragment Arm Exchange form: antibodies that exchange Fab arms by swapping a heavy chain and attached light chain (half-molecule) with a heavy-light chain pair from another molecule, resulting in bispecific antibodies. Fab Arm Exchange form (cFae) is a heterodimer containing 2 Fab fragments binding to target 1 and 2, and an Fc stabilized by heterodimerization mutations.
**FIG. 27** is a representation of a TriNKET in the SEED Body form, which is a heterodimer containing 2 Fab fragments binding to target 1 and 2, and an Fc stabilized by heterodimerization mutations.

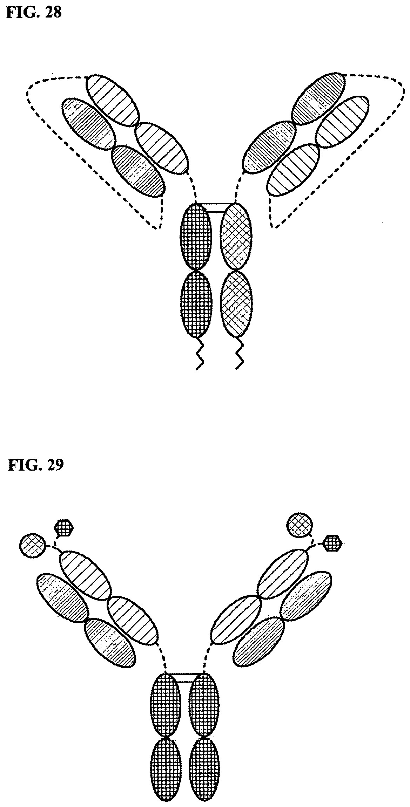

**FIG. 28** is a representation of a TriNKET in the LuZ-Y form, in which a leucine zipper is used to induce heterodimerization of two different HCs. The LuZ-Y form is a heterodimer containing two different scFabs binding to target 1 and 2, fused to Fc. Heterodimerization is ensured through leucine zipper motifs fused to C-terminus of Fc.
**FIG. 29** is a representation of a TriNKET in the Cov-X-Body form.
**FIGs. 30A and 30B** are representations of TriNKETs in the κλ-Body forms, which are heterodimeric constructs with two different Fab fragments fused to Fc stabilized by heterodimerization mutations: one Fab fragment targeting antigen 1 contains kappa LC, and the second Fab fragment targeting antigen 2 contains lambda LC. FIG. 30A is an exemplary representation of one form of a κλ-Body; FIG. 30B is an exemplary representation of another κλ-Body.

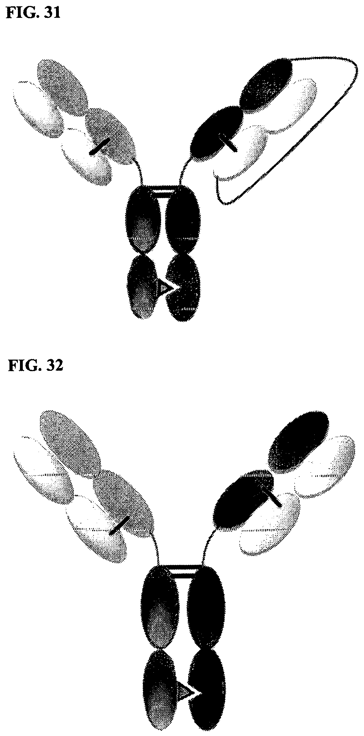

**FIG. 31** is an Oasc-Fab heterodimeric construct that includes Fab fragment binding to target 1 and scFab binding to target 2, both of which are fused to the Fc domain. Heterodimerization is ensured by mutations in the Fc domain.
**FIG. 32** is a DuetMab, which is a heterodimeric construct containing two different Fab fragments binding to antigens 1 and 2, and an Fc that is stabilized by heterodimerization mutations. Fab fragments 1 and 2 contain differential S-S bridges that ensure correct light chain and heavy chain pairing.

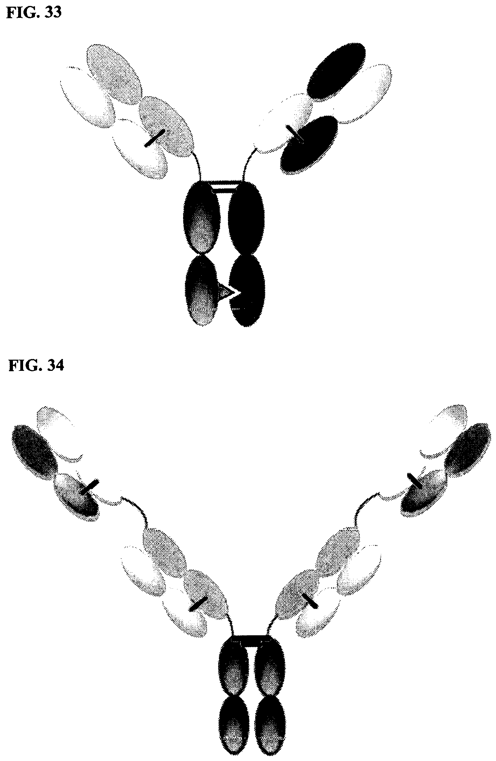

**FIG. 33** is a CrossmAb, which is a heterodimeric construct with two different Fab fragments binding to targets 1 and 2, and an Fc stabilized by heterodimerization mutations. CL and CH1 domains, and VH and VL domains are switched, e.g., CH1 is fused in-line with VL, while CL is fused in-line with VH
**FIG. 34** is a Fit-Ig, which is a homodimeric construct where Fab fragment binding to antigen 2 is fused to the N-terminus of HC of Fab fragment that binds to antigen 1. The construct contains wild-type Fc.
**FIG. 35** are line graphs showing that TriNKETs and monoclonal antibodies ("mAbs") bind to EGFR expressed on NCI-H2172 human lung cancer cells.
**FIG. 36** are line graphs showing that TriNKETs and mAbs bind to EGFR expressed on HCC827 human lung cancer cells.
**FIG. 37** are line graphs showing that TriNKETs and mAbs bind to EGFR expressed on NCI-H747 human colon cancer cells.
**FIG. 38** are line graphs showing TriNKET-mediated killing of NCI-H2172 cells (lung, EGFR L858R T790M) with rested human NK cells.
**FIG. 39** are line graphs showing TriNKET-mediated killing of NCI-H2172 cells (lung, EGFR L858R T790M) with rested human NK cells.
**FIG. 40** are line graphs showing TriNKET-mediated killing of NCI-H747 cells (colon, KRAS G13D) with rested human NK cells.
**FIG. 41** are line graphs showing TriNKET-mediated killing of NCI-H747 cells (colon, KRAS G13D) with rested human NK cells.
**FIG. 42** are line graphs showing TriNKET-mediated killing of NCI-H2172 cells (lung, EGFR L858R T790M) with KHYG1-CD16V cells.
**FIG. 43** are line graphs showing TriNKET-mediated killing of NCI-H1975 cells (lung, EGFR L858R) with KHYG1-CD16V cells.
**FIG. 44** are line graphs showing TriNKET-mediated killing of NCI-N87 cells (gastric) with KHYG1-CD16V cells.
**FIG. 45** are line graphs showing TriNKET-mediated killing of HCT116 cells (colon, KRAS G13D) with KHYG1-CD16V cells.
**FIG. 46** are line graphs showing TriNKET-mediated killing of A549 cells (lung, KRAS G12S) with KHYG1-CD16V cells.

### DETAILED DESCRIPTION

The invention provides multi-specific binding proteins that bind the NKG2D receptor and CD16 receptor on natural killer cells, and the tumor-associated antigen EGFR, HLA-E, CCR4, or PD-L1. In some embodiments, the multi-specific proteins further include an additional antigen-binding site that binds EGFR, HLA-E, CCR4, or PD-L1 or another tumor-associated antigen. The invention also provides pharmaceutical compositions comprising such multi-specific binding proteins, and therapeutic methods using such multi-specific proteins and pharmaceutical compositions, for purposes such as treating cancer. Various aspects of the invention are set forth below in sections; however, aspects of the invention described in one particular section are not to be limited to any particular section.

To facilitate an understanding of the present invention, a number of terms and phrases are defined below.

The terms "a" and "an" as used herein mean "one or more" and include the plural unless the context is inappropriate.

As used herein, the term "antigen-binding site" refers to the part of the immunoglobulin molecule that participates in antigen binding. In human antibodies, the antigen-binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FR." Thus the term "FR" refers to amino acid sequences which are naturally found between and adjacent to hypervariable regions in immunoglobulins. In a human antibody molecule, the three hypervariable regions of a light chain and the three hypervariable regions of a heavy chain are disposed relative to each other in three dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs." In certain animals, such as camels and cartilaginous fish, the antigen-binding site is formed by a single antibody chain providing a "single-domain antibody." Antigen-binding sites can exist in an intact antibody, in an antigen-binding fragment of an antibody that retains the antigen-binding surface, or in a recombinant polypeptide such as an scFv, using a peptide linker to connect the heavy chain variable domain to the light chain variable domain in a single polypeptide.

The term "tumor associated antigen" as used herein means any antigen including but not limited to a protein, glycoprotein, ganglioside, carbohydrate, lipid that is associated with cancer. Such antigen can be expressed on malignant cells or in the tumor microenvironment such as on tumor-associated blood vessels, extracellular matrix, mesenchymal stroma, or immune infiltrates.

As used herein, "EGFR" (Epidermal growth factor receptor, also known as ERBB, ERBB1, and HER1) refers to the protein of Uniprot Accession No. P00533 and related isoforms.

As used herein, "HLA-E" (HLA class I histocompatibility antigen, alpha chain E, also known as MHC class I antigen E, HLA-6.2, and HLAE) refers to the protein of Uniprot Accession No. P13747 and related isoforms.

As used herein, "CCR4" (C-C chemokine receptor type 4, also known as C-C CKR-4, CC-CKR-4, CCR-4, K5-5, and CMKBR4) refers to the protein of Uniprot Accession No. P51679 and related isoforms.

As used herein, "PD-L1" (Programmed cell death 1 ligand 1, also known as PDCD1 ligand 1, Programmed death ligand 1, B7 homolog 1, B7-H1, CD274, B7H1, PDCD1L1, PDCD1LG1, and PDL1) refers to the protein of Uniprot Accession No. Q9NZQ7 and related isoforms.

As used herein, the terms "subject" and "patient" refer to an organism to be treated by the methods and compositions described herein. Such organisms preferably include, but are not limited to, mammals (*e.g*., murines, simians, equines, bovines, porcines, canines, felines, and the like), and more preferably include humans.

As used herein, the term "effective amount" refers to the amount of a compound *(e.g.,* a compound of the present invention) sufficient to effect beneficial or desired results. An effective amount can be administered in one or more administrations, applications or dosages and is not intended to be limited to a particular formulation or administration route. As used herein, the term "treating" includes any effect, *e.g*., lessening, reducing, modulating, ameliorating or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use *in vivo* or *ex vivo.*

As used herein, the term "pharmaceutically acceptable carrier" refers to any of the standard pharmaceutical carriers, such as a phosphate buffered saline solution, water, emulsions (*e.g*., such as an oil/water or water/oil emulsions), and various types of wetting agents. The compositions also can include stabilizers and preservatives. For examples of carriers, stabilizers and adjuvants, *see e.g.,* Martin, Remington's Pharmaceutical Sciences, 15th Ed., Mack Publ. Co., Easton, PA [1975].

As used herein, the term "pharmaceutically acceptable salt" refers to any pharmaceutically acceptable salt *(e.g.,* acid or base) of a compound of the present invention which, upon administration to a subject, is capable of providing a compound of this invention or an active metabolite or residue thereof. As is known to those of skill in the art, "salts" of the compounds of the present invention may be derived from inorganic or organic acids and bases. Exemplary acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Exemplary bases include, but are not limited to, alkali metal (*e.g*., sodium) hydroxides, alkaline earth metal (*e.g*., magnesium) hydroxides, ammonia, and compounds of formula NW₄⁺, wherein W is C₁₋₄ alkyl, and the like.

Exemplary salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like.

For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

Throughout the description, where compositions are described as having, including, or comprising specific components, or where processes and methods are described as having, including, or comprising specific steps, it is contemplated that, additionally, there are compositions of the present invention that consist essentially of, or consist of, the recited components, and that there are processes and methods according to the present invention that consist essentially of, or consist of, the recited processing steps.

As a general matter, compositions specifying a percentage are by weight unless otherwise specified. Further, if a variable is not accompanied by a definition, then the previous definition of the variable controls.

### I. PROTEINS

The invention provides multi-specific binding proteins that bind to the NKG2D receptor and CD16 receptor on natural killer cells, and the tumor-associated antigen EGFR, HLA-E, CCR4, or PD-L1. The multi-specific binding proteins are useful in the pharmaceutical compositions and therapeutic methods described herein. Binding of the multi-specific binding proteins to the NKG2D receptor and CD16 receptor on a natural killer cell enhances the activity of the natural killer cell toward destruction of tumor cells expressing EGFR, HLA-E, CCR4, or PD-L1. Binding of the multi-specific binding proteins to EGFR, HLA-E, CCR4, or PD-L1-expressing cells brings the cancer cells into proximity with the natural killer cell, which facilitates direct and indirect destruction of the cancer cells by the natural killer cell. Further description of some exemplary multi-specific binding proteins is provided below.

The first component of the multi-specific binding proteins binds to NKG2D receptor-expressing cells, which can include but are not limited to NK cells, γδ T cells and CD8⁺ αβ T cells. Upon NKG2D binding, the multi-specific binding proteins may block natural ligands, such as ULBP6 (UL16 binding protein 6) and MICA (Major Histocompatibility Complex Class I Chain-Related A), from binding to NKG2D and activating NKG2D receptors.

The second component of the multi-specific binding proteins binds EGFR, HLA-E, CCR4, or PD-L1. EGFR, HLA-E, CCR4, or PD-L1-expressing cells, which may be found in leukemias such as, for example, acute myeloid leukemia and T-cell leukemia.

The third component for the multi-specific binding proteins binds to cells expressing CD16, an Fc receptor on the surface of leukocytes including natural killer cells, macrophages, neutrophils, eosinophils, mast cells, and follicular dendritic cells.

The multi-specific binding proteins described herein can take various formats. For example, one format is a heterodimeric, multi-specific antibody including a first immunoglobulin heavy chain, a first immunoglobulin light chain, a second immunoglobulin heavy chain and a second immunoglobulin light chain (FIG. 1). The first immunoglobulin heavy chain includes a first Fc (hinge-CH2-CH3) domain, a first heavy chain variable domain and optionally a first CH1 heavy chain domain. The first immunoglobulin light chain includes a first light chain variable domain and a first light chain constant domain. The first immunoglobulin light chain, together with the first immunoglobulin heavy chain, forms an antigen-binding site that binds NKG2D. The second immunoglobulin heavy chain comprises a second Fc (hinge-CH2-CH3) domain, a second heavy chain variable domain and optionally a second CH1 heavy chain domain. The second immunoglobulin light chain includes a second light chain variable domain and a second light chain constant domain. The second immunoglobulin light chain, together with the second immunoglobulin heavy chain, forms an antigen-binding site that binds EGFR, HLA-E, CCR4, or PD-L1. The first Fc domain and second Fc domain together are able to bind to CD16 (FIG. 1). In some embodiments, the first immunoglobulin light chain is identical to the second immunoglobulin light chain.

Another exemplary format involves a heterodimeric, multi-specific antibody including a first immunoglobulin heavy chain, a second immunoglobulin heavy chain and an immunoglobulin light chain (FIG. 2). The first immunoglobulin heavy chain includes a first Fc (hinge-CH2-CH3) domain fused via either a linker or an antibody hinge to a single-chain variable fragment (scFv) composed of a heavy chain variable domain and light chain variable domain which pair and bind NKG2D, or bind the EGFR, HLA-E, CCR4, or PD-L1 antigen. The second immunoglobulin heavy chain includes a second Fc (hinge-CH2-CH3) domain, a second heavy chain variable domain and optionally a CH1 heavy chain domain. The immunoglobulin light chain includes a light chain variable domain and a light chain constant domain. The second immunoglobulin heavy chain pairs with the immunoglobulin light chain and binds to NKG2D or binds the tumor-associated antigen EGFR, HLA-E, CCR4, or PD-L1. The first Fc domain and the second Fc domain together are able to bind to CD16 (FIG. 2).

One or more additional binding motifs may be fused to the C-terminus of the constant region CH3 domain, optionally via a linker sequence. In certain embodiments, the antigen-binding motif is a single-chain or disulfide-stabilized variable region (scFv) forming a tetravalent or trivalent molecule.

In some embodiments, the multi-specific binding protein is in the Triomab form, which is a trifunctional, bispecific antibody that maintains an IgG-like shape. This chimera consists of two half antibodies, each with one light and one heavy chain, that originate from two parental antibodies.

In some embodiments, the multi-specific binding protein is the KiH Common Light Chain (LC) form, which involves the knobs-into-holes (KIHs) technology. The KIH involves engineering C_{H}3 domains to create either a "knob" or a "hole" in each heavy chain to promote heterodimerization. The concept behind the "Knobs-into-Holes (KiH)" Fc technology was to introduce a "knob" in one CH3 domain (CH3A) by substitution of a small residue with a bulky one (*e.g*., T366W_{CH3A} in EU numbering). To accommodate the "knob," a complementary "hole" surface was created on the other CH3 domain (CH3B) by replacing the closest neighboring residues to the knob with smaller ones (*e.g.,* T366S/L368A/Y407V_{CH3B}). The "hole" mutation was optimized by structured-guided phage library screening (Atwell S, Ridgway JB, Wells JA, Carter P., Stable heterodimers from remodeling the domain interface of a homodimer using a phage display library, J. Mol. Biol. (1997) 270(1):26-35). X-ray crystal structures of KiH Fc variants (Elliott JM, Ultsch M, Lee J, Tong R, Takeda K, Spiess C, et al., Antiparallel conformation of knob and hole aglycosylated half-antibody homodimers is mediated by a CH2-CH3 hydrophobic interaction. J. Mol. Biol. (2014) 426(9):1947-57; Mimoto F, Kadono S, Katada H, Igawa T, Kamikawa T, Hattori K. Crystal structure of a novel asymmetrically engineered Fc variant with improved affinity for FcγRs. Mol. Immunol. (2014) 58(1): 132-8) demonstrated that heterodimerization is thermodynamically favored by hydrophobic interactions driven by steric complementarity at the inter-CH3 domain core interface, whereas the knob-knob and the hole-hole interfaces do not favor homodimerization owing to steric hindrance and disruption of the favorable interactions, respectively.

In some embodiments, the multi-specific binding protein is in the dual-variable domain immunoglobulin (DVD-Ig™) form, which combines the target binding domains of two monoclonal antibodies via flexible naturally occurring linkers, and yields a tetravalent IgG-like molecule.

In some embodiments, the multi-specific binding protein is in the Orthogonal Fab interface (Ortho-Fab) form. In the ortho-Fab IgG approach (Lewis SM, Wu X, Pustilnik A, Sereno A, Huang F, Rick HL, et al., Generation of bispecific IgG antibodies by structure-based design of an orthogonal Fab interface. Nat. Biotechnol. (2014) 32(2): 191-8), structure-based regional design introduces complementary mutations at the LC and HC_{VH-CH1} interface in only one Fab fragment, without any changes being made to the other Fab fragment.

In some embodiments, the multi-specific binding protein is in the 2-in-1 Ig format. In some embodiments, the multi-specific binding protein is in the ES form, which is a heterodimeric construct containing two different Fab fragments binding to targets 1 and target 2 fused to the Fc. Heterodimerization is ensured by electrostatic steering mutations in the Fc.

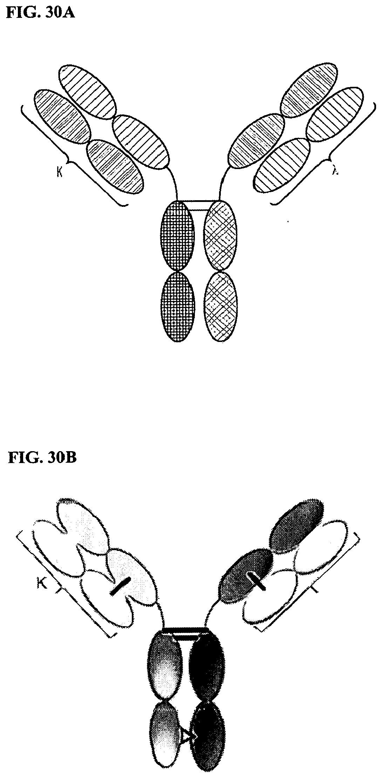

In some embodiments, the multi-specific binding protein is in the κλ-Body form, which is a heterodimeric construct with two different Fab fragments fused to Fc stabilized by heterodimerization mutations: Fab fragment1 targeting antigen 1 contains kappa LC, while second Fab fragment targeting antigen 2 contains lambda LC. FIG. 30A is an exemplary representation of one form of a κλ-Body; FIG. 30B is an exemplary representation of another κλ-Body.

In some embodiments, the multi-specific binding protein is in Fab Arm Exchange form (antibodies that exchange Fab arms by swapping a heavy chain and attached light chain (half-molecule) with a heavy-light chain pair from another molecule, which results in bispecific antibodies).

In some embodiments, the multi-specific binding protein is in the SEED Body form. The strand-exchange engineered domain (SEED) platform was designed to generate asymmetric and bispecific antibody-like molecules, a capability that expands therapeutic applications of natural antibodies. This protein engineered platform is based on exchanging structurally related sequences of immunoglobulin within the conserved CH3 domains. The SEED design allows efficient generation of AG/GA heterodimers, while disfavoring homodimerization of AG and GA SEED CH3 domains. (Muda M. et al., Protein Eng. Des. Sel. (2011, 24(5):447-54)).

In some embodiments, the multi-specific binding protein is in the LuZ-Y form, in which a leucine zipper is used to induce heterodimerization of two different HCs. (Wranik, BJ. et al., J. Biol. Chem. (2012), 287:43331-9).

In some embodiments, the multi-specific binding protein is in the Cov-X-Body form. In bispecific CovX-Bodies, two different peptides are joined together using a branched azetidinone linker and fused to the scaffold antibody under mild conditions in a site-specific manner. Whereas the pharmacophores are responsible for functional activities, the antibody scaffold imparts long half-life and Ig-like distribution. The pharmacophores can be chemically optimized or replaced with other pharmacophores to generate optimized or unique bispecific antibodies. (Doppalapudi VR et al., PNAS (2010), 107(52);22611-22616).

In some embodiments, the multi-specific binding protein is in an Oasc-Fab heterodimeric form that includes Fab fragment binding to target 1, and scFab binding to target 2 fused to Fc. Heterodimerization is ensured by mutations in the Fc.

In some embodiments, the multi-specific binding protein is in a DuetMab form, which is a heterodimeric construct containing two different Fab fragments binding to antigens 1 and 2, and Fc stabilized by heterodimerization mutations. Fab fragments 1 and 2 contain differential S-S bridges that ensure correct LC and HC pairing.

In some embodiments, the multi-specific binding protein is in a CrossmAb form, which is a heterodimeric construct with two different Fab fragments binding to targets 1 and 2, fused to Fc stabilized by heterodimerization. CL and CH1 domains and VH and VL domains are switched, e.g., CH1 is fused in-line with VL, while CL is fused in-line with VH

In some embodiments, the multi-specific binding protein is in a Fit-Ig form, which is a homodimeric construct where Fab fragment binding to antigen 2 is fused to the N terminus of HC of Fab fragment that binds to antigen 1. The construct contains wild-type Fc.

Table 1 lists peptide sequences of heavy chain variable domains and light chain variable domains that, in combination, can bind to NKG2D. The NKG2D binding domains can vary in their binding affinity to NKG2D, nevertheless, they all activate human NKG2D and NK cells.

| Table 1 | | |
|---|---|---|
| Clones | Heavy chain variable region amino acid sequence | Light chain variable region amino acid sequence |
| ADI-27705 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:1) | DIQMTQSPSTLSASVGDRVTIT CRASQSISSWLAWYQQKPGK APKLLIYKASSLESGVPSRFSG SGSGTEFTLTISSLQPDDFATY YCQQYNSYPITFGGGTKVEIK (SEQ ID NO:2) |
| | CDR1 (SEQ ID NO:105) - GSFSGYYWS | |
| | CDR2 (SEQ ID NO:106) - EIDHSGSTNYNPSLKS | |
| | CDR3 (SEQ ID NO:107)-ARARGPWSFDP | |
| ADI-27724 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:3) | EIVLTQSPGTLSLSPGERATLS CRASQSVSSSYLAWYQQKPG QAPRLLIYGASSRATGIPDRFS GSGSGTDFTLTISRLEPEDFAV YYCQQYGSSPITFGGGTKVEI K (SEQ ID NO:4) |
| ADI-27740 (A40) | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:5) | DIQMTQSPSTLSASVGDRVTIT CRASQSIGSWLAWYQQKPGK APKLLIYKASSLESGVPSRFSG SGSGTEFTLTISSLQPDDFATY YCQQYHSFYTFGGGTKVEIK (SEQ ID NO:6) |
| ADI-27741 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:7) | DIQMTQSPSTLSASVGDRVTIT CRASQSIGSWLAWYQQKPGK APKLLIYKASSLESGVPSRFSG SGSGTEFTLTISSLQPDDFATY YCQQSNSYYTFGGGTKVEIK (SEQ ID NO:8) |
| ADI-27743 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:9) | DIQMTQSPSTLSASVGDRVTIT CRASQSISSWLAWYQQKPGK APKLLIYKASSLESGVPSRFSG SGSGTEFTLTISSLQPDDFATY YCQQYNSYPTFGGGTKVEIK (SEQ ID NO:10) |
| ADI-28153 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWGFDPWGQGTLVTVSS | ELQMTQSPSSLSASVGDRVTIT CRTSQSISSYLNWYQQKPGQP PKLLIYWASTRESGVPDRFSGS GSGTDFTLTISSLQPEDSATYY CQQSYDIPYTFGQGTKLEIK |
| | (SEQ ID NO:11) | (SEQ ID NO:12) |
| ADI-28226 (C26) | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:13) | DIQMTQSPSTLSASVGDRVTIT CRASQSISSWLAWYQQKPGK APKLLIYKASSLESGVPSRFSG SGSGTEFTLTISSLQPDDFATY YCQQYGSFPITFGGGTKVEIK (SEQ ID NO:14) |
| ADI-28154 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:15) | DIQMTQSPSTLSASVGDRVTIT CRASQSISSWLAWYQQKPGK APKLLIYKASSLESGVPSRFSG SGSGTDFTLTISSLQPDDFATY YCQQSKEVPWTFGQGTKVEIK (SEQ ID NO:16) |
| ADI-29399 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:17) | DIQMTQSPSTLSASVGDRVTIT CRASQSISSWLAWYQQKPGK APKLLIYKASSLESGVPSRFSG SGSGTEFTLTISSLQPDDFATY YCQQYNSFPTFGGGTKVEIK (SEQ ID NO:18) |
| ADI-29401 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:19) | DIQMTQSPSTLSASVGDRVTIT CRASQSIGSWLAWYQQKPGK APKLLIYKASSLESGVPSRFSG SGSGTEFTLTISSLQPDDFATY YCQQYDIYPTFGGGTKVEIK (SEQ ID NO:20) |
| ADI-29403 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:21) | DIQMTQSPSTLSASVGDRVTIT CRASQSISSWLAWYQQKPGK APKLLIYKASSLESGVPSRFSG SGSGTEFTLTISSLQPDDFATY YCQQYDSYPTFGGGTKVEIK (SEQ ID NO:22) |
| ADI-29405 | QVQLQQWGAGLLKPSETLSLTCAV YGGSFSGYYWSWIRQPPGKGLEWI | DIQMTQSPSTLSASVGDRVTIT CRASQSISSWLAWYQQKPGK |
| | GEIDHSGSTNYNPSLKSRVTISVDTS KNQFSLKLSSVTAADTAVYYCARA RGPWSFDPWGQGTLVTVSS (SEQ ID NO:23) | APKLLIYKASSLESGVPSRFSG SGSGTEFTLTISSLQPDDFATY YCQQYGSFPTFGGGTKVEIK (SEQ ID NO:24) |
| ADI-29407 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:25) | DIQMTQSPSTLSASVGDRVTIT CRASQSISSWLAWYQQKPGK APKLLIYKASSLESGVPSRFSG SGSGTEFTLTISSLQPDDFATY YCQQYQSFPTFGGGTKVEIK (SEQ ID NO:26) |
| ADI-29419 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:27) | DIQMTQSPSTLSASVGDRVTIT CRASQSISSWLAWYQQKPGK APKLLIYKASSLESGVPSRFSG SGSGTEFTLTISSLQPDDFATY YCQQYSSFSTFGGGTKVEIK (SEQ ID NO:28) |
| ADI-29421 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:29) | DIQMTQSPSTLSASVGDRVTIT CRASQSISSWLAWYQQKPGK APKLLIYKASSLESGVPSRFSG SGSGTEFTLTISSLQPDDFATY YCQQYESYSTFGGGTKVEIK (SEQ ID NO:30) |
| ADI-29424 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:31) | DIQMTQSPSTLSASVGDRVTIT CRASQSISSWLAWYQQKPGK APKLLIYKASSLESGVPSRFSG SGSGTEFTLTISSLQPDDFATY YCQQYDSFITFGGGTKVEIK (SEQ ID NO:32) |
| ADI-29425 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS | DIQMTQSPSTLSASVGDRVTIT CRASQSISSWLAWYQQKPGK APKLLIYKASSLESGVPSRFSG SGSGTEFTLTISSLQPDDFATY YCQQYQSYPTFGGGTKVEIK |
| | (SEQ ID NO:33) | (SEQ ID NO:34) |
| ADI-29426 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:35) | DIQMTQSPSTLSASVGDRVTIT CRASQSIGSWLAWYQQKPGK APKLLIYKASSLESGVPSRFSG SGSGTEFTLTISSLQPDDFATY YCQQYHSFPTFGGGTKVEIK (SEQ ID NO:36) |
| ADI-29429 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:37) | DIQMTQSPSTLSASVGDRVTIT CRASQSIGSWLAWYQQKPGK APKLLIYKASSLESGVPSRFSG SGSGTEFTLTISSLQPDDFATY YCQQYELYSYTFGGGTKVEIK (SEQ ID NO:38) |
| ADI-29447 (F47) | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:39) | DIQMTQSPSTLSASVGDRVTIT CRASQSISSWLAWYQQKPGK APKLLIYKASSLESGVPSRFSG SGSGTEFTLTISSLQPDDFATY YCQQYDTFITFGGGTKVEIK (SEQ ID NO:40) |
| ADI-27727 | QVQLVQSGAEVKKPGSSVKVSCKA SGGTFSSYAISWVRQAPGQGLEWM GGIIPIFGTANYAQKFQGRVTITADE STSTAYMELSSLRSEDTAVYYCAR GDSSIRHAYYYYGMDVWGQGTTV TVSS (SEQ ID NO:41) | DIVMTQSPDSLAVSLGERATIN CKSSQSVLYSSNNKNYLAWY QQKPGQPPKLLIYWASTRESG VPDRFSGSGSGTDFTLTISSLQ AEDVAVYYCQQYYSTPITFGG GTKVEIK (SEQ ID NO:42) |
| | CDR1 (SEQ ID NO:43) - GTFSSY AIS | CDR1 (SEQ ID NO:46) - KSSQSVLYSSNNKNYLA |
| | CDR2 (SEQ ID NO:44) - GIIPIFGTANYAQKFQG | CDR2 (SEQ ID NO:47) - WASTRES |
| | CDR3 (SEQ ID NO:45) - ARGDSSIRHAYYYYGMDV | CDR3 (SEQ ID NO:48) - QQYYSTPIT |
| ADI-29443 (F43) | QLQLQESGPGLVKPSETLSLTCTVS GGSISSSSYYWGWIRQPPGKGLEWI GSIYYSGSTYYNPSLKSRVTISVDTS KNQFSLKLSSVTAADTAVYYCARG SDRFHPYFDYWGQGTLVTVSS (SEQ ID NO:49) | EIVLTQSPATLSLSPGERATLS CRASQSVSRYLAWYQQKPGQ APRLLIYDASNRATGIPARFSG SGSGTDFTLTISSLEPEDFAVY YCQQFDTWPPTFGGGTKVEIK (SEQ ID NO:50) |
| | CDR1 (SEQ ID NO:51) - GSISSSSYYWG | CDR1 (SEQ ID NO:54) - RASQSVSRYLA |
| | CDR2 (SEQ ID NO:52) - SIYYSGSTYYNPSLKS | CDR2 (SEQ ID NO:55) - DASNRAT |
| | CDR3 (SEQ ID NO:53) - ARGSDRFHPYFDY | CDR3 (SEQ ID NO:56) - QQFDTWPPT |
| ADI-29404 (F04) | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:57) | DIQMTQSPSTLSASVGDRVTIT CRASQSISSWLAWYQQKPGK APKLLIYKASSLESGVPSRFSG SGSGTEFTLTISSLQPDDFATY YCEQYDSYPTFGGGTKVEIK (SEQ ID NO:58) |
| ADI-28200 | QVQLVQSGAEVKKPGSSVKVSCKA SGGTFSSYAISWVRQAPGQGLEWM GGIIPIFGTANYAQKFQGRVTITADE STSTAYMELSSLRSEDTAVYYCAR RGRKASGSFYYYYGMDVWGQGTT VTVSS (SEQ ID NO:59) |  (SEQ ID NO:60) |
| | CDR1 (SEQ ID NO: 134) - GTFSSY AIS | CDR1 (SEQ ID NO:137) - ESSQSLLNSGNQKNYLT |
| | CDR2 (SEQ ID NO:135) - GIIPIFGTANYAQKFQG | CDR2 (SEQ ID NO:138) - WASTRES |
| | CDR3 (SEQ ID NO:136) - ARRGRKASGSFYYYYGMDV | CDR3 (SEQ ID NO:139) - QNDYSYPYT |
| ADI-29379 (E79) | QVQLVQSGAEVKKPGASVKVSCK ASGYTFTSYYMHWVRQAPGQGLE WMGIINPSGGSTSYAQKFQGRVTM TRDTSTSTVYMELSSLRSEDTAVYY CARGAPNYGDTTHDYYYMDVWG KGTTVTVSS (SEQ ID NO:61) | EIVMTQSPATLSVSPGERATLS CRASQSVSSNLAWYQQKPGQ APRLLIYGASTRATGIPARFSG SGSGTEFTLTISSLQSEDFAVY YCQQYDDWPFTFGGGTKVEI K (SEQ ID NO:62) |
| | CDR1 (SEQ ID NO:63) - YTFTSYYMH | CDR1 (SEQ ID NO:66) - RASQSVSSNLA |
| | CDR2 (SEQ ID NO:64) - IINPSGGSTSYAQKFQG | CDR2 (SEQ ID NO:67) - GASTRAT |
| | CDR3 (SEQ ID NO:65) - ARGAPNYGDTTHDYYYMDV | CDR3 (SEQ ID NO:68) - QQYDDWPFT |
| ADI-29463 (F63) | QVQLVQSGAEVKKPGASVKVSCK ASGYTFTGYYMHWVRQAPGQGLE WMGWINPNSGGTNYAQKFQGRVT MTRDTSISTAYMELSRLRSDDTAV YYCARDTGEYYDTDDHGMDVWG QGTTVTVSS (SEQ ID NO:69) | EIVLTQSPGTLSLSPGERATLS CRASQSVSSNLAWYQQKPGQ APRLLIYGASTRATGIPARFSG SGSGTEFTLTISSLQSEDFAVY YCQQDDYWPPTFGGGTKVEI K (SEQ ID NO:70) |
| | CDR1 (SEQ ID NO:71) - YTFTGYYMH | CDR1 (SEQ ID NO:74) - RASQSVSSNLA |
| | CDR2 (SEQ ID NO:72) - WINPNSGGTNYAQKFQG | CDR2 (SEQ ID NO:75) - GASTRAT |
| | CDR3 (SEQ ID NO:73) - ARDTGEYYDTDDHGMDV | CDR3 (SEQ ID NO:76) - QQDDYWPPT |
| ADI-27744 (A44) | EVQLLESGGGLVQPGGSLRLSCAAS GFTFSSYAMSWVRQAPGKGLEWV SAISGSGGSTYYADSVKGRFTISRD NSKNTLYLQMNSLRAEDTAVYYC AKDGGYYDSGAGDYWGQGTLVTV SS (SEQ ID NO:77) | DIQMTQSPSSVSASVGDRVTIT CRASQGIDSWLAWYQQKPGK APKLLIYAASSLQSGVPSRFSG SGSGTDFTLTISSLQPEDFATY YCQQGVSYPRTFGGGTKVEIK (SEQ ID NO:78) |
| | CDR1 (SEQ ID NO:79) - FTFSSYAMS | CDR1 (SEQ ID NO:82) - |
| | CDR2 (SEQ ID NO:80) - | RASQGIDSWLA |
| | AISGSGGSTYYADSVKG | CDR2 (SEQ ID NO:83) - |
| | CDR3 (SEQ ID NO:81) - | AASSLQS |
| | AKDGGYYDSGAGDY | CDR3 (SEQ ID NO:84) - QQGVSYPRT |
| ADI-27749 (A49) | EVQLVESGGGLVKPGGSLRLSCAA SGFTFSSYSMNWVRQAPGKGLEW VSSISSSSSYIYYADSVKGRFTISRD NAKNSLYLQMNSLRAEDTAVYYC ARGAPMGAAAGWFDPWGQGTLVT VSS (SEQ ID NO:85) | DIQMTQSPSSVSASVGDRVTIT CRASQGISSWLAWYQQKPGK APKLLIYAASSLQSGVPSRFSG SGSGTDFTLTISSLQPEDFATY YCQQGVSFPRTFGGGTKVEIK (SEQ ID NO:86) |
| | | CDR1 (SEQ ID NO:90) - |
| | CDR1 (SEQ ID NO:87) - FTFSSYSMN | RASQGISSWLA |
| | CDR2 (SEQ ID NO:88) - | CDR2 (SEQ ID NO:91) - |
| | SISSSSSYIYYADSVKG | AASSLQS |
| | CDR3 (SEQ ID NO:89) - | CDR3 (SEQ ID NO:92) - |
| | ARGAPMGAAAGWFDP | QQGVSFPRT |
| ADI-29378 (E78) | QVQLVQSGAEVKKPGASVKVSCK ASGYTFTSYYMHWVRQAPGQGLE WMGIINPSGGSTSYAQKFQGRVTM TRDTSTSTVYMELSSLRSEDTAVYY CAREGAGFAYGMDYYYMDVWGK GTTVTVSS (SEQ ID NO:93) | EIVLTQSPATLSLSPGERATLS CRASQSVSSYLAWYQQKPGQ APRLLIYDASNRATGIPARFSG SGSGTDFTLTISSLEPEDFAVY YCQQSDNWPFTFGGGTKVEIK (SEQ ID NO:94) |
| | | CDR1 (SEQ ID NO:98) - |
| | CDR1 (SEQ ID NO:95) - | RASQSVSSYLA |
| | YTFTSYYMH | CDR2 (SEQ ID NO:99) - |
| | CDR2 (SEQ ID NO:96) - | DASNRAT |
| | IINPSGGSTSYAQKFQG | CDR3 (SEQ ID NO:100) - |
| | CDR3 (SEQ ID NO:97) - | QQSDNWPFT |
| | AREGAGFAYGMDYYYMDV | |

Alternatively, a heavy chain variable domain represented by SEQ ID NO:101 can be paired with a light chain variable domain represented by SEQ ID NO:102 to form an antigen-binding site that can bind to NKG2D, as illustrated in US 9,273,136.

SEQ ID NO:101
QVQLVESGGGLVKPGGSLRLSCAASGFTFSSYGMHWVRQAPGKGLEWVAFI
RYDGSNKYYADSVKGRFTISRDNSKNTLYLQMNSLRAEDTAVYYCAKDRGL
GDGTYFDYWGQGTTVTVSS

SEQ ID NO:102
QSALTQPASVSGSPGQSITISCSGSSSNIGNNAVNWYQQLPGKAPKLLIYYDDL
LPSGVSDRFSGSKSGTSAFLAISGLQSEDEADYYCAAWDDSLNGPVFGGGTK
LTVL

Alternatively, a heavy chain variable domain represented by SEQ ID NO:103 can be paired with a light chain variable domain represented by SEQ ID NO:104 to form an antigen-binding site that can bind to NKG2D, as illustrated in US 7,879,985.

SEQ ID NO:103
QVHLQESGPGLVKPSETLSLTCTVSDDSISSYYWSWIRQPPGKGLEWIGHISYS
GSANYNPSLKSRVTISVDTSKNQFSLKLSSVTAADTAVYYCANWDDAFNIWG
QGTMVTVSS

SEQ ID NO:104
EIVLTQSPGTLSLSPGERATLSCRASQSVSSSYLAWYQQKPGQAPRLLIYGASS
RATGIPDRFSGSGSGTDFTLTISRLEPEDFAVYYCQQYGSSPWTFGQGTKVEIK

A protein of the present disclosure binds to NKG2D with a K_{D} of 10 nM or weaker affinity.

In one aspect, the present disclosure provides multi-specific binding proteins that bind to the NKG2D receptor and CD16 receptor on natural killer cells, and the antigen EGFR. Table 2 lists some exemplary sequences of heavy chain variable domains and light chain variable domains that, in combination, can bind to EGFR.

| Table 2 | | |
|---|---|---|
| Clones | Heavy chain variable domain amino acid sequence | Light chain variable domain amino acid sequence |
| Cetuximab | QVQLKQSGPGLVQPSQSLSITCTVS GFSLTNYGVHWVRQSPGKGLEWL GVIWSGGNTDYNTPFTSRLSINKD NSKSQVFFKMNSLQSNDTAIYYCA RALTYYDYEFAYWGQGTLVTVSA A (SEQ ID NO:217) | DILLTQSPVILSVSPGERVSFSCRAS QSIGTNIHWYQQRTNGSPRLLIKY ASESISGIPSRFSGSGSGTDFTLSINS VESEDIADYYCQQNNNWPTTFGA GTKLELKR (SEQ ID NO:109) CDR1(SEQ ID NO:110) - |
| | CDR1 (SEQ ID NO:218) - NYGVH | RASQSIGTNIH |
| | CDR2 (SEQ ID NO:219) - | CDR2 (SEQ ID NO:111) - YASESIS |
| | IWSGGNTDYN | CDR3 (SEQ ID NO:112) - |
| | CDR3 (SEQ ID NO:220) - | QQNNNWPTT |
| | ALTYYDYEFAY | |
| Panitumumab | QVQLQESGPGLVKPSETLSLTCTV SGGSVSSGDYYWTWIRQSPGKGL EWIGHIYYSGNTNYNPSLKSRLTIS IDTSKTQFSLKLSSVTAADTAIYYC VRDRVTGAFDIWGQGTMVTVSSA (SEQ ID NO:113) | DIQMTQSPSSLSASVGDRVTITCQA SQDISNYLNWYQQKPGKAPKLLIY DASNLETGVPSRFSGSGSGTDFTFT ISSLQPEDIATYFCQHFDHLPLAFG GGTKVEIKR (SEQ ID NO:117) |
| | CDR1 (SEQ ID NO:114) - | CDR1 (SEQ ID NO:118) - |
| | SGDYYWT | QASQDISNYLN |
| | CDR2 (SEQ ID NO:115) - | CDR2 (SEQ ID NO:119) - DASNLET |
| | HIYYSGNTNYNPSLKS | CDR3 (SEQ ID NO:120) - |
| | CDR3 (SEQ ID NO:116) - | QHFDHLPLA |
| | DRVTGAFDI | |
| Necitumumab | QVQLQESGPGLVKPSQTLSLTCTV SGGSISSGDYYWSWIRQPPGKGLE WIGYIYYSGSTDYNPSLKSRVTMS VDTSKNQFSLKVNSVTAADTAVY | EIVMTQSPATLSLSPGERATLSCRA SQSVSSYLAWYQQKPGQAPRLLIY DASNRATGIPARFSGSGSGTDFTLT ISSLEPEDFAVYYCHQYGSTPLTFG GGTKAEIKR |
| | YCARVSIFGVGTFDYWGQGTLVT VSSA (SEQ ID NO:121) | (SEQ ID NO:125) |
| | | CDR1 (SEQ ID NO:126) - |
| | | RASQSVSSYLA |
| | CDR1 (SEQ ID NO:122) - | CDR2 (SEQ ID NO:127) - DASNRAT |
| | SGDYYWS | CDR3 (SEQ ID NO:128) - |
| | CDR2 (SEQ ID NO:123) - | HQYGSTPLT |
| | YIYYSGSTDYNPSLKS | |
| | CDR3 (SEQ ID NO:124) - | |
| | VSIFGVGTFDY | |
| Zalutumumab |  (SEQ ID NO:129) |  (SEQ ID NO:133) |
| | CDR1 (SEQ ID NO:130) - GFTFSTY | CDR1 (SEQ ID NO:140) - QDISSALV |
| | CDR2 (SEQ ID NO:131) - WDDGSY | CDR2 (SEQ ID NO:141) - DASSLES |
| | CDR3 (SEQ ID NO:132) - | CDR3 (SEQ ID NO:142) - |
| | DGITMVRGVMKDYFDY | QQFNSYPLT |
| Matuzumab | QVQLVQSGAEVKKPGASVKVSCK ASGYTFTSHWMHWVRQAPGQGL EWIGEFNPSNGRTNYNEKFKSKAT MTVDTSTNTAYMELSSLRSEDTAV YYCASRDYDYAGRYFDYWGQGT LVTVSSA (SEQ ID NO:143) | DIQMTQSPSSLSASVGDRVTITCSA SSSVTYMYWYQQKPGKAPKLLIY DTSNLASGVPSRFSGSGSGTDYTFT ISSLQPEDIATYYCQQWSSHIFTFG QGTKVEIKR (SEQ ID NO:147) |
| | | CDR1 (SEQ ID NO:148) - |
| | CDR1 (SEQ ID NO:144) - GYTFTSH | SSVTYMY |
| | CDR2 (SEQ ID NO:145) - NPSNGR | CDR2 (SEQ ID NO:149) - DTSNLAS |
| | CDR3 (SEQ ID NO:146) - | CDR3 (SEQ ID NO:150) - |
| | RDYDYAGRYFDY | QQWSSHIFT |

Additional exemplary sequences of heavy chain variable domains and light chain variable domains that, in combination, can bind to EGFR are provided below (CDRs are underlined)
**P1X**
   > Gm_CA17P1X_HC (SEQ ID NO:151) 
QVQLVQSGAEVKKPGSSVKVSCKASGGTFSSYAISWVRQAPGQGLEWMGSII
PIFGTVNYAQKFQGRVTITADESTSTAYMELSSLRSEDTAVYYCARDPSVNLY
WYFDLWGRGTLVTVSS
      CDR1 (SEQ ID NO:221): SYAIS
      CDR2 (SEQ ID NO:222): SIIPIFGTVNYAQKFQG
      CDR3 (SEQ ID NO:223): DPSVNLYWYFDL
   > Gm_CA17P1X_LC (SEQ ID NO:152) 
DIQMTQSPSTLSASVGDRVTITCRASQSISSWWAWYQQKPGKAPKLLIYDASS
LESGVPSRFSGSGSGTEFTLTISSLQPDDFATYYCQQYHAHPTTFGGGTKVEIK
      CDR1 (SEQ ID NO:224): RASQSISSWWA
      CDR2 (SEQ ID NO:225): DASSLES
      CDR3 (SEQ ID NO:226): QQYHAHPTT
P2X
   > Gm_CA17P2X HC (SEQ ID NO:153) 
QVQLVQSGAEVKKPGSSVKVSCKASGGTFGSYAISWVRQAPGQGLEWMGSII
PIFGAANPAQKSQGRVTITADESTSTAYMELSSLRSEDTAVYYCAKMGRGKV
AFDIWGQGTMVTVSS
      CDR1 (SEQ ID NO:227): SYAIS
      CDR2 (SEQ ID NO:228): SIIPIFGAANPAQKSQG
      CDR3 (SEQ ID NO:229): MGRGKVAFDI
      > Gm_CA17P2X LC (SEQ ID NO:154) 
DIVMTQSPDSLAVSLGERATINCKSSQSVLYSPNNKNYLAWYQQKPGQPPKL
LIYWASTRESGVPDRFSGSGSGTDFTLTISSLQAEDVAVYYCQQYYGSPITFG
GGTKVEIK
      CDR1 (SEQ ID NO:230): KSSQSVLYSPNNKNYLA
      CDR2 (SEQ ID NO:231): WASTRES
      CDR3 (SEQ ID NO:232): QQYYGSPIT
**Panitumumab**
   > WT_CA17Pan_HC (SEQ ID NO:155)
      CDR1 (SEQ ID NO:233): SGDYYWT
      CDR2 (SEQ ID NO:234): HIYYSGNTNYNPSLKS
      CDR3 (SEQ ID NO:235): DRVTGAFDI
   > WT_CA17Pan_LC (SEQ ID NO:156)
      CDR1 (SEQ ID NO:236): QASQDISNYLN
      CDR2 (SEQ ID NO:237): DASNLET
      CDR3 (SEQ ID NO:238): QHFDHLPLA
**AdiCLC2**
   > WT_CA17AdiCLC2_HC (SEQ ID NO:157) 
QVQLQESGPGLVKPSETLSLTCTVSGGSVSSGSYYWSWIRQPPGKGLEWIGYI
YYSGSTNYNPSLKSRVTISVDTSKNQFSLKLSSVTAADTAVYYCARTNLYSTP
FDIWGQGTMVTVSS
      CDR1 (SEQ ID NO:239): SGSYYWS
      CDR2 (SEQ ID NO:240): YIYYSGSTNYNPSLKS
      CDR3 (SEQ ID NO:241): TNLYSTPFDI
   > WT_CA17AdiCLC2_LC (SEQ ID NO:158) 
DIQLTQSPSSVSASVGDRVTITCRASQDISSWLAWYQQKPGKAPKLLIYAASS
LQSGVPSRFSGSGSGTDFTLTISSLQPEDFATYYCQQEHDFPWTFGGGTKVEIK
      CDR1 (SEQ ID NO:242): RASODISSWLA
      CDR2 (SEQ ID NO:243): AASSLQS
      CDR3 (SEQ ID NO:244): QQEHDFPWT
**Necitumumab**
   >Necitumumab_HC (SEQ ID NO:159)
      CDR1 (SEQ ID NO:245): SGDYYWS
      CDR2 (SEQ ID NO:246): YIYYSGSTDYNPSLKS
      CDR3 (SEQ ID NO:247): VSIFGVGTFDY
   >Necitumumab_LC (SEQ ID NO:160)
      CDR1 (SEQ ID NO:248): RASQSVSSYLA
      CDR2 (SEQ ID NO:249): DASNRAT
      CDR3 (SEQ ID NO:250): HQYGSTPLT
**Cetuximab**
   >Cetuximab HC (SEQ ID NO:161) 
QVQLKQSGPGLVQPSQSLSITCTVSGFSLTNYGVHWVRQSPGKGLEWLGVIW
SGGNTDYNTPFTSRLSINKDNSKSQVFFKMNSLQSNDTAIYYCARALTYYDY
EFAYWGQGTLVTVSA
      CDR1 (SEQ ID NO:251): NYGVH
      CDR2 (SEQ ID NO:252): IWSGGNTDYN
      CDR3 (SEQ ID NO:253): ALTYYDYEFAY
   >Cetuximab_LC (SEQ ID NO:162) 
DILLTQSPVILSVSPGERVSFSCRASQSIGTNIHWYQQRTNGSPRLLIKYASESIS
GIPSRFSGSGSGTDFTLSINSVESEDIADYYCQQNNNWPTTFGGGTKLELK
      CDR1 (SEQ ID NO:254): RASOSIGTNIH
      CDR2 (SEQ ID NO:255): YASESIS
      CDR3 (SEQ ID NO:256): QQNNNWPTT
**AdiCLC3**
   > WT_CA17AdiCLC3_HC (SEQ ID NO:163) 
QVQLQESGPGLVKPSETLSLTCTVSGGSVNSGDYYWSWIRQPPGKGLEWIGYI
YYSGSTNYNPSLKSRVTISVDTSKNQFSLKLSSVTAADTAVYYCARTNLYSTP
FDIWGQGTMVTVSS
      CDR1 (SEQ ID NO:257): SGDYYWS
      CDR2 (SEQ ID NO:258): YIYYSGSTNYNPSLKS
      CDR3 (SEQ ID NO:259): TNLYSTPFDI
   > WT_CA17AdiCLC3_LC (SEQ ID NO:164) 
DIQMTQSPSTLSASVGDRVTITCRASQSISSWLAWYQQKPGKAPKLLIYDASS
LESGVPSRFSGSGSGTEFTLTISSLQPDDFATYYCHQYQSYSWTFGGGTKVEIK
      CDR1 (SEQ ID NO:260): RASOSISSWLA
      CDR2 (SEQ ID NO:261): DASSLES
      CDR3 (SEQ ID NO:262): HQYQSYSWT

Some TriNKETs of the present disclosure are in the form A49-F3'-TriNKET-EGFR, sequences of which are provided below (CDRs (Kabat numbering) are underlined).

An A49-F3'-TriNKET-EGFR includes a single-chain variable fragment (scFv) that binds EGFR (SEQ ID NOs: 264, 272, 265, 273, 274, and 266 are exemplary sequences of such EGFR-binding scFv polypeptides), linked to an Fc domain via a hinge comprising Ala-Ser *(e.g.,* SEQ ID NO:267); and an NKG2D-binding Fab fragment ("A49") including a heavy chain portion comprising an heavy chain variable domain (SEQ ID NO:85) and a CH1 domain, and a light chain portion comprising a light chain variable domain (SEQ ID NO:86) and a light chain constant domain, wherein the heavy chain variable domain is connected to the CH1 domain, and the CH1 domain is connected to the Fc domain.

An EGFR-binding scFv of the present disclosure can include a heavy chain variable domain of necitumumab, panitumumab, or AdiCLC2 connected to a light chain variable domain of necitumumab, panitumumab, or AdiCLC2 with a (G4S)₄ linker (represented as V_{L}(G4S)₄V_{H} or LH where V_{L} is N-terminal to V_{H}, and represented as V_{H}(G4S)₄V_{L} or HL where V_{H} is N-terminal to V_{L}). SEQ ID NOs: 264, 272, 265, 273, 274, and 266 are exemplary sequences of such EGFR-binding scFv polypeptides. The V_{L} and V_{H} of the necitumumab scFv (SEQ ID NO:264 or 272) contain 100V_{L} - 105V_{H} S-S bridge (resulting from G100C and Q105C substitutions, respectively) (cysteine residues are in bold-italics-underlined in the sequences below). The V_{L} and V_{H} of the panitumumab scFv (SEQ ID NO:265 or 273) contain 100V_{L} - 44V_{H} S-S bridge (resulting from G100C and G44C substitutions, respectively) (cysteine residues are in bold-italics-underlined in the sequences below). (G4S)₄ is the bolded-underlined sequence GGGGSGGGGSGGGGSGGGGS (SEQ ID NO:263) in, *e.g.,* SEQ ID NO:264.
***EGFR (neciLH) scFv (variable domains derived from necitumumab)*** 
EIVMTQSPATLSLSPGERATLSCRASQSVSSYLAWYQQKPGQAPRLLIYDASNRATGI
PARFSGSGSGTDFTLTISSLEPEDFAVYYCHQYGSTPLTFGCGTKAEIK
GGGGSGGGGSGGGGSGGGGS
QVQLQESGPGLVKPSQTLSLTCTVSGGSISSGDYYWSWIRQPPGKGLEWIGYIYYSGS
TDYNPSLKSRVTMSVDTSKNQFSLKVNSVTAADTAVYYCARVSIFGVGTFDYWGCG
TLVTVSS (SEQ ID NO:264)
***EGFR (neciHL) scFv (variable domains derived from necitumumab)*** 
QVQLQESGPGLVKPSQTLSLTCTVSGGSISSGDYYWSWIRQPPGKGLEWIGYIYYSGS
TDYNPSLKSRVTMSVDTSKNQFSLKVNSVTAADTAVYYCARVSIFGVGTFDYWGCG
TLVTVSS
GGGGSGGGGSGGGGSGGGGS
EIVMTQSPATLSLSPGERATLSCRASQSVSSYLAWYQQKPGQAPRLLIYDASNRATGI
PARFSGSGSGTDFTLTISSLEPEDFAVYYCHQYGSTPLTFGCGTKAEIK
(SEQ ID NO:272)
***EGFR (panLH) scFv (variable domains derived from panitumumab)*** 
DIQMTQSPSSLSASVGDRVTITCQASQDISNYLNWYQQKPGKAPKLLIYDASNLETG
VPSRFSGSGSGTDFTFTISSLQPEDIATYFCQHFDHLPLAFGCGTKVEIK
GGGGSGGGGSGGGGSGGGGS
QVQLQESGPGLVKPSETLSLTCTVSGGSVSSGDYYWTWIRQSPGKCLEWIGHIYYSG
NTNYNPSLKSRLTISIDTSKTQFSLKLSSVTAADTAIYYCVRDRVTGAFDIWGQGTMV
TVSS (SEQ ID NO:265)
***EGFR (panHL) scFv (variable domains derived from panitumumab)*** 
QVQLQESGPGLVKPSETLSLTCTVSGGSVSSGDYYWTWIRQSPGKCLEWIGHIYYSG
NTNYNPSLKSRLTISIDTSKTQFSLKLSSVTAADTAIYYCVRDRVTGAFDIWGQGTMV
TVSS
GGGGSGGGGSGGGGSGGGGS
DIQMTQSPSSLSASVGDRVTITCQASQDISNYLNWYQQKPGKAPKLLIYDASNLETG
VPSRFSGSGSGTDFTFTISSLQPEDIATYFCQHFDHLPLAFGCGTKVEIK
(SEQ ID NO:273)
***EGFR (adiCLC2LH) scFv (variable domains derived from AdiCLC2)*** 
DIQLTQSPSSVSASVGDRVTITCRASQDISSWLAWYQQKPGKAPKLLIYAASSLQSGV
PSRFSGSGSGTDFTLTISSLQPEDFATYYCQQEHDFPWTFGGGTKVEIK
GGGGSGGGGSGGGGSGGGGS
QVQLQESGPGLVKPSETLSLTCTVSGGSVSSGSYYWSWIRQPPGKGLEWIGYIYYSGS
TNYNPSLKSRVTISVDTSKNQFSLKLSSVTAADTAVYYCARTNLYSTPFDIWGQGTM
VTVSS (SEQ ID NO:274)
***EGFR (adiCLC2HL) scFv (variable domains derived from AdiCLC2)***

QVQLQESGPGLVKPSETLSLTCTVSGGSVSSGSYYWSWIRQPPGKGLEWIGYIYYSGS
TNYNPSLKSRVTISVDTSKNQFSLKLSSVTAADTAVYYCARTNLYSTPFDIWGQGTM
VTVSSGGGGSGGGGSGGGGSGGGGS
DIQLTQSPSSVSASVGDRVTITCRASQDISSWLAWYQQKPGKAPKLLIYAASSLQSGV
PSRFSGSGSGTDFTLTISSLQPEDFATYYCQQEHDFPWTFGGGTKVEIK (SEQ ID
NO:266)

SEQ ID NO:267, SEQ ID NO:275, SEQ ID NO:268, SEQ ID NO: 276, SEQ ID NO:269, and SEQ ID NO:277 represent six sequences of an EGFR-binding scFv linked to an Fc domain via a hinge comprising Ala-Ser (scFv-Fc). The Fc domain linked to the scFv includes Q347R, D399V, and F405T substitutions.
***EGFR (neciLH) scFv-Fc*** 
EIVMTQSPATLSLSPGERATLSCRASQSVSSYLAWYQQKPGQAPRLLIYDASNRATGI
PARFSGSGSGTDFTLTISSLEPEDFAVYYCHQYGSTPLTFGCGTKAEIK
GGGGSGGGGSGGGGSGGGGS
QVQLQESGPGLVKPSQTLSLTCTVSGGSISSGDYYWSWIRQPPGKGLEWIGYIYYSGS
TDYNPSLKSRVTMSVDTSKNQFSLKVNSVTAADTAVYYCARVSIFGVGTFDYWGCG
TLVTVSS
AS
 
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPRVYTLPPCRDELTKNQVSLTCLVKGFYPSDIAVEWESNGQPENN
YKTTPPVLVSDGSFTLYSKLTVDKSRWQQGNVFSCSVMHEALHNHYTQKSLSLSPG

(SEQ ID NO:267)
***EGFR (neciHL) scFv-Fc*** 
QVQLQESGPGLVKPSQTLSLTCTVSGGSISSGDYYWSWIRQPPGKGLEWIGYIYYSGS
TDYNPSLKSRVTMSVDTSKNQFSLKVNSVTAADTAVYYCARVSIFGVGTFDYWGCG
TLVTVSS
GGGGSGGGGSGGGGSGGGGS
EIVMTQSPATLSLSPGERATLSCRASQSVSSYLAWYQQKPGQAPRLLIYDASNRATGI
PARFSGSGSGTDFTLTISSLEPEDFAVYYCHQYGSTPLTFGCGTKAEIK
AS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPRVYTLPPCRDELTKNQVSLTCLVKGFYPSDIAVEWESNGQPENN
YKTTPPVLVSDGSFTLYSKLTVDKSRWQQGNVFSCSVMHEALHNHYTQKSLSLSPG

(SEQ ID NO:275)
***EGFR (panLH) scFv-Fc*** 
DIQMTQSPSSLSASVGDRVTITCQASQDISNYLNWYQQKPGKAPKLLIYDASNLETG
VPSRFSGSGSGTDFTFTISSLQP EDIATYFCQHFDHLPLAFGCGTKVEIK
GGGGSGGGGSGGGGSGGGGS
QVQLQESGPGLVKPSETLSLTCTVSGGSVSSGDYYWTWIRQSPGKCLEWIGHIYYSG
NTNYNPSLKSRLTISIDTSKTQFSLKLSSVTAADTAIYYCVRDRVTGAFDIWGQGTMV
TVSS
AS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPRVYTLPPCRDELTKNQVSLTCLVKGFYPSDIAVEWESNGQPENN
YKTTPPVLVSDGSFTLYSKLTVDKSRWQQGNVFSCSVMHEALHNHYTQKSLSLSPG

(SEQ ID NO:268)
***EGFR (panHL) scFv-Fc*** 
QVQLQESGPGLVKPSETLSLTCTVSGGSVSSGDYYWTWIRQSPGKCLEWIGHIYYSG
NTNYNPSLKSRLTISIDTSKTQFSLKLSSVTAADTAIYYCVRDRVTGAFDIWGQGTMV
TVSS
GGGGSGGGGSGGGGSGGGGS
DIQMTQSPSSLSASVGDRVTITCQASQDISNYLNWYQQKPGKAPKLLIYDASNLETG
VPSRFSGSGSGTDFTFTISSLQPEDIATYFCQHFDHLPLAFGCGTKVEIK
AS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
 
KTISKAKGQPREPRVYTLPPCRDELTKNQVSLTCLVKGFYPSDIAVEWESNGQPENN
YKTTPPVLVSDGSFTLYSKLTVDKSRWQQGNVFSCSVMHEALHNHYTQKSLSLSPG

(SEQ ID NO:276)
***EGFR (adiCLC2LH) scFv (variable domains derived from AdiCLC2)*** 
DIQLTQSPSSVSASVGDRVTITCRASQDISSWLAWYQQKPGKAPKLLIYAASSLQSGV
PSRFSGSGSGTDFTLTISSLQPEDFATYYCQQEHDFPWTFGGGTKVEIK
GGGGSGGGGSGGGGSGGGGS
QVQLQESGPGLVKPSETLSLTCTVSGGSVSSGSYYWSWIRQPPGKGLEWIGYIYYSGS
TNYNPSLKSRVTISVDTSKNQFSLKLSSVTAADTAVYYCARTNLYSTPFDIWGQGTM
VTVSS
AS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPRVYTLPPCRDELTKNQVSLTCLVKGFYPSDIAVEWESNGQPENN
YKTTPPVLVSDGSFTLYSKLTVDKSRWQQGNVFSCSVMHEALHNHYTQKSLSLSPG

(SEQ ID NO:277)
***EGFR (adiCLC2HL) scFv-Fc***

QVQLQESGPGLVKPSETLSLTCTVSGGSVSSGSYYWSWIRQPPGKGLEWIGYIYYSGS
TNYNPSLKSRVTISVDTSKNQFSLKLSSVTAADTAVYYCARTNLYSTPFDIWGQGTM
VTVSS
GGGGSGGGGSGGGGSGGGGS
DIQLTQSPSSVSASVGDRVTITCRASQDISSWLAWYQQKPGKAPKLLIYAASSLQSGV
PSRFSGSGSGTDFTLTISSLQPEDFATYYCQQEHDFPWTFGGGTKVEIK
AS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPRVYTLPPCRDELTKNQVSLTCLVKGFYPSDIAVEWESNGQPENN
YKTTPPVLVSDGSFTLYSKLTVDKSRWQQGNVFSCSVMHEALHNHYTQKSLSLSPG

(SEQ ID NO:269)

SEQ ID NO:270 represents the heavy chain portion of a Fab fragment, which comprises an heavy chain variable domain (SEQ ID NO:85) of an NKG2D-binding site and a CH1 domain, connected to an Fc domain. The Fc domain in SEQ ID NO:270 includes a Y349C substitution in the CH3 domain, which forms a disulfide bond with a S354C substitution on the Fc linked to the EGFR-binding scFv *(e.g.,* SEQ ID NO:264, SEQ ID NO:265, and SEQ ID NO:266). In SEQ ID NO:270, the Fc domain also includes K360E and K409W substitutions.
***A49*** - ***V_{H}*** 
EVQLVESGGGLVKPGGSLRLSCAASGFTFSSYSMNWVRQAPGKGLEWVSSISSSSSYI
YYADSVKGRFTISRDNAKNSLYLQMNSLRAEDTAVYYCARGAPMGAAAGWFDPW
GQGTLVTVSS (SEQ ID NO:85)
***A49 V_{H}-CH1-Fc***

EVQLVESGGGLVKPGGSLRLSCAASGFTFSSYSMNWVRQAPGKGLEWVSSISSSSSYI
YYADSVKGRFTISRDNAKNSLYLQMNSLRAEDTAVYYCARGAPMGAAAGWFDPW
GQGTLVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEK
TISKAKGQPREPQVCTLPPSRDELTENQVSLTCLVKGFYPSDIAVEWESNGQPENNY
KTTPPVLDSDGSFFLYSWLTVDKSRWQQGNVFSCSVMHEALHNHYTQKSLSLSPG
(SEQ ID NO:270)

SEQ ID NO:271 represents the light chain portion of a Fab fragment comprising a light chain variable domain (SEQ ID NO:86) of an NKG2D-binding site and a light chain constant domain.
***A49*** - ***V_{L}*** 
DIQMTQSPSSVSASVGDRVTITCRASQGISSWLAWYQQKPGKAPKLLIYAASSLQSG
VPSRFSGSGSGTDFTLTISSLQPEDFATYYCQQGVSFPRTFGGGTKVEIK (SEQ ID
NO:86)
***A49 LC V_{L} - Constant domain***

DIQMTQSPSSVSASVGDRVTITCRASQGISSWLAWYQQKPGKAPKLLIYAASSLQSG
VPSRFSGSGSGTDFTLTISSLQPEDFATYYCQQGVSFPRTFGGGTKVEIK
RTVAAPSPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID
NO:271)

In an exemplary embodiment, the Fc domain linked to the NKG2D-binding Fab fragment includes the mutations of Q347R, D399V, and F405T, and the Fc domain linked to the EGFR scFv comprises matching mutations K360E and K409W for forming a heterodimer. In an exemplary embodiment, the Fc domain linked to the NKG2D-binding Fab fragment includes a S354C substitution in the CH3 domain, which forms a disulfide bond with a Y349C substitution on the Fc linked to the EGFR-binding scFv.

Alternatively, novel antigen-binding sites that can bind to EGFR can be identified by screening for binding to the amino acid sequence defined by SEQ ID NO: 165.

SEQ ID NO:165

























Antigen-binding sites that can bind to HLA-E can be identified by screening for binding to the amino acid sequence defined by SEQ ID NO:166.

SEQ ID NO:166














Table 3 lists peptide sequences of heavy chain variable domains and light chain variable domains that, in combination, can bind to PD-L1.

| Table 3 | | |
|---|---|---|
| Clones | Heavy chain variable domain amino acid sequence | Light chain variable domain amino acid sequence |
| Durvalumab | EVQLVESGGGLVQPGGSLRLSCAA SGFTFSRYWMSWVRQAPGKGLE WVANIKQDGSEKYYVDSVKGRFT ISRDNAKNSLYLQMNSLRAEDTAV YYCAREGGWFGELAFDYWGQGT LVTVSS (SEQ ID NO:167) | EIVLTQSPGTLSLSPGERATLSCRA SQRVSSSYLAWYQQKPGQAPRLLI YDASSRATGIPDRFSGSGSGTDFTL TISRLEPEDFAVYYCQQYGSLPWT FGQGTKVEIKR (SEQ ID NO:171) |
| | CDR1 (SEQ ID NO:168) - GFTFSRY | CDR1(SEQ ID NO:172) - QRVSSSYLA |
| | CDR2 (SEQ ID NO:169) - KQDGSE | CDR2 (SEQ ID NO:173) - DASSRAT |
| | CDR3 (SEQ ID NO:170) - EGGWFGELAFDY | CDR3 (SEQ ID NO:174) - QQYGSLPWT |
| Atezolizumab | EVQLVESGGGLVQPGGSLRLSCAA SGFTFSDSWIHWVRQAPGKGLEW VAWISPYGGSTYYADSVKGRFTIS ADTSKNTAYLQMNSLRAEDTAVY YCARRHWPGGFDYWGQGTLVTV SSA (SEQ ID NO:175) | DIQMTQSPSSLSASVGDRVTITCRA SQDVSTAVAWYQQKPGKAPKLLI YSASFLYSGVPSRFSGSGSGTDFTL TISSLQPEDFATYYCQQYLYHPATF GQGTKVEIKR (SEQ ID NO:179) |
| | CDR1 (SEQ ID NO:176) - GFTFSDS | CDR1 (SEQ ID NO:180)-QDVSTAVA |
| | CDR2 (SEQ ID NO:177) - SPYGGS | CDR2 (SEQ ID NO:181) - SASFLYS |
| | CDR3 (SEQ ID NO:178) - RHWPGGFDY | CDR3 (SEQ ID NO:182) - QQYLYHPAT |
| Avelumab | EVQLLESGGGLVQPGGSLRLSCAA SGFTFSSYIMMWVRQAPGKGLEW | QSALTQPASVSGSPGQSITISCTGTS SDVGGYNYVSWYQQHPGKAPKL |
| | VSSIYPSGGITFYADTVKGRFTISR DNSKNTLYLQMNSLRAEDTAVYY CARIKLGTVTTVDYWGQGTLVTV SSA (SEQ ID NO:183) | MIYDVSNRPSGVSNRFSGSKSGNT ASLTISGLQAEDEADYYCSSYTSSS TRVFGTGTKVTVLG (SEQ ID NO:187) |
| | CDR1 (SEQ ID NO:184) - GFTFSSY | CDR1 (SEQ ID NO:188) - SSDVGGYNYVS |
| | CDR2 (SEQ ID NO:185) - YPSGGI | CDR2 (SEQ ID NO:189) - DVSNRPS |
| | CDR3 (SEQ ID NO:186) - IKLGTVTTVDY | CDR3 (SEQ ID NO:190) - SSYTSSSTRV |

Alternatively, novel antigen-binding sites that can bind to PD-L1 can be identified by screening for binding to the amino acid sequence defined by SEQ ID NO:191.

SEQ ID NO:191

MRIFAVFIFMTYWHLLNAFTVTVPKDLYVVEYGSNMTIECKFPVEKQLDLAA

LIVYWEMEDKNIIQFVHGEEDLKVQHSSYRQRARLLKDQLSLGNAALQITDV

KLQDAGVYRCMISYGGADYKRITVKVNAPYNKINQRILVVDPVTSEHELTCQ

AEGYPKAEVIWTSSDHQVLSGKTTTTNSKREEKLFNVTSTLRINTTTNEIFYCT

FRRLDPEENHTAELVIPELPLAHPPNERTHLVILGAILLCLGVALTFIFRLRKGR

MMDVKKCGIQDTNSKKQSDTHLEET

Table 4 lists peptide sequences of heavy chain variable domains and light chain variable domains that, in combination, can bind to CCR4.

| Table 4 | | |
|---|---|---|
| Clones | Heavy chain variable domain amino acid sequence | Light chain variable domain amino acid sequence |
| anti-CCR4 (WO20050355 82) | EVQLVESGGDLVQPGRSLRLSCAA SGFIFSNYGMSWVRQAPGKGLEW VATISSASTYSYYPDSVKGRFTISR DNAKNSLYLQMNSLRVEDTALYY CGRHSDGNFAFGYWGQGTLVTVS SA | DVLMTQSPLSLPVTPGEPASISCRS SRNIVHINGDTYLEWYLQKPGQSP QLLIYKVSNRFSGVPDRFSGSGSGT DFTLKISRVEAEDVGVYYCFQGSL LPWTFGQGTKVEIKR (SEQ ID NO:196) |
| | (SEQ ID NO:192) CDR1 (SEQ ID NO:193) - GFIFSNY | CDR1(SEQ ID NO:197) - RNIVHINGDTYLE |
| | CDR2 (SEQ ID NO:194) - SSASTY | CDR2 (SEQ ID NO:198) - KVSNRFS |
| | CDR3 (SEQ ID NO:195)-HSDGNFAFGY | CDR3 (SEQ ID NO:199) - FQGSLLPWT |
| anti-CCR4 (U.S. Patent No. 8,895,007) | QVQLVQSGAEVKKPGSSVKVSCK ASEGTFSSYAMSWVRQAPGQGLE WMGGIIPIFGTVNYAQKFQGRVTM TRDTSTSTVYMELSSLRSDDTAVY YCARRRGAKFDYWGQGTLVTVSS (SEQ ID NO:200) | SYVLTQPPSASGTPGQSVTISCSGS TSNIGSHYVVWYQQLPGTAPRLLI YRNHQRPSGVPDRLSGSKSGTSAS LAIGGLRSEDEADYYCAVWDDTL SGWVFGGGTKLTVL (SEQ ID NO:204) |
| | CDR1 (SEQ ID NO:201) - SYAMS | CDR1 (SEQ ID N0:205) - SGSTSNIGSHYVV |
| | CDR2 (SEQ ID NO:202) - GIIPIFGTVNYAQKFQ | |
| | | CDR2 (SEQ ID NO:206) - RNHQRPS |
| | CDR3 (SEQ ID NO:203) - RRGAKFDY | CDR3 (SEQ ID NO:207) - AVWDDTLSGWV |
| anti-CCR4 (U.S. Patent No. 9,441,045) | QVQLVQSGAEVKKPGASVKVSCK ASGYTFASQWMHWMRQAPGQGL EWIGWINPGNVNTKYNEKFKGRA TLTVDTSTNTAYMELSSLRSEDTA VYYCARSTWYRPLDYWGQGTLV TVSS (SEQ ID NO:208) | DIVMTQSPDSLAVSLGERATINCKS SQSILYSSNQKNYLAWYQQKPGQS PKLLIYWASTRESGVPDRFSGSGS GTDFTLTISSLQAEDVAVYYCHQY ISSYTFGQGTKLEIK (SEQ ID NO:212) |
| | CDR1 (SEQ ID NO:209) - GYTFASQW | CDR1 (SEQ ID NO:213) - QSILYSSNQKNY |
| | | CDR2 (SEQ ID NO:214) - WASTRE |
| | CDR2 (SEQ ID NO:210) - INPGNVNT | CDR3 (SEQ ID NO:215) - HQYISSYT |
| | CDR3 (SEQ ID NO:211) - STWYRPLDY | |

Alternatively, novel antigen-binding sites that can bind to CCR4 can be identified by screening for binding to the amino acid sequence defined by SEQ ID NO:216.

SEQ ID NO:216

MNPTDIADTTLDESIYSNYYLYESIPKPCTKEGIKAFGELFLPPLYSLVFVFGLL

GNSVVVLVLFKYKRLRSMTDVYLLNLAISDLLFVFSLPFWGYYAADQWVFG

LGLCKMISWMYLVGFYSGIFFVMLMSIDRYLAIVHAVFSLRARTLTYGVITSL

ATWSVAVFASLPGFLFSTCYTERNHTYCKTKYSLNSTTWKVLSSLEINILGLVI

PLGIMLFCYSMIIRTLQHCKNEKKNKAVKMIFAVVVLFLGFWTPYNIVLFLET

LVELEVLQDCTFERYLDYAIQATETLAFVHCCLNPIIYFFLGEKFRKYILQLFK

TCRGLFVLCQYCGLLQIYSADTPSSSYTQSTMDHDLHDAL

Within the Fc domain, CD16 binding is mediated by the hinge region and the CH2 domain. For example, within human IgG1, the interaction with CD16 is primarily focused on amino acid residues Asp 265 - Glu 269, Asn 297 - Thr 299, Ala 327 - Ile 332, Leu 234 - Ser 239, and carbohydrate residue N-acetyl-D-glucosamine in the CH2 domain (see, Sondermann et al., Nature, 406 (6793):267-273). Based on the known domains, mutations can be selected to enhance or reduce the binding affinity to CD 16, such as by using phage-displayed libraries or yeast surface-displayed cDNA libraries, or can be designed based on the known three-dimensional structure of the interaction.

The assembly of heterodimeric antibody heavy chains can be accomplished by expressing two different antibody heavy chain sequences in the same cell, which may lead to the assembly of homodimers of each antibody heavy chain as well as assembly of heterodimers. Promoting the preferential assembly of heterodimers can be accomplished by incorporating different mutations in the CH3 domain of each antibody heavy chain constant region as shown in US13/494870, US16/028850, US11/533709, US12/875015, US13/289934, US14/773418, US12/811207, US13/866756, US14/647480, and US14/830336. For example, mutations can be made in the CH3 domain based on human IgG1 and incorporating distinct pairs of amino acid substitutions within a first polypeptide and a second polypeptide that allow these two chains to selectively heterodimerize with each other. The positions of amino acid substitutions illustrated below are all numbered according to the EU index as in Kabat.

In one scenario, an amino acid substitution in the first polypeptide replaces the original amino acid with a larger amino acid, selected from arginine (R), phenylalanine (F), tyrosine (Y) or tryptophan (W), and at least one amino acid substitution in the second polypeptide replaces the original amino acid(s) with a smaller amino acid(s), chosen from alanine (A), serine (S), threonine (T), or valine (V), such that the larger amino acid substitution (a protuberance) fits into the surface of the smaller amino acid substitutions (a cavity). For example, one polypeptide can incorporate a T366W substitution, and the other can incorporate three substitutions including T366S, L368A, and Y407V.

An antibody heavy chain variable domain of the invention can optionally be coupled to an amino acid sequence at least 90% identical to an antibody constant region, such as an IgG constant region including hinge, CH2 and CH3 domains with or without CH1 domain. In some embodiments, the amino acid sequence of the constant region is at least 90% identical to a human antibody constant region, such as an human IgG1 constant region, an IgG2 constant region, IgG3 constant region, or IgG4 constant region. In some other embodiments, the amino acid sequence of the constant region is at least 90% identical to an antibody constant region from another mammal, such as rabbit, dog, cat, mouse, or horse. One or more mutations can be incorporated into the constant region as compared to human IgG1 constant region, for example at Q347, Y349, L351, S354, E356, E357, K360, Q362, S364, T366, L368, K370, N390, K392, T394, D399, S400, D401, F405, Y407, K409, T411 and/or K439. Exemplary substitutions include, for example, Q347E, Q347R, Y349S, Y349K, Y349T, Y349D, Y349E, Y349C, T350V, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, T394W, D399R, D399K, D399V, S400K, S400R, D401K, F405A, F405T, Y407A, Y407I, Y407V, K409F, K409W, K409D, T411D, T411E, K439D, and K439E.

In certain embodiments, mutations that can be incorporated into the CH1 of a human IgG1 constant region may be at amino acid V125, F126, P127, T135, T139, A140, F170, P171, and/or V173. In certain embodiments, mutations that can be incorporated into the Cκ of a human IgG1 constant region may be at amino acid E123, F116, S176, V163, S174, and/or T164.

Alternatively, amino acid substitutions could be selected from the following sets of substitutions shown in Table 5.

| Table 5 | | |
|---|---|---|
| | First Polypeptide | Second Polypeptide |
| Set 1 | S364E/F405A | Y349K/T394F |
| Set 2 | S364H/D401K | Y349T/T411E |
| Set 3 | S364H/T394F | Y349T/F405A |
| Set 4 | S364E/T394F | Y349K/F405A |
| Set 5 | S364E/T411E | Y349K/D401K |
| Set 6 | S364D/T394F | Y349K/F405A |
| Set 7 | S364H/F405A | Y349T/T394F |
| Set 8 | S364K/E357Q | L368D/K370S |
| Set 9 | L368D/K370S | S364K |
| Set 10 | L368E/K370S | S364K |
| Set 11 | K360E/Q362E | D401K |
| Set 12 | L368D/K370S | S364K/E357L |
| Set 13 | K370S | S364K/E357Q |
| Set 14 | F405L | K409R |
| Set 15 | K409R | F405L |

Alternatively, amino acid substitutions could be selected from the following sets of substitutions shown in Table 6.

| Table 6 | | |
|---|---|---|
| | First Polypeptide | Second Polypeptide |
| Set 1 | K409W | D399V/F405T |
| Set 2 | Y349S | E357W |
| Set 3 | K360E | Q347R |
| Set 4 | K360E/K409W | Q347R/D399V/F405T |
| Set 5 | Q347E/K360E/K409W | Q347R/D399V/F405T |
| Set 6 | Y349S/K409W | E357W/D399V/F405T |

Alternatively, amino acid substitutions could be selected from the following set of substitutions shown in Table 7.

| Table 7 | | |
|---|---|---|
| | First Polypeptide | Second Polypeptide |
| Set 1 | T366K/L351K | L351D/L368E |
| Set 2 | T366K/L351K | L351D/Y349E |
| Set 3 | T366K/L351K | L351D/Y349D |
| Set 4 | T366K/L351K | L351D/Y349E/L368E |
| Set 5 | T366K/L351K | L351D/Y349D/L368E |
| Set 6 | E356K/D399K | K392D/K409D |

Alternatively, at least one amino acid substitution in each polypeptide chain could be selected from Table 8.

| Table 8 | |
|---|---|
| First Polypeptide | Second Polypeptide |
| L351Y, D399R, D399K, S400K, S400R, Y407A, Y407I, Y407V | T366V, T366I, T366L, T366M, N390D, N390E, K392L, K392M, K392V, K392F K392D, K392E, K409F, K409W, T411D and T411E |

Alternatively, at least one amino acid substitutions could be selected from the following set of substitutions in Table 9, where the position(s) indicated in the First Polypeptide column is replaced by any known negatively-charged amino acid, and the position(s) indicated in the Second Polypeptide Column is replaced by any known positively-charged amino acid.

| Table 9 | |
|---|---|
| First Polypeptide | Second Polypeptide |
| K392, K370, K409, or K439 | D399, E356, or E357 |

Alternatively, at least one amino acid substitutions could be selected from the following set of in Table 10, where the position(s) indicated in the First Polypeptide column is replaced by any known positively-charged amino acid, and the position(s) indicated in the Second Polypeptide Column is replaced by any known negatively-charged amino acid.

| Table 10 | |
|---|---|
| First Polypeptide | Second Polypeptide |
| D399, E356, or E357 | K409, K439, K370, or K392 |

Alternatively, amino acid substitutions could be selected from the following set in Table 11.

| Table 11 | |
|---|---|
| First Polypeptide | Second Polypeptide |
| T350V, L351Y, F405A, and Y407V | T350V, T366L, K392L, and T394W |

Alternatively, or in addition, the structural stability of a hetero-multimeric protein may be increased by introducing S354C on either of the first or second polypeptide chain, and Y349C on the opposing polypeptide chain, which forms an artificial disulfide bridge within the interface of the two polypeptides.

In some embodiments, the amino acid sequence of one polypeptide chain of the antibody constant region differs from the amino acid sequence of an IgG1 constant region at position T366, and wherein the amino acid sequence of the other polypeptide chain of the antibody constant region differs from the amino acid sequence of an IgG1 constant region at one or more positions selected from the group consisting of T366, L368 and Y407.

In some embodiments, the amino acid sequence of one polypeptide chain of the antibody constant region differs from the amino acid sequence of an IgG1 constant region at one or more positions selected from the group consisting of T366, L368 and Y407, and wherein the amino acid sequence of the other polypeptide chain of the antibody constant region differs from the amino acid sequence of an IgG1 constant region at position T366.

In some embodiments, the amino acid sequence of one polypeptide chain of the antibody constant region differs from the amino acid sequence of an IgG1 constant region at one or more positions selected from the group consisting of E357, K360, Q362, S364, L368, K370, T394, D401, F405, and T411 and wherein the amino acid sequence of the other polypeptide chain of the antibody constant region differs from the amino acid sequence of an IgG1 constant region at one or more positions selected from the group consisting of Y349, E357, S364, L368, K370, T394, D401, F405 and T411.

In some embodiments, the amino acid sequence of one polypeptide chain of the antibody constant region differs from the amino acid sequence of an IgG1 constant region at one or more positions selected from the group consisting of Y349, E357, S364, L368, K370, T394, D401, F405 and T411 and wherein the amino acid sequence of the other polypeptide chain of the antibody constant region differs from the amino acid sequence of an IgG1 constant region at one or more positions selected from the group consisting of E357, K360, Q362, S364, L368, K370, T394, D401, F405, and T411.

In some embodiments, the amino acid sequence of one polypeptide chain of the antibody constant region differs from the amino acid sequence of an IgG1 constant region at one or more positions selected from the group consisting of L351, D399, S400 and Y407 and wherein the amino acid sequence of the other polypeptide chain of the antibody constant region differs from the amino acid sequence of an IgG1 constant region at one or more positions selected from the group consisting of T366, N390, K392, K409 and T411.

In some embodiments, the amino acid sequence of one polypeptide chain of the antibody constant region differs from the amino acid sequence of an IgG1 constant region at one or more positions selected from the group consisting of T366, N390, K392, K409 and T411 and wherein the amino acid sequence of the other polypeptide chain of the antibody constant region differs from the amino acid sequence of an IgG1 constant region at one or more positions selected from the group consisting of L351, D399, S400 and Y407.

In some embodiments, the amino acid sequence of one polypeptide chain of the antibody constant region differs from the amino acid sequence of an IgG1 constant region at one or more positions selected from the group consisting of Q347, Y349, K360, and K409, and wherein the amino acid sequence of the other polypeptide chain of the antibody constant region differs from the amino acid sequence of an IgG1 constant region at one or more positions selected from the group consisting of Q347, E357, D399 and F405.

In some embodiments, the amino acid sequence of one polypeptide chain of the antibody constant region differs from the amino acid sequence of an IgG1 constant region at one or more positions selected from the group consisting of Q347, E357, D399 and F405, and wherein the amino acid sequence of the other polypeptide chain of the antibody constant region differs from the amino acid sequence of an IgG1 constant region at one or more positions selected from the group consisting of Y349, K360, Q347 and K409.

In some embodiments, the amino acid sequence of one polypeptide chain of the antibody constant region differs from the amino acid sequence of an IgG1 constant region at one or more positions selected from the group consisting of K370, K392, K409 and K439, and wherein the amino acid sequence of the other polypeptide chain of the antibody constant region differs from the amino acid sequence of an IgG1 constant region at one or more positions selected from the group consisting of D356, E357 and D399.

In some embodiments, the amino acid sequence of one polypeptide chain of the antibody constant region differs from the amino acid sequence of an IgG1 constant region at one or more positions selected from the group consisting of D356, E357 and D399, and wherein the amino acid sequence of the other polypeptide chain of the antibody constant region differs from the amino acid sequence of an IgG1 constant region at one or more positions selected from the group consisting of K370, K392, K409 and K439.

In some embodiments, the amino acid sequence of one polypeptide chain of the antibody constant region differs from the amino acid sequence of an IgG1 constant region at one or more positions selected from the group consisting of L351, E356, T366 and D399, and wherein the amino acid sequence of the other polypeptide chain of the antibody constant region differs from the amino acid sequence of an IgG1 constant region at one or more positions selected from the group consisting of Y349, L351, L368, K392 and K409.

In some embodiments, the amino acid sequence of one polypeptide chain of the antibody constant region differs from the amino acid sequence of an IgG1 constant region at one or more positions selected from the group consisting of Y349, L351, L368, K392 and K409, and wherein the amino acid sequence of the other polypeptide chain of the antibody constant region differs from the amino acid sequence of an IgG1 constant region at one or more positions selected from the group consisting of L351, E356, T366 and D399.

In some embodiments, the amino acid sequence of one polypeptide chain of the antibody constant region differs from the amino acid sequence of an IgG1 constant region by an S354C substitution and wherein the amino acid sequence of the other polypeptide chain of the antibody constant region differs from the amino acid sequence of an IgG1 constant region by a Y349C substitution.

In some embodiments, the amino acid sequence of one polypeptide chain of the antibody constant region differs from the amino acid sequence of an IgG1 constant region by a Y349C substitution and wherein the amino acid sequence of the other polypeptide chain of the antibody constant region differs from the amino acid sequence of an IgG1 constant region by an S354C substitution.

In some embodiments, the amino acid sequence of one polypeptide chain of the antibody constant region differs from the amino acid sequence of an IgG1 constant region by K360E and K409W substitutions and wherein the amino acid sequence of the other polypeptide chain of the antibody constant region differs from the amino acid sequence of an IgG1 constant region by O347R, D399V and F405T substitutions.

In some embodiments, the amino acid sequence of one polypeptide chain of the antibody constant region differs from the amino acid sequence of an IgG1 constant region by O347R, D399V and F405T substitutions and wherein the amino acid sequence of the other polypeptide chain of the antibody constant region differs from the amino acid sequence of an IgG1 constant region by K360E and K409W substitutions.

In some embodiments, the amino acid sequence of one polypeptide chain of the antibody constant region differs from the amino acid sequence of an IgG1 constant region by a T366W substitutions and wherein the amino acid sequence of the other polypeptide chain of the antibody constant region differs from the amino acid sequence of an IgG1 constant region by T366S, T368A, and Y407V substitutions.

In some embodiments, the amino acid sequence of one polypeptide chain of the antibody constant region differs from the amino acid sequence of an IgG1 constant region by T366S, T368A, and Y407V substitutions and wherein the amino acid sequence of the other polypeptide chain of the antibody constant region differs from the amino acid sequence of an IgG1 constant region by a T366W substitution.

In some embodiments, the amino acid sequence of one polypeptide chain of the antibody constant region differs from the amino acid sequence of an IgG1 constant region by T350V, L351Y, F405A, and Y407V substitutions and wherein the amino acid sequence of the other polypeptide chain of the antibody constant region differs from the amino acid sequence of an IgG1 constant region by T350V, T366L, K392L, and T394W substitutions.

In some embodiments, the amino acid sequence of one polypeptide chain of the antibody constant region differs from the amino acid sequence of an IgG1 constant region by T350V, T366L, K392L, and T394W substitutions and wherein the amino acid sequence of the other polypeptide chain of the antibody constant region differs from the amino acid sequence of an IgG1 constant region by T350V, L351Y, F405A, and Y407V substitutions.

The multi-specific proteins described above can be made using recombinant DNA technology well known to a skilled person in the art. For example, a first nucleic acid sequence encoding the first immunoglobulin heavy chain can be cloned into a first expression vector; a second nucleic acid sequence encoding the second immunoglobulin heavy chain can be cloned into a second expression vector; a third nucleic acid sequence encoding the immunoglobulin light chain can be cloned into a third expression vector; and the first, second, and third expression vectors can be stably transfected together into host cells to produce the multimeric proteins.

To achieve the highest yield of the multi-specific protein, different ratios of the first, second, and third expression vector can be explored to determine the optimal ratio for transfection into the host cells. After transfection, single clones can be isolated for cell bank generation using methods known in the art, such as limited dilution, ELISA, FACS, microscopy, or Clonepix.

Clones can be cultured under conditions suitable for bio-reactor scale-up and maintained expression of the multi-specific protein. The multispecific proteins can be isolated and purified using methods known in the art including centrifugation, depth filtration, cell lysis, homogenization, freeze-thawing, affinity purification, gel filtration, ion exchange chromatography, hydrophobic interaction exchange chromatography, and mixed-mode chromatography.

### II. CHARACTERISTICS OF THE MULTI-SPECIFIC PROTEINS

The multi-specific proteins described herein include an NKG2D-binding site, a CD16-binding site, and an EGFR, HLA-E, CCR4, or PD-L1-binding site. In some embodiments, the multi-specific proteins bind simultaneously to cells expressing NKG2D and/or CD16, such as NK cells, and to tumor cells expressing EGFR, HLA-E, CCR4, or PD-L1. Binding of the multi-specific proteins to NK cells can enhance the activity of the NK cells toward destruction of the tumor cells.

In some embodiments, the multi-specific proteins bind to EGFR, HLA-E, CCR4, or PD-L1 with a similar affinity to the corresponding EGFR, HLA-E, CCR4, or PD-L1 monoclonal antibody (*i.e.,* a monoclonal antibody containing the same EGFR, HLA-E, CCR4, or PD-L1-binding site as the one incorporated in the multi-specific proteins) In some embodiments, the multi-specific proteins are more effective in killing the tumor cells expressing EGFR, HLA-E, CCR4, or PD-L1 than the corresponding EGFR, HLA-E, CCR4, or PD-L1 monoclonal antibodies.

In certain embodiments, the multi-specific proteins described herein, which include an NKG2D-binding site and a binding site for EGFR, HLA-E, CCR4, or PD-L1, activate primary human NK cells when co-culturing with cells expressing EGFR, HLA-E, CCR4, or PD-L1. NK cell activation is marked by the increase in CD107a degranulation and IFN-γ cytokine production. Furthermore, compared to a corresponding EGFR, HLA-E, CCR4, or PD-L1 monoclonal antibody, the multi-specific proteins may show superior activation of human NK cells in the presence of cells expressing EGFR, HLA-E, CCR4, or PD-L1.

In certain embodiments, the multi-specific proteins described herein, which include an NKG2D-binding site and a binding site for EGFR, HLA-E, CCR4, or PD-L1, enhance the activity of rested and IL-2-activated human NK cells co-culturing with cells expressing EGFR, HLA-E, CCR4, or PD-L1.

In certain embodiments, compared to a corresponding monoclonal antibody that binds to EGFR, HLA-E, CCR4, or PD-L1, the multi-specific proteins offer an advantage in targeting tumor cells that express medium and low levels of EGFR, HLA-E, CCR4, or PD-L1. The multi-specific binding proteins described herein are more effective in reducing tumor growth and killing cancer cells. For example, a multi-specific binding protein of the present disclosure that targets EGFR-expressing tumor/cancer cells is more effective than panitumumab or necitumumab. A TriNKET of the present disclosure A49-F3'-TriNKET-EGFR (comprising an EGFR-binding scFv *(e.g.,* SEQ ID NO:264) linked to an Fc domain *via* a hinge comprising Ala-Ser (scFv-Fc represented by SEQ ID NO:267); and an NKG2D-binding Fab fragment including a heavy chain portion comprising an heavy chain variable domain of ADI-27749 (A49) (SEQ ID NO:85) and a CH1 domain, and a light chain portion comprising a light chain variable domain (SEQ ID NO:86) and a light chain constant domain, where the heavy chain variable domain is connected to the CH1, and the CH1 domain is connected to the Fc domain (heavy chain portion represented as V_{H}-CH1-Fc, amino acid sequence set forth in SEQ ID NO:270)) is effective in promoting NK-mediated cell lysis of an EGFR-expressing human cancer cell line.

### III. THERAPEUTIC APPLICATIONS

The invention provides methods for treating cancer using a multi-specific binding protein described herein and/or a pharmaceutical composition described herein. The methods may be used to treat a variety of cancers expressing EGFR, HLA-E, CCR4, or PD-L1. In some embodiments, the cancer is leukemia, for example acute myeloid leukemia, T-cell leukemia, acute lymphocytic leukemia, chronic lymphocytic leukemia, chronic myeloid leukemia, or hairy cell leukemia.

In some other embodiments, the cancer is breast, ovarian, esophageal, bladder or gastric cancer, salivary duct carcinoma, salivary duct carcinomas, adenocarcinoma of the lung or aggressive forms of uterine cancer, such as uterine serous endometrial carcinoma. In some other embodiments, the cancer is brain cancer, breast cancer, cervical cancer, colon cancer, colorectal cancer, endometrial cancer, esophageal cancer, leukemia, lung cancer, liver cancer, melanoma, ovarian cancer, pancreatic cancer, rectal cancer, renal cancer, stomach cancer, testicular cancer, or uterine cancer. In yet other embodiments, the cancer is a squamous cell carcinoma, adenocarcinoma, small cell carcinoma, melanoma, neuroblastoma, sarcoma *(e.g.,* an angiosarcoma or chondrosarcoma), larynx cancer, parotid cancer, biliary tract cancer, thyroid cancer, acral lentiginous melanoma, actinic keratoses, acute lymphocytic leukemia, acute myeloid leukemia, adenoid cystic carcinoma, adenomas, adenosarcoma, adenosquamous carcinoma, anal canal cancer, anal cancer, anorectum cancer, astrocytic tumor, bartholin gland carcinoma, basal cell carcinoma, biliary cancer, bone cancer, bone marrow cancer, bronchial cancer, bronchial gland carcinoma, carcinoid, cholangiocarcinoma, chondosarcoma, choroid plexus papilloma/carcinoma, chronic lymphocytic leukemia, chronic myeloid leukemia, clear cell carcinoma, connective tissue cancer, cystadenoma, digestive system cancer, duodenum cancer, endocrine system cancer, endodermal sinus tumor, endometrial hyperplasia, endometrial stromal sarcoma, endometrioid adenocarcinoma, endothelial cell cancer, ependymal cancer, epithelial cell cancer, Ewing's sarcoma, eye and orbit cancer, female genital cancer, focal nodular hyperplasia, gallbladder cancer, gastric antrum cancer, gastric fundus cancer, gastrinoma, glioblastoma, glucagonoma, heart cancer, hemangiblastomas, hemangioendothelioma, hemangiomas, hepatic adenoma, hepatic adenomatosis, hepatobiliary cancer, hepatocellular carcinoma, Hodgkin's disease, ileum cancer, insulinoma, intraepithelial neoplasia, interepithelial squamous cell neoplasia, intrahepatic bile duct cancer, invasive squamous cell carcinoma, jejunum cancer, joint cancer, Kaposi's sarcoma, pelvic cancer, large cell carcinoma, large intestine cancer, leiomyosarcoma, lentigo maligna melanomas, lymphoma, male genital cancer, malignant melanoma, malignant mesothelial tumors, medulloblastoma, medulloepithelioma, meningeal cancer, mesothelial cancer, metastatic carcinoma, mouth cancer, mucoepidermoid carcinoma, multiple myeloma, muscle cancer, nasal tract cancer, nervous system cancer, neuroepithelial adenocarcinoma nodular melanoma, non-epithelial skin cancer, non-Hodgkin's lymphoma, oat cell carcinoma, oligodendroglial cancer, oral cavity cancer, osteosarcoma, papillary serous adenocarcinoma, penile cancer, pharynx cancer, pituitary tumors, plasmacytoma, pseudosarcoma, pulmonary blastoma, rectal cancer, renal cell carcinoma, respiratory system cancer, retinoblastoma, rhabdomyosarcoma, sarcoma, serous carcinoma, sinus cancer, skin cancer, small cell carcinoma, small intestine cancer, smooth muscle cancer, soft tissue cancer, somatostatin-secreting tumor, spine cancer, squamous cell carcinoma, striated muscle cancer, submesothelial cancer, superficial spreading melanoma, T cell leukemia, tongue cancer, undifferentiated carcinoma, ureter cancer, urethra cancer, urinary bladder cancer, urinary system cancer, uterine cervix cancer, uterine corpus cancer, uveal melanoma, vaginal cancer, verrucous carcinoma, VIPoma, vulva cancer, well-differentiated carcinoma, or Wilms tumor.

In some other embodiments, the cancer to be treated is non-Hodgkin's lymphoma, such as a B-cell lymphoma or a T-cell lymphoma. In certain embodiments, the non-Hodgkin's lymphoma is a B-cell lymphoma, such as a diffuse large B-cell lymphoma, primary mediastinal B-cell lymphoma, follicular lymphoma, small lymphocytic lymphoma, mantle cell lymphoma, marginal zone B-cell lymphoma, extranodal marginal zone B-cell lymphoma, nodal marginal zone B-cell lymphoma, splenic marginal zone B-cell lymphoma, Burkitt lymphoma, lymphoplasmacytic lymphoma, hairy cell leukemia, or primary central nervous system (CNS) lymphoma. In certain other embodiments, the non-Hodgkin's lymphoma is a T-cell lymphoma, such as a precursor T-lymphoblastic lymphoma, peripheral T-cell lymphoma, cutaneous T-cell lymphoma, angioimmunoblastic T-cell lymphoma, extranodal natural killer/T-cell lymphoma, enteropathy type T-cell lymphoma, subcutaneous panniculitis-like T-cell lymphoma, anaplastic large cell lymphoma, or peripheral T-cell lymphoma.

In some other embodiments, the cancer to be treated is selected from the group consisting of head and neck cancer, colorectal cancer, non-small cell lung cancer, glioma, renal cell carcinoma, bladder cancer, cervical cancer, ovarian cancer, pancreatic cancer, and liver cancer.

In some other embodiments, the cancer to be treated is selected from the group consisting of lymphoma, head and neck cancer, bladder cancer, cervical cancer, lung cancer, renal cancer, melanoma, colorectal cancer, ovarian cancer, glioblastoma, and a sarcoma.

In some other embodiments, the cancer to be treated is selected from the group consisting of lymphoma, leukemia, multiple myeloma, head and neck cancer, bladder cancer, cervical cancer, lung cancer, renal cancer, melanoma, colorectal cancer, ovarian cancer, glioblastoma, a sarcoma, and gastric cancer.

In some other embodiments, the cancer to be treated is selected from the group consisting of adult T-cell lymphoma/leukemia, peripheral T cell lymphoma, cutaneous T cell lymphoma, chronic lymphocytic leukemia, a B cell malignancy, non-Hodgkin's lymphoma, Hodgkin's lymphoma, anaplastic large cell lymphoma, mature T/natural killer (NK) cell neoplasms, thymoma, gastric cancer, and renal cell carcinoma.

### IV. COMBINATION THERAPY

Another aspect of the invention provides for combination therapy. A multi-specific binding protein described herein can be used in combination with additional therapeutic agents to treat the cancer.

Exemplary therapeutic agents that may be used as part of a combination therapy in treating cancer, include, for example, radiation, mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma (IFN-γ), colony stimulating factor-1, colony stimulating factor-2, denileukin diftitox, interleukin-2, luteinizing hormone releasing factor and variations of the aforementioned agents that may exhibit differential binding to its cognate receptor, and increased or decreased serum half-life.

An additional class of agents that may be used as part of a combination therapy in treating cancer is immune checkpoint inhibitors. Exemplary immune checkpoint inhibitors include agents that inhibit one or more of (i) cytotoxic T lymphocyte-associated antigen 4 (CTLA4), (ii) programmed cell death protein 1 (PD1), (iii) PDL1, (iv) LAG3, (v) B7-H3, (vi) B7-H4, and (vii) TIM3. The CTLA4 inhibitor ipilimumab has been approved by the United States Food and Drug Administration for treating melanoma.

Yet other agents that may be used as part of a combination therapy in treating cancer are monoclonal antibody agents that target non-checkpoint targets *(e.g.,* herceptin) and non-cytotoxic agents *(e.g.,* tyrosine-kinase inhibitors).

Yet other categories of anti-cancer agents include, for example: (i) an inhibitor selected from an ALK Inhibitor, an ATR Inhibitor, an A2A Antagonist, a Base Excision Repair Inhibitor, a Bcr-Abl Tyrosine Kinase Inhibitor, a Bruton's Tyrosine Kinase Inhibitor, a CDC7 Inhibitor, a CHK1 Inhibitor, a Cyclin-Dependent Kinase Inhibitor, a DNA-PK Inhibitor, an Inhibitor of both DNA-PK and mTOR, a DNMT1 Inhibitor, a DNMT1 Inhibitor plus 2-chloro-deoxyadenosine, an HDAC Inhibitor, a Hedgehog Signaling Pathway Inhibitor, an IDO Inhibitor, a JAK Inhibitor, a mTOR Inhibitor, a MEK Inhibitor, a MELK Inhibitor, a MTH1 Inhibitor, a PARP Inhibitor, a Phosphoinositide 3-Kinase Inhibitor, an Inhibitor of both PARP1 and DHODH, a Proteasome Inhibitor, a Topoisomerase-II Inhibitor, a Tyrosine Kinase Inhibitor, a VEGFR Inhibitor, and a WEE1 Inhibitor; (ii) an agonist of OX40, CD137, CD40, GITR, CD27, HVEM, TNFRSF25, or ICOS; and (iii) a cytokine selected from IL-12, IL-15, GM-CSF, and G-CSF.

Proteins of the invention can also be used as an adjunct to surgical removal of the primary lesion.

The amount of multi-specific binding protein and additional therapeutic agent and the relative timing of administration may be selected in order to achieve a desired combined therapeutic effect. For example, when administering a combination therapy to a patient in need of such administration, the therapeutic agents in the combination, or a pharmaceutical composition or compositions comprising the therapeutic agents, may be administered in any order such as, for example, sequentially, concurrently, together, simultaneously and the like. Further, for example, a multi-specific binding protein may be administered during a time when the additional therapeutic agent(s) exerts its prophylactic or therapeutic effect, or *vice versa.*

### V. PHARMACEUTICAL COMPOSITIONS

The present disclosure also features pharmaceutical compositions that contain a therapeutically effective amount of a protein described herein. The composition can be formulated for use in a variety of drug delivery systems. One or more physiologically acceptable excipients or carriers can also be included in the composition for proper formulation. Suitable formulations for use in the present disclosure are found in Remington's Pharmaceutical Sciences, Mack Publishing Company, Philadelphia, Pa., 17th ed., 1985. For a brief review of methods for drug delivery, *see, e.g.,* Langer (Science 249:1527-1533, 1990).

The intravenous drug delivery formulation of the present disclosure may be contained in a bag, a pen, or a syringe. In certain embodiments, the bag may be connected to a channel comprising a tube and/or a needle. In certain embodiments, the formulation may be a lyophilized formulation or a liquid formulation. In certain embodiments, the formulation may freeze-dried (lyophilized) and contained in about 12-60 vials. In certain embodiments, the formulation may be freeze-dried and 45 mg of the freeze-dried formulation may be contained in one vial. In certain embodiments, the about 40 mg - about 100 mg of freeze-dried formulation may be contained in one vial. In certain embodiments, freeze dried formulation from 12, 27, or 45 vials are combined to obtained a therapeutic dose of the protein in the intravenous drug formulation. In certain embodiments, the formulation may be a liquid formulation and stored as about 250 mg/vial to about 1000 mg/vial. In certain embodiments, the formulation may be a liquid formulation and stored as about 600 mg/vial. In certain embodiments, the formulation may be a liquid formulation and stored as about 250 mg/vial.

The protein could exist in a liquid aqueous pharmaceutical formulation including a therapeutically effective amount of the protein in a buffered solution forming a formulation.

These compositions may be sterilized by conventional sterilization techniques, or may be sterile filtered. The resulting aqueous solutions may be packaged for use as-is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of the above-mentioned agent or agents. The composition in solid form can also be packaged in a container for a flexible quantity.

In certain embodiments, the present disclosure provides a formulation with an extended shelf life including the protein of the present disclosure, in combination with mannitol, citric acid monohydrate, sodium citrate, disodium phosphate dihydrate, sodium dihydrogen phosphate dihydrate, sodium chloride, polysorbate 80, water, and sodium hydroxide.

In certain embodiments, an aqueous formulation is prepared including the protein of the present disclosure in a pH-buffered solution. The buffer of this invention may have a pH ranging from about 4 to about 8, *e.g.,* from about 4.5 to about 6.0, or from about 4.8 to about 5.5, or may have a pH of about 5.0 to about 5.2. Ranges intermediate to the above recited pH's are also intended to be part of this disclosure. For example, ranges of values using a combination of any of the above recited values as upper and/or lower limits are intended to be included. Examples of buffers that will control the pH within this range include acetate (e.g., sodium acetate), succinate (such as sodium succinate), gluconate, histidine, citrate and other organic acid buffers.

In certain embodiments, the formulation includes a buffer system which contains citrate and phosphate to maintain the pH in a range of about 4 to about 8. In certain embodiments the pH range may be from about 4.5 to about 6.0, or from about pH 4.8 to about 5.5, or in a pH range of about 5.0 to about 5.2. In certain embodiments, the buffer system includes citric acid monohydrate, sodium citrate, disodium phosphate dihydrate, and/or sodium dihydrogen phosphate dihydrate. In certain embodiments, the buffer system includes about 1.3 mg/mL of citric acid *(e.g.,* 1.305 mg/mL), about 0.3 mg/mL of sodium citrate (*e.g.,* 0.305 mg/mL), about 1.5 mg/mL of disodium phosphate dihydrate (*e.g.,* 1.53 mg/mL), about 0.9 mg/mL of sodium dihydrogen phosphate dihydrate (*e.g*., 0.86), and about 6.2 mg/mL of sodium chloride *(e.g.,* 6.165 mg/mL). In certain embodiments, the buffer system includes 1-1.5 mg/mL of citric acid, 0.25 to 0.5 mg/mL of sodium citrate, 1.25 to 1.75 mg/mL of disodium phosphate dihydrate, 0.7 to 1.1 mg/mL of sodium dihydrogen phosphate dihydrate, and 6.0 to 6.4 mg/mL of sodium chloride. In certain embodiments, the pH of the formulation is adjusted with sodium hydroxide.

A polyol, which acts as a tonicifier and may stabilize the antibody, may also be included in the formulation. The polyol is added to the formulation in an amount which may vary with respect to the desired isotonicity of the formulation. In certain embodiments, the aqueous formulation may be isotonic. The amount of polyol added may also be altered with respect to the molecular weight of the polyol. For example, a lower amount of a monosaccharide (e.g., mannitol) may be added, compared to a disaccharide (such as trehalose). In certain embodiments, the polyol which may be used in the formulation as a tonicity agent is mannitol. In certain embodiments, the mannitol concentration may be about 5 to about 20 mg/mL. In certain embodiments, the concentration of mannitol may be about 7.5 to 15 mg/mL. In certain embodiments, the concentration of mannitol may be about 10-14 mg/mL. In certain embodiments, the concentration of mannitol may be about 12 mg/mL. In certain embodiments, the polyol sorbitol may be included in the formulation.

A detergent or surfactant may also be added to the formulation. Exemplary detergents include nonionic detergents such as polysorbates (e.g., polysorbates 20, 80 etc.) or poloxamers (e.g., poloxamer 188). The amount of detergent added is such that it reduces aggregation of the formulated antibody and/or minimizes the formation of particulates in the formulation and/or reduces adsorption. In certain embodiments, the formulation may include a surfactant which is a polysorbate. In certain embodiments, the formulation may contain the detergent polysorbate 80 or Tween 80. Tween 80 is a term used to describe polyoxyethylene (20) sorbitanmonooleate (*see* Fiedler, Lexikon der Hifsstoffe, Editio Cantor Verlag Aulendorf, 4th ed., 1996). In certain embodiments, the formulation may contain between about 0.1 mg/mL and about 10 mg/mL of polysorbate 80, or between about 0.5 mg/mL and about 5 mg/mL. In certain embodiments, about 0.1% polysorbate 80 may be added in the formulation.

In embodiments, the protein product of the present disclosure is formulated as a liquid formulation. The liquid formulation may be presented at a 10 mg/mL concentration in either a USP / Ph Eur type I 50R vial closed with a rubber stopper and sealed with an aluminum crimp seal closure. The stopper may be made of elastomer complying with USP and Ph Eur. In certain embodiments vials may be filled with 61.2 mL of the protein product solution in order to allow an extractable volume of 60 mL. In certain embodiments, the liquid formulation may be diluted with 0.9% saline solution.

In certain embodiments, the liquid formulation of the disclosure may be prepared as a 10 mg/mL concentration solution in combination with a sugar at stabilizing levels. In certain embodiments the liquid formulation may be prepared in an aqueous carrier. In certain embodiments, a stabilizer may be added in an amount no greater than that which may result in a viscosity undesirable or unsuitable for intravenous administration. In certain embodiments, the sugar may be disaccharides, *e.g.*, sucrose. In certain embodiments, the liquid formulation may also include one or more of a buffering agent, a surfactant, and a preservative.

In certain embodiments, the pH of the liquid formulation may be set by addition of a pharmaceutically acceptable acid and/or base. In certain embodiments, the pharmaceutically acceptable acid may be hydrochloric acid. In certain embodiments, the base may be sodium hydroxide.

In addition to aggregation, deamidation is a common product variant of peptides and proteins that may occur during fermentation, harvest/cell clarification, purification, drug substance/drug product storage and during sample analysis. Deamidation is the loss of NH₃ from a protein forming a succinimide intermediate that can undergo hydrolysis. The succinimide intermediate results in a 17 dalton mass decrease of the parent peptide. The subsequent hydrolysis results in an 18 dalton mass increase. Isolation of the succinimide intermediate is difficult due to instability under aqueous conditions. As such, deamidation is typically detectable as 1 dalton mass increase. Deamidation of an asparagine results in either aspartic or isoaspartic acid. The parameters affecting the rate of deamidation include pH, temperature, solvent dielectric constant, ionic strength, primary sequence, local polypeptide conformation and tertiary structure. The amino acid residues adjacent to Asn in the peptide chain affect deamidation rates. Gly and Ser following an Asn in protein sequences results in a higher susceptibility to deamidation.

In certain embodiments, the liquid formulation of the present disclosure may be preserved under conditions of pH and humidity to prevent deamination of the protein product.

The aqueous carrier of interest herein is one which is pharmaceutically acceptable (safe and non-toxic for administration to a human) and is useful for the preparation of a liquid formulation. Illustrative carriers include sterile water for injection (SWFI), bacteriostatic water for injection (BWFI), a pH buffered solution (e.g., phosphate-buffered saline), sterile saline solution, Ringer's solution or dextrose solution.

A preservative may be optionally added to the formulations herein to reduce bacterial action. The addition of a preservative may, for example, facilitate the production of a multi-use (multiple-dose) formulation.

Intravenous (IV) formulations may be the preferred administration route in particular instances, such as when a patient is in the hospital after transplantation receiving all drugs via the IV route. In certain embodiments, the liquid formulation is diluted with 0.9% Sodium Chloride solution before administration. In certain embodiments, the diluted drug product for injection is isotonic and suitable for administration by intravenous infusion.

In certain embodiments, a salt or buffer components may be added in an amount of 10 mM - 200 mM. The salts and/or buffers are pharmaceutically acceptable and are derived from various known acids (inorganic and organic) with "base forming" metals or amines. In certain embodiments, the buffer may be phosphate buffer. In certain embodiments, the buffer may be glycinate, carbonate, citrate buffers, in which case, sodium, potassium or ammonium ions can serve as counterion.

A preservative may be optionally added to the formulations herein to reduce bacterial action. The addition of a preservative may, for example, facilitate the production of a multi-use (multiple-dose) formulation.

The aqueous carrier of interest herein is one which is pharmaceutically acceptable (safe and non-toxic for administration to a human) and is useful for the preparation of a liquid formulation. Illustrative carriers include sterile water for injection (SWFI), bacteriostatic water for injection (BWFI), a pH buffered solution *(e.g.,* phosphate-buffered saline), sterile saline solution, Ringer's solution or dextrose solution.

The protein of the present disclosure could exist in a lyophilized formulation including the proteins and a lyoprotectant. The lyoprotectant may be sugar, *e.g.,* disaccharides. In certain embodiments, the lyoprotectant may be sucrose or maltose. The lyophilized formulation may also include one or more of a buffering agent, a surfactant, a bulking agent, and/or a preservative.

The amount of sucrose or maltose useful for stabilization of the lyophilized drug product may be in a weight ratio of at least 1:2 protein to sucrose or maltose. In certain embodiments, the protein to sucrose or maltose weight ratio may be of from 1:2 to 1:5.

In certain embodiments, the pH of the formulation, prior to lyophilization, may be set by addition of a pharmaceutically acceptable acid and/or base. In certain embodiments the pharmaceutically acceptable acid may be hydrochloric acid. In certain embodiments, the pharmaceutically acceptable base may be sodium hydroxide.

Before lyophilization, the pH of the solution containing the protein of the present disclosure may be adjusted between 6 to 8. In certain embodiments, the pH range for the lyophilized drug product may be from 7 to 8.

In certain embodiments, a salt or buffer components may be added in an amount of 10 mM - 200 mM. The salts and/or buffers are pharmaceutically acceptable and are derived from various known acids (inorganic and organic) with "base forming" metals or amines. In certain embodiments, the buffer may be phosphate buffer. In certain embodiments, the buffer may be glycinate, carbonate, citrate buffers, in which case, sodium, potassium or ammonium ions can serve as counterion.

In certain embodiments, a "bulking agent" may be added. A "bulking agent" is a compound which adds mass to a lyophilized mixture and contributes to the physical structure of the lyophilized cake *(e.g.,* facilitates the production of an essentially uniform lyophilized cake which maintains an open pore structure). Illustrative bulking agents include mannitol, glycine, polyethylene glycol and sorbitol. The lyophilized formulations of the present invention may contain such bulking agents.

A preservative may be optionally added to the formulations herein to reduce bacterial action. The addition of a preservative may, for example, facilitate the production of a multi-use (multiple-dose) formulation.

In certain embodiments, the lyophilized drug product may be constituted with an aqueous carrier. The aqueous carrier of interest herein is one which is pharmaceutically acceptable *(e.g.,* safe and non-toxic for administration to a human) and is useful for the preparation of a liquid formulation, after lyophilization. Illustrative diluents include sterile water for injection (SWFI), bacteriostatic water for injection (BWFI), a pH buffered solution *(e.g.,* phosphate-buffered saline), sterile saline solution, Ringer's solution or dextrose solution.

In certain embodiments, the lyophilized drug product of the current disclosure is reconstituted with either Sterile Water for Injection, USP (SWFI) or 0.9% Sodium Chloride Injection, USP. During reconstitution, the lyophilized powder dissolves into a solution.

In certain embodiments, the lyophilized protein product of the instant disclosure is constituted to about 4.5 mL water for injection and diluted with 0.9% saline solution (sodium chloride solution).

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The specific dose can be a uniform dose for each patient, for example, 50-5000 mg of protein. Alternatively, a patient's dose can be tailored to the approximate body weight or surface area of the patient. Other factors in determining the appropriate dosage can include the disease or condition to be treated or prevented, the severity of the disease, the route of administration, and the age, sex and medical condition of the patient. Further refinement of the calculations necessary to determine the appropriate dosage for treatment is routinely made by those skilled in the art, especially in light of the dosage information and assays disclosed herein. The dosage can also be determined through the use of known assays for determining dosages used in conjunction with appropriate dose-response data. An individual patient's dosage can be adjusted as the progress of the disease is monitored. Blood levels of the targetable construct or complex in a patient can be measured to see if the dosage needs to be adjusted to reach or maintain an effective concentration. Pharmacogenomics may be used to determine which targetable constructs and/or complexes, and dosages thereof, are most likely to be effective for a given individual (Schmitz et al., Clinica Chimica Acta 308: 43-53, 2001; Steimer et al., Clinica Chimica Acta 308: 33-41, 2001).

In general, dosages based on body weight are from about 0.01 µg to about 100 mg per kg of body weight, such as about 0.01 µg to about 100 mg/kg of body weight, about 0.01 µg to about 50 mg/kg of body weight, about 0.01 µg to about 10 mg/kg of body weight, about 0.01 µg to about 1 mg/kg of body weight, about 0.01 µg to about 100 µg/kg of body weight, about 0.01 µg to about 50 µg/kg of body weight, about 0.01 µg to about 10 µg/kg of body weight, about 0.01 µg to about 1 µg/kg of body weight, about 0.01 µg to about 0.1 µg/kg of body weight, about 0.1 µg to about 100 mg/kg of body weight, about 0.1 µg to about 50 mg/kg of body weight, about 0.1 µg to about 10 mg/kg of body weight, about 0.1 µg to about 1 mg/kg of body weight, about 0.1 µg to about 100 µg/kg of body weight, about 0.1 µg to about 10 µg/kg of body weight, about 0.1 µg to about 1 µg/kg of body weight, about 1 µg to about 100 mg/kg of body weight, about 1 µg to about 50 mg/kg of body weight, about 1 µg to about 10 mg/kg of body weight, about 1 µg to about 1 mg/kg of body weight, about 1 µg to about 100 µg/kg of body weight, about 1 µg to about 50 µg/kg of body weight, about 1 µg to about 10 µg/kg of body weight, about 10 µg to about 100 mg/kg of body weight, about 10 µg to about 50 mg/kg of body weight, about 10 µg to about 10 mg/kg of body weight, about 10 µg to about 1 mg/kg of body weight, about 10 µg to about 100 µg/kg of body weight, about 10 µg to about 50 µg/kg of body weight, about 50 µg to about 100 mg/kg of body weight, about 50µg to about 50 mg/kg of body weight, about 50 µg to about 10 mg/kg of body weight, about 50 µg to about 1 mg/kg of body weight, about 50 µg to about 100 µg/kg of body weight, about 100 µg to about 100 mg/kg of body weight, about 100 µg to about 50 mg/kg of body weight, about 100 µg to about 10 mg/kg of body weight, about 100 µg to about 1 mg/kg of body weight, about 1 mg to about 100 mg/kg of body weight, about 1 mg to about 50 mg/kg of body weight, about 1 mg to about 10 mg/kg of body weight, about 10 mg to about 100 mg/kg of body weight, about 10 mg to about 50 mg/kg of body weight, about 50 mg to about 100 mg/kg of body weight.

Doses may be given once or more times daily, weekly, monthly or yearly, or even once every 2 to 20 years. Persons of ordinary skill in the art can easily estimate repetition rates for dosing based on measured residence times and concentrations of the targetable construct or complex in bodily fluids or tissues. Administration of the present invention could be intravenous, intraarterial, intraperitoneal, intramuscular, subcutaneous, intrapleural, intrathecal, intracavitary, by perfusion through a catheter or by direct intralesional injection. This may be administered once or more times daily, once or more times weekly, once or more times monthly, and once or more times annually.

The description above describes multiple aspects and embodiments of the invention. The patent application specifically contemplates all combinations and permutations of the aspects and embodiments.

### EXAMPLES

The invention now being generally described, will be more readily understood by reference to the following examples, which are included merely for purposes of illustration of certain aspects and embodiments of the present invention, and which are not intended to limit the invention.

### Example 1 - NKG2D binding domains bind to NKG2D

### NKG2D-binding domains bind to purified recombinant NKG2D

The nucleic acid sequences of human, mouse, or cynomolgus NKG2D ectodomains were fused with nucleic acid sequences encoding human IgG1 Fc domains and introduced into mammalian cells to be expressed. After purification, NKG2D-Fc fusion proteins were adsorbed to wells of microplates. After blocking the wells with bovine serum albumin to prevent non-specific binding, NKG2D-binding domains were titrated and added to the wells pre-adsorbed with NKG2D-Fc fusion proteins. Primary antibody binding was detected using a secondary antibody which was conjugated to horseradish peroxidase and specifically recognizes a human kappa light chain to avoid Fc cross-reactivity. 3,3',5,5'-Tetramethylbenzidine (TMB), a substrate for horseradish peroxidase, was added to the wells to visualize the binding signal, whose absorbance was measured at 450 nM and corrected at 540 nM. An NKG2D-binding domain clone, an isotype control or a positive control (comprising heavy chain and light chain variable domains selected from SEQ ID NOs:101-104, or anti-mouse NKG2D clones MI-6 and CX-5 available at eBioscience) was added to each well.

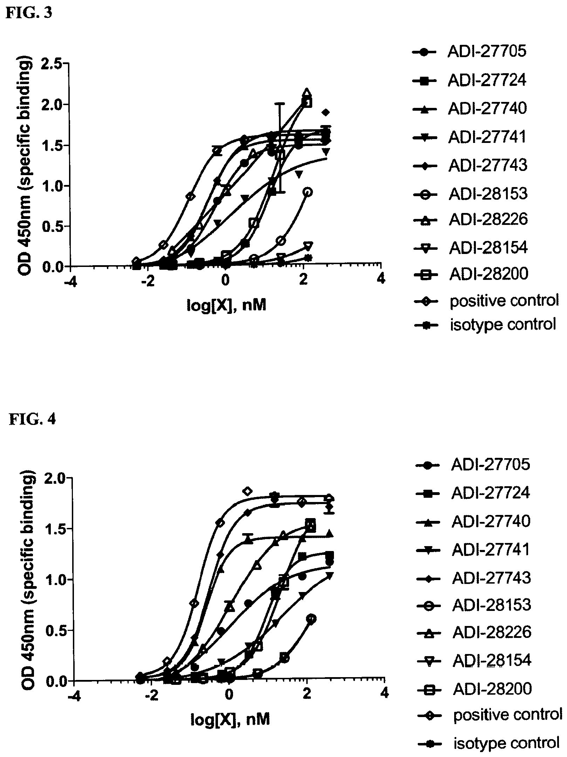

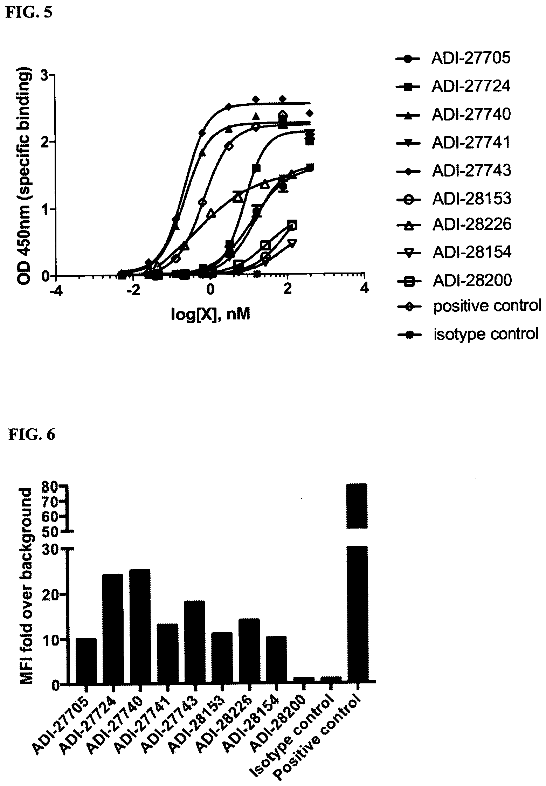

The isotype control showed minimal binding to recombinant NKG2D-Fc proteins, while the positive control bound strongest to the recombinant antigens. NKG2D-binding domains produced by all clones demonstrated binding across human, mouse, and cynomolgus recombinant NKG2D-Fc proteins, although with varying affinities from clone to clone. Generally, each anti-NKG2D clone bound to human (FIG. 3) and cynomolgus (FIG. 4) recombinant NKG2D-Fc with similar affinity, but with lower affinity to mouse (FIG. 5) recombinant NKG2D-Fc.

### NKG2D-binding domains bind to cells expressing NKG2D

EL4 mouse lymphoma cell lines were engineered to express human or mouse NKG2D-CD3 zeta signaling domain chimeric antigen receptors. An NKG2D-binding clone, an isotype control, or a positive control was used at a 100 nM concentration to stain extracellular NKG2D expressed on the EL4 cells. The antibody binding was detected using fluorophore-conjugated anti-human IgG secondary antibodies. Cells were analyzed by flow cytometry, and fold-over-background (FOB) was calculated using the mean fluorescence intensity (MFI) of NKG2D-expressing cells compared to parental EL4 cells.

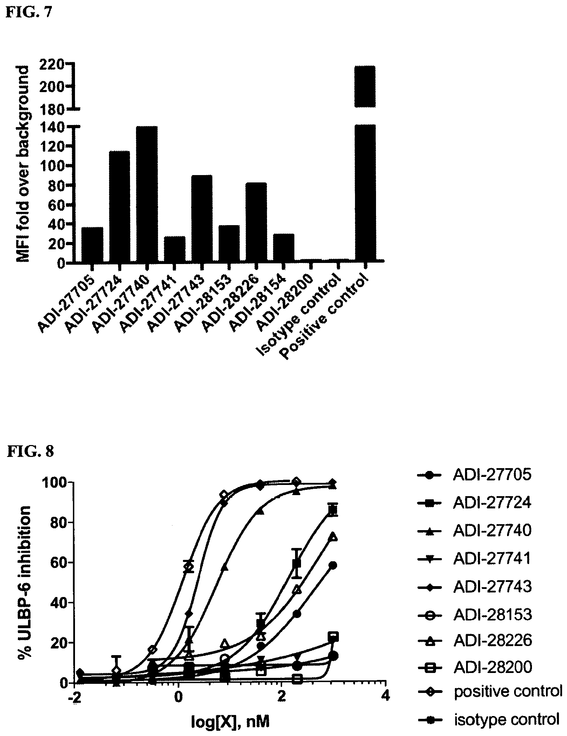

NKG2D-binding domains produced by all clones bound to EL4 cells expressing human and mouse NKG2D. Positive control antibodies (comprising heavy chain and light chain variable domains selected from SEQ ID NOs: 101-104, or anti-mouse NKG2D clones MI-6 and CX-5 available at eBioscience) gave the best FOB binding signal. The NKG2D-binding affinity for each clone was similar between cells expressing human NKG2D (FIG. 6) and mouse (FIG. 7) NKG2D.

### Example 2 - NKG2D-binding domains block natural ligand binding to NKG2D

### Competition With ULBP-6

Recombinant human NKG2D-Fc proteins were adsorbed to wells of a microplate, and the wells were blocked with bovine serum albumin to reduce non-specific binding. A saturating concentration of ULBP-6-His-biotin was added to the wells, followed by addition of the NKG2D-binding domain clones. After a 2-hour incubation, wells were washed and ULBP-6-His-biotin that remained bound to the NKG2D-Fc coated wells was detected by streptavidin-conjugated to horseradish peroxidase and TMB substrate. Absorbance was measured at 450 nM and corrected at 540 nM. After subtracting background, specific binding of NKG2D-binding domains to the NKG2D-Fc proteins was calculated from the percentage of ULBP-6-His-biotin that was blocked from binding to the NKG2D-Fc proteins in wells. The positive control antibody (comprising heavy chain and light chain variable domains selected from SEQ ID NOs: 101-104) and various NKG2D-binding domains blocked ULBP-6 binding to NKG2D, while isotype control showed little competition with ULBP-6 (FIG. 8). ULBP-6 sequence is represented by SEQ ID NO:108

MAAAAIPALLLCLPLLFLLFGWSRARRDDPHSLCYDITVIPKFRPGPRWCAVQ
GQVDEKTFLHYDCGNKTVTPVSPLGKKLNVTMAWKAQNPVLREVVDILTEQ
LLDIQLENYTPKEPLTLQARMSCEQKAEGHSSGSWQFSIDGQTFLLFDSEKRM
WTTVHPGARKMKEKWENDKDVAMSFHYISMGDCIGWLEDFLMGMDSTLEP
SAGAPLAMSSGTTQLRATATTLILCCLLIILPCFILPGI (SEQ ID NO:108)

### Competition With MICA

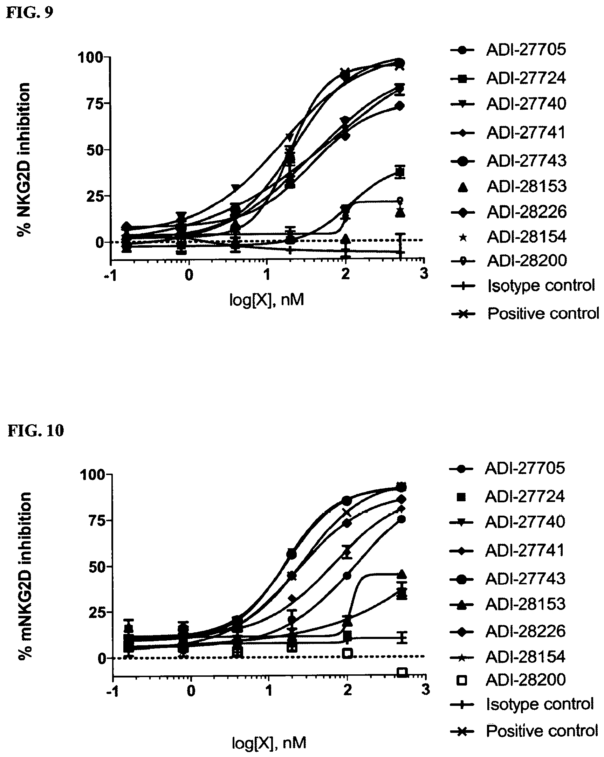

Recombinant human MICA-Fc proteins were adsorbed to wells of a microplate, and the wells were blocked with bovine serum albumin to reduce non-specific binding. NKG2D-Fc-biotin was added to wells followed by NKG2D-binding domains. After incubation and washing, NKG2D-Fc-biotin that remained bound to MICA-Fc coated wells was detected using streptavidin-HRP and TMB substrate. Absorbance was measured at 450 nM and corrected at 540 nM. After subtracting background, specific binding of NKG2D-binding domains to the NKG2D-Fc proteins was calculated from the percentage of NKG2D-Fc-biotin that was blocked from binding to the MICA-Fc coated wells. The positive control antibody (comprising heavy chain and light chain variable domains selected from SEQ ID NOs: 101-104) and various NKG2D-binding domains blocked MICA binding to NKG2D, while isotype control showed little competition with MICA (FIG. 9).

### Competition With Rae-1 delta

Recombinant mouse Rae-1 delta-Fc (purchased from R&D Systems) was adsorbed to wells of a microplate, and the wells were blocked with bovine serum albumin to reduce non-specific binding. Mouse NKG2D-Fc-biotin was added to the wells followed by NKG2D-binding domains. After incubation and washing, NKG2D-Fc-biotin that remained bound to Rae-1delta-Fc coated wells was detected using streptavidin-HRP and TMB substrate. Absorbance was measured at 450 nM and corrected at 540 nM. After subtracting background, specific binding of NKG2D-binding domains to the NKG2D-Fc proteins was calculated from the percentage of NKG2D-Fc-biotin that was blocked from binding to the Rae-1delta-Fc coated wells. The positive control (comprising heavy chain and light chain variable domains selected from SEQ ID NOs:101-104, or anti-mouse NKG2D clones MI-6 and CX-5 available at eBioscience) and various NKG2D-binding domain clones blocked Rae-1delta binding to mouse NKG2D, while the isotype control antibody showed little competition with Rae-1delta (FIG. 10).

### Example 3 - NKG2D-binding domain clones activate NKG2D

Nucleic acid sequences of human and mouse NKG2D were fused to nucleic acid sequences encoding a CD3 zeta signaling domain to obtain chimeric antigen receptor (CAR) constructs. The NKG2D-CAR constructs were then cloned into a retrovirus vector using Gibson assembly and transfected into expi293 cells for retrovirus production. EL4 cells were infected with viruses containing NKG2D-CAR together with 8 µg/mL polybrene. 24 hours after infection, the expression levels of NKG2D-CAR in the EL4 cells were analyzed by flow cytometry, and clones which express high levels of the NKG2D-CAR on the cell surface were selected.

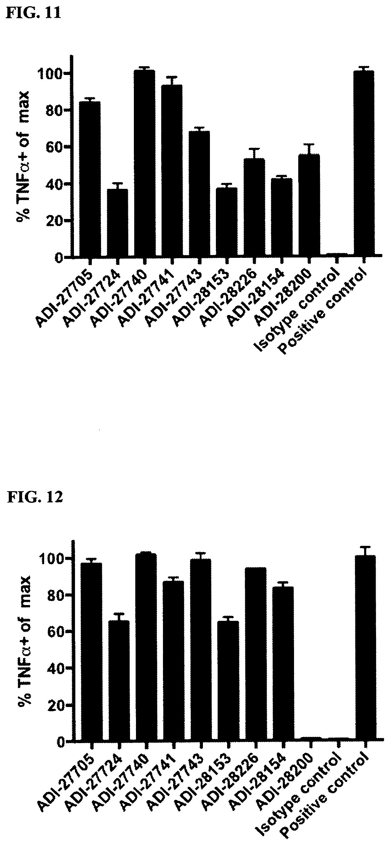

To determine whether NKG2D-binding domains activate NKG2D, they were adsorbed to wells of a microplate, and NKG2D-CAR EL4 cells were cultured on the antibody fragment-coated wells for 4 hours in the presence of brefeldin-A and monensin. Intracellular TNF-α production, an indicator for NKG2D activation, was assayed by flow cytometry. The percentage of TNF-α positive cells was normalized to the cells treated with the positive control. All NKG2D-binding domains activated both human NKG2D (FIG. 11) and mouse NKG2D (FIG. 12).

### Example 4 - NKG2D-binding domains activate NK cells

### Primary human NK cells

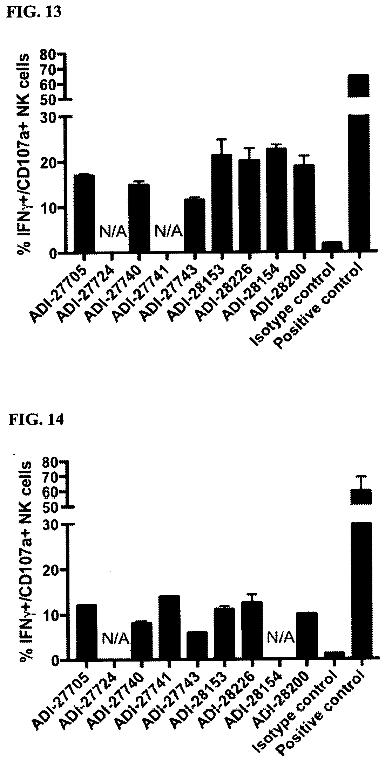

Peripheral blood mononuclear cells (PBMCs) were isolated from human peripheral blood buffy coats using density gradient centrifugation. NK cells (CD3⁻ CD56⁺) were isolated using negative selection with magnetic beads from PBMCs, and the purity of the isolated NK cells was typically >95%. Isolated NK cells were then cultured in media containing 100 ng/mL IL-2 for 24-48 hours before they were transferred to the wells of a microplate to which the NKG2D-binding domains were adsorbed, and cultured in the media containing fluorophore-conjugated anti-CD107a antibody, brefeldin-A, and monensin. Following culture, NK cells were assayed by flow cytometry using fluorophore-conjugated antibodies against CD3, CD56 and IFN-γ. CD107a and IFN-γ staining were analyzed in CD3⁻ CD56⁺ cells to assess NK cell activation. The increase in CD107a/IFN-γ double-positive cells is indicative of better NK cell activation through engagement of two activating receptors rather than one receptor. NKG2D-binding domains and the positive control *(e.g.,* heavy chain variable domain represent by SEQ ID NO:101 or SEQ ID NO:103, and light chain variable domain represented by SEQ ID NO: 102 or SEQ ID NO: 104) showed a higher percentage of NK cells becoming CD107a⁺ and IFN-γ⁺ than the isotype control (FIG. 13 & FIG. 14 represent data from two independent experiments, each using a different donor's PBMC for NK cell preparation).

### Primary mouse NK cells

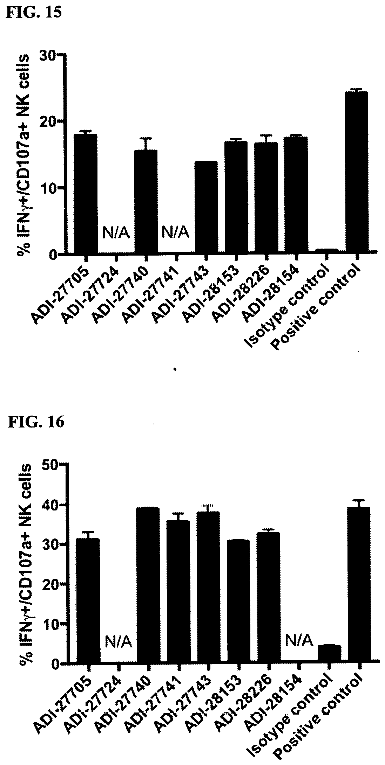

Spleens were obtained from C57B1/6 mice and crushed through a 70 µm cell strainer to obtain single cell suspension. Cells were pelleted and resuspended in ACK lysis buffer (purchased from Thermo Fisher Scientific #A1049201; 155 mM ammonium chloride, 10 mM potassium bicarbonate, 0.01 mM EDTA) to remove red blood cells. The remaining cells were cultured with 100 ng/mL hIL-2 for 72 hours before being harvested and prepared for NK cell isolation. NK cells (CD3⁻NK1.1⁺) were then isolated from spleen cells using a negative depletion technique with magnetic beads with typically >90% purity. Purified NK cells were cultured in media containing 100 ng/mL mIL-15 for 48 hours before they were transferred to the wells of a microplate to which the NKG2D-binding domains were adsorbed, and cultured in the media containing fluorophore-conjugated anti-CD107a antibody, brefeldin-A, and monensin. Following culture in NKG2D-binding domain-coated wells, NK cells were assayed by flow cytometry using fluorophore-conjugated antibodies against CD3, NK1.1 and IFN-γ. CD107a and IFN-γ staining were analyzed in CD3⁻ NK1.1⁺ cells to assess NK cell activation. The increase in CD107a/IFN-γ double-positive cells is indicative of better NK cell activation through engagement of two activating receptors rather than one receptor. NKG2D-binding domains and the positive control (selected from anti-mouse NKG2D clones MI-6 and CX-5 available at eBioscience) showed a higher percentage of NK cells becoming CD107a⁺ and IFN-γ⁺ than the isotype control (FIG. 15 & FIG. 16 represent data from two independent experiments, each using a different mouse for NK cell preparation).

### Example 5 - NKG2D-binding domains enable cytotoxicity of target tumor cells

Human and mouse primary NK cell activation assays demonstrated increased cytotoxicity markers on NK cells after incubation with NKG2D-binding domains. To address whether this translates into increased tumor cell lysis, a cell-based assay was utilized where each NKG2D-binding domain was developed into a monospecific antibody. The Fc region was used as one targeting arm, while the Fab fragment regions (NKG2D-binding domain) acted as another targeting arm to activate NK cells. THP-1 cells, which are of human origin and express high levels of Fc receptors, were used as a tumor target and a Perkin Elmer DELFIA Cytotoxicity Kit was used. THP-1 cells were labeled with BATDA reagent, and resuspended at 10⁵/mL in culture media. Labeled THP-1 cells were then combined with NKG2D antibodies and isolated mouse NK cells in wells of a microtiter plate at 37 °C for 3 hours. After incubation, 20 µL of the culture supernatant was removed, mixed with 200 µL of Europium solution and incubated with shaking for 15 minutes in the dark. Fluorescence was measured over time by a PheraStar plate reader equipped with a time-resolved fluorescence module (Excitation 337 nM, Emission 620 nM) and specific lysis was calculated according to the kit instructions.

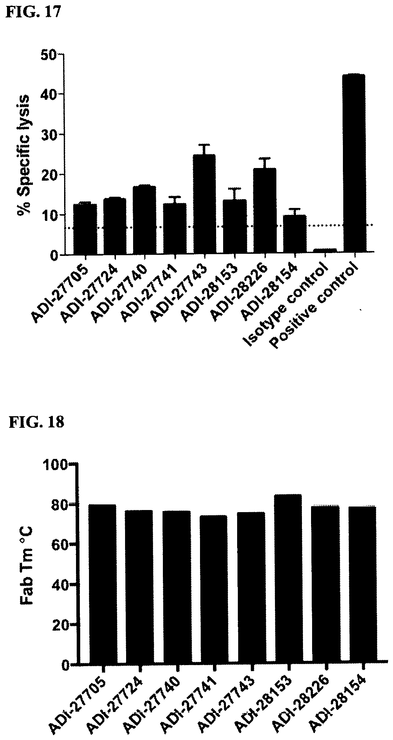

The positive control, ULBP-6 - a natural ligand for NKG2D - conjugated to Fc, showed increased specific lysis of THP-1 target cells by mouse NK cells. NKG2D antibodies also increased specific lysis of THP-1 target cells, while isotype control antibody showed reduced specific lysis. The dotted line indicates specific lysis of THP-1 cells by mouse NK cells without antibody added (FIG. 17).

### Example 6 - NKG2D antibodies show high thermostability

Melting temperatures of NKG2D-binding domains were assayed using differential scanning fluorimetry. The extrapolated apparent melting temperatures are high relative to typical IgG1 antibodies (FIG. 18).

### Example 7 - Synergistic activation of human NK cells by cross-linking NKG2D and CD16

### Primary human NK cell activation assay

Peripheral blood mononuclear cells (PBMCs) were isolated from peripheral human blood buffy coats using density gradient centrifugation. NK cells were purified from PBMCs using negative magnetic beads (StemCell # 17955). NK cells were >90% CD3⁻ CD56⁺ as determined by flow cytometry. Cells were then expanded 48 hours in media containing 100 ng/mL hIL-2 (Peprotech #200-02) before use in activation assays. Antibodies were coated onto a 96-well flat-bottom plate at a concentration of 2 µg/mL (anti-CD 16, Biolegend # 302013) and 5 µg/mL (anti-NKG2D, R&D #MAB139) in 100 µL sterile PBS overnight at 4 °C followed by washing the wells thoroughly to remove excess antibody. For the assessment of degranulation IL-2-activated NK cells were resuspended at 5×10⁵ cells/mL in culture media supplemented with 100 ng/mL human IL-2 (hIL2) and 1 µg/mL APC-conjugated anti-CD107a mAb (Biolegend # 328619). 1×10⁵ cells/well were then added onto antibody coated plates. The protein transport inhibitors Brefeldin A (BFA, Biolegend # 420601) and Monensin (Biolegend # 420701) were added at a final dilution of 1:1000 and 1:270, respectively. Plated cells were incubated for 4 hours at 37 °C in 5% CO₂. For intracellular staining of IFN-γ, NK cells were labeled with anti-CD3 (Biolegend #300452) and anti-CD56 mAb (Biolegend # 318328), and subsequently fixed, permeabilized and labeled with anti-IFN-y mAb (Biolegend # 506507). NK cells were analyzed for expression of CD107a and IFN-γ by flow cytometry after gating on live CD56⁺CD3⁻cells.

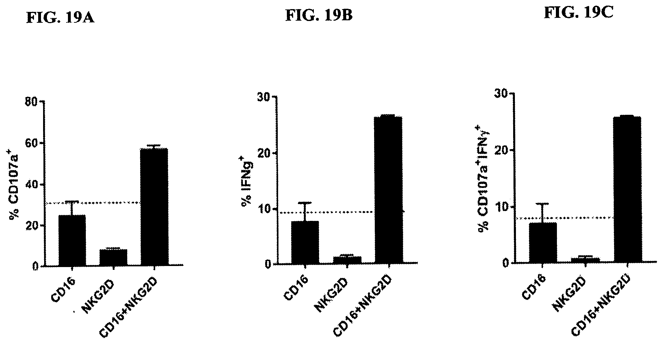

To investigate the relative potency of receptor combination, crosslinking of NKG2D or CD16, and co-crosslinking of both receptors by plate-bound stimulation was performed. As shown in Figure 19 (FIGs. 19A-19C), combined stimulation of CD16 and NKG2D resulted in highly elevated levels of CD107a (degranulation) (FIG. 19A) and/or IFN-γ production (FIG. 19B). Dotted lines represent an additive effect of individual stimulations of each receptor.

CD107a levels and intracellular IFN-γ production of IL-2-activated NK cells were analyzed after 4 hours of plate-bound stimulation with anti-CD 16, anti-NKG2D or a combination of both monoclonal antibodies. Graphs indicate the mean (n = 2) ± Sd. FIG. 19A demonstrates levels of CD107a; FIG. 19B demonstrates levels of IFN-γ; FIG. 19C demonstrates levels of CD107a and IFN-γ. Data shown in FIGs. 19A-19C are representative of five independent experiments using five different healthy donors.

### Example 8 - Assessment of TriNKET or mAb binding to cell expressed human cancer antigens

Human cancer cell lines expressing EGFR *(e.g.,* H2172, H747, H1975, N87, HCT116, and A549 cell lines) were used to assess tumor antigen binding of TriNKETs derived from different EGFR targeting monoclonal antibodies (mAbs). TriNKETs tested include A49-F3'-TriNKET-EGFR-panitumumab (an NKG2D-binding domain from clone ADI-27749 and an scFv targeting EGFR derived from an EGFR monoclonal antibody panitumumab), A49-F3'-TriNKET-EGFR-necitumumab (an NKG2D-binding domain from clone ADI-27749 and an scFv targeting EGFR derived from monoclonal antibody necitumumab), and A49-F3'-TriNKET-EGFR-AdiCLC3 (an NKG2D-binding domain from clone ADI-27749 and an scFv targeting EGFR derived from monoclonal antibody AdiCLC2).

TriNKETs or mAbs were diluted and incubated with the respective cell lines. Binding of the TriNKET or mAbs was detected using a fluorophore-conjugated anti-human IgG secondary antibody. Cells were analyzed by flow cytometry, and binding median fluorescent intensity (MFI) to cell-expressed EGFR by TriNKETs and mAbs was normalized to the maximal signal to obtain percentage of maximal signal values for TriNKETs and mAbs.

### Primary human NK cell cytotoxicity assay

PBMCs were isolated from human peripheral blood buffy coats using density gradient centrifugation. Isolated PBMCs were washed and prepared for NK cell isolation. NK cells were isolated using a negative selection technique with magnetic beads. Purity of isolated NK cells achieved was typically greater than 90% CD3⁻ CD56⁺. Isolated NK cells were incubated overnight without cytokine, and used the following day in cytotoxicity assays.

KHYG-1 cells transduced to express CD16-F158V were used to investigate the contribution of dual NKG2D and CD16 stimulation. KHYG-1 CD16-F158V cells were maintained in 10% HI-FBS-RPMI-1640 with 10ng/mL IL-2. The day before use as effector cells in killing assays, cells were harvest from culture, and IL-2 was washed out. KHYG-1 CD16-F158V cells were resuspended in 10% HI-FBS-RPMI-1640 and were incubated overnight without cytokine.

### DELFIA cytotoxicity assay

Human cancer cell lines expressing a target of interest were harvested from culture, washed with HBS, and resuspended in growth media at 10⁶ cells/mL for labeling with BATDA reagent (Perkin Elmer, AD0116). Manufacturer instructions were followed for labeling of the target cells. After labeling, cells were washed 3 times with HBS and resuspended at 0.5x10⁵ cells/mL in culture media. To prepare the background wells, an aliquot of the labeled cells was put aside, and the cells were spun out of the media. 100 µL of the media was carefully added to wells in triplicate to avoid disturbing the pelleted cells. 100 µL of BATDA-labeled cells were added to each well of the 96-well plate. Wells were saved for spontaneous release from target cells and prepared for lysis of target cells by addition of 1% Triton-X. Monoclonal antibodies or TriNKETs against the tumor target of interest were diluted in culture media, and 50 µL of diluted mAb or TriNKET was added to each well. Rested NK cells were harvested from culture, washed, and resuspended at 1.0x10⁵-2.0x10⁶ cell/mL in culture media, depending on the desired effector to target cell ratio. 50 µL of NK cells were added to each well of the plate to provide a total of 200 µL culture volume. The plate was incubated at 37 °C with 5% CO₂ for 2-4 hours before developing the assay.

After culturing for 2-4 hours, the plate was removed from the incubator and the cells were pelleted by centrifugation at 200xg for 5 minutes. 20 µL of culture supernatant was transferred to a clean microplate provided from the manufacturer, and 200 µL of room temperature Europium solution was added to each well. The plate was protected from light and incubated on a plate shaker at 250 rpm for 15 minutes. The plate was read using a SpectraMax® i3X instrument (Molecular Devices), and percent specific lysis was calculated (% Specific lysis = (Experimental release - Spontaneous release) / (Maximum release - Spontaneous release)) x 100).

### Cell antigen binding

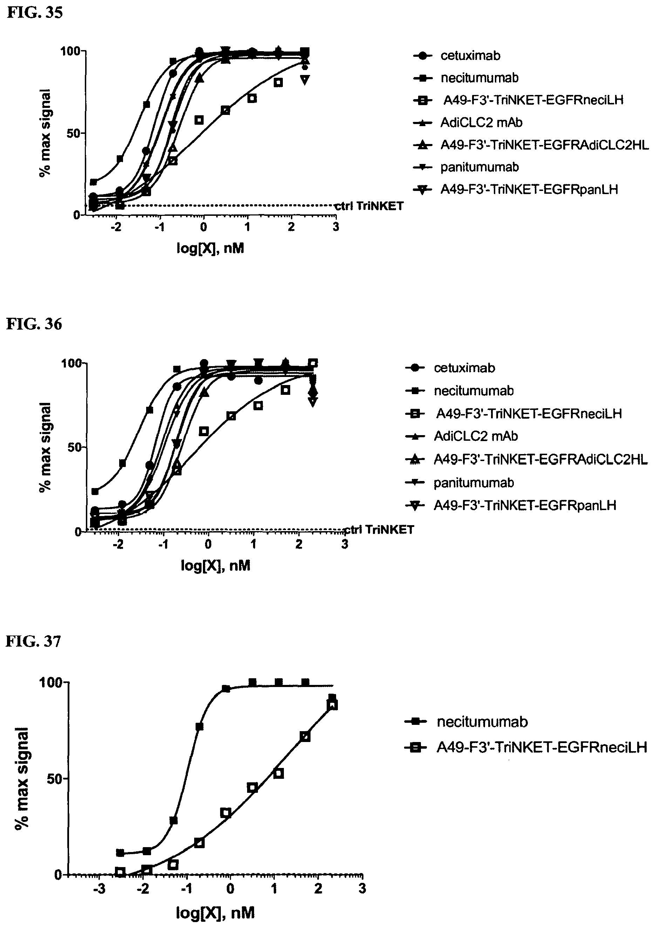

FIG. 35 shows binding of TriNKETs and mAbs to EGFR expressed on NCI-H2172 human lung cancer cells. FIG. 36 shows binding of TriNKETs and mAbs to EGFR expressed on HCC827 human lung cancer cells. FIG. 37 shows binding of TriNKETs and mAbs to EGFR expressed on NCI-H747 human colon cancer cells. Cells were treated with TriNKETs or monoclonal antibodies at concentrations indicated in the graphs of FIGs. 35-37.

### Primary human NK cytotoxicity assay

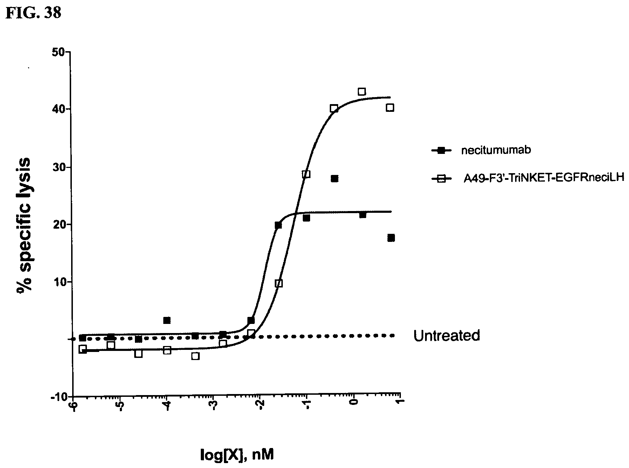

FIGs. 38-46 show TriNKET-mediated cytotoxicity of rested human NK cells or KHYG1-CD16V cells against various cell types. TriNKETs killed target cells more effectively than their parental mAbs.

Cells were treated with TriNKETs or monoclonal antibodies at concentrations indicated in each graph. The effector-to-target ratio was 10:1 in each experiment. FIG. 38 shows TriNKET-mediated (A49-F3'-TriNKET-EGFR-neciLH) and monoclonal antibody-mediated (necitumumab) killing of NCI-H2172 cells (lung, EGFR L858R T790M) with rested human NK cells (DELFIA assay).

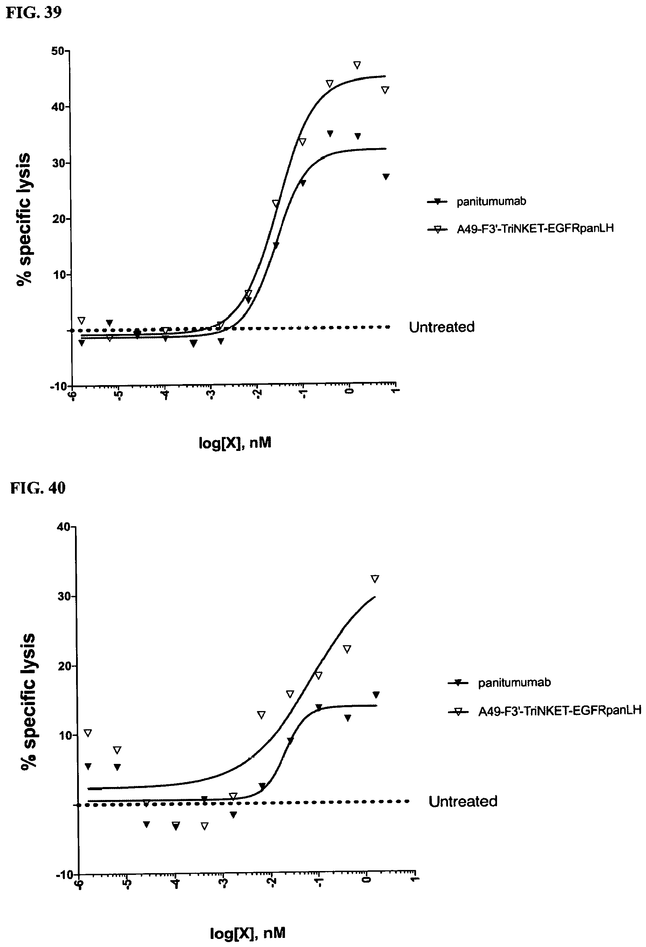

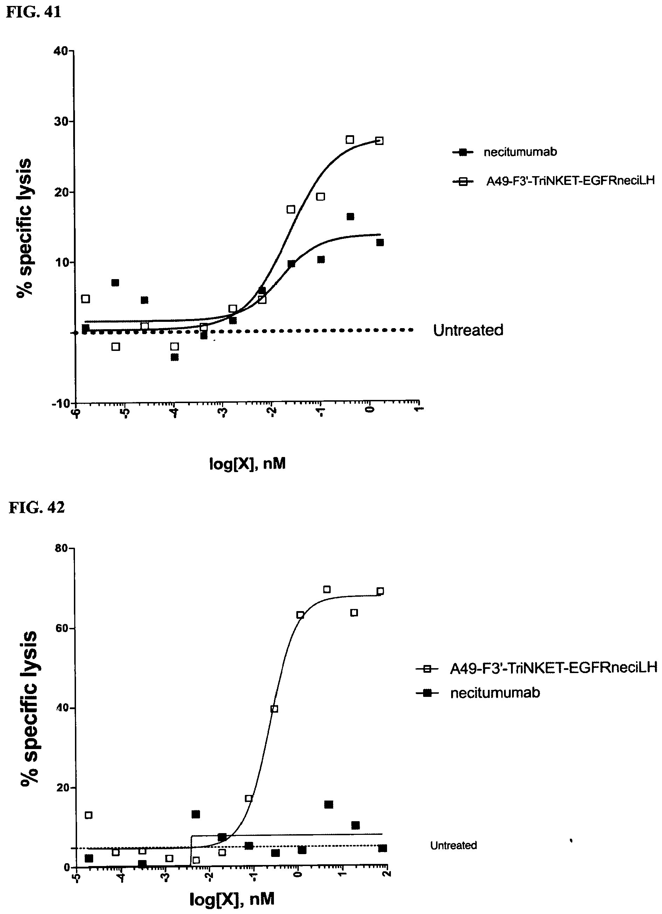

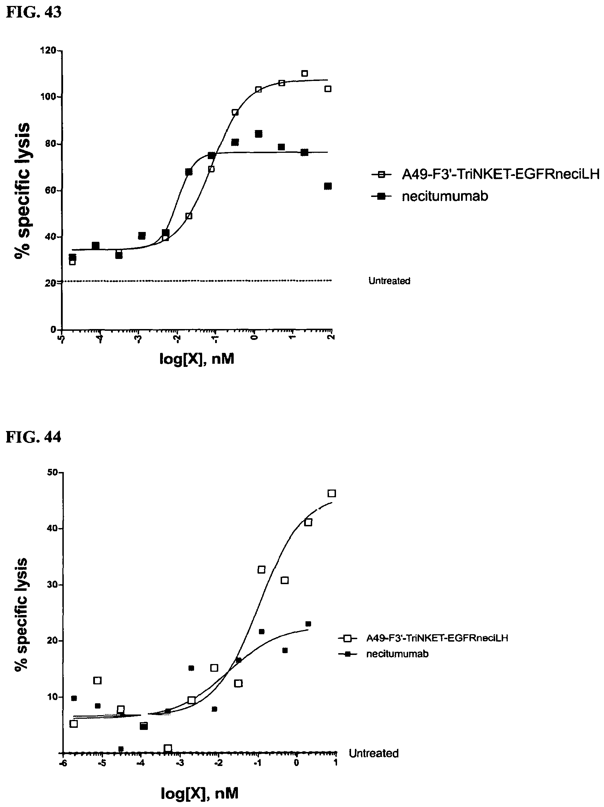

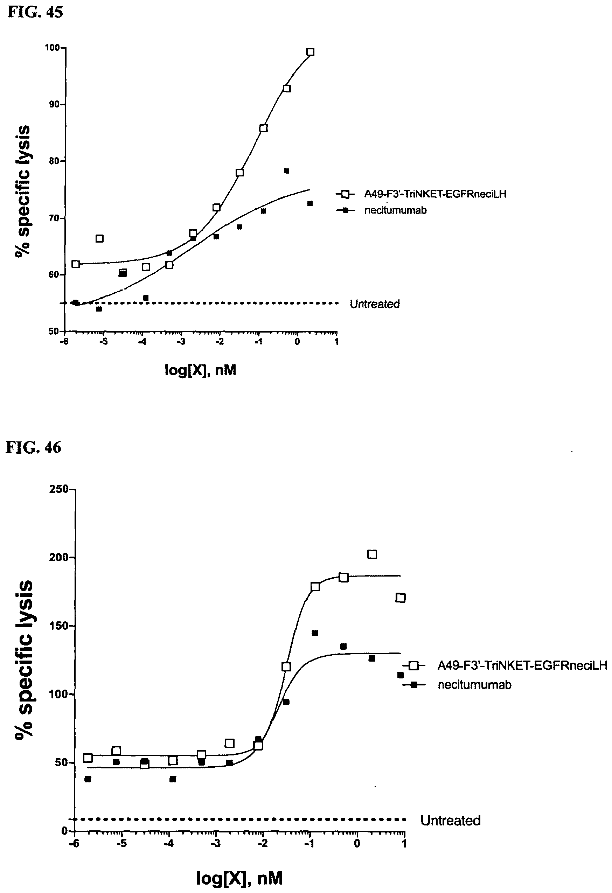

FIG. 39 shows TriNKET-mediated (A49-F3'-TriNKET-EGFR- panLH)) and monoclonal antibody-mediated (panitumumab) killing of NCI-H2172 cells (lung, EGFR L858R T790M) with rested human NK cells (DELFIA assay). FIG. 40 shows TriNKET-mediated (A49-F3'-TriNKET-EGFR- panitumumabLH (panLH)) and monoclonal antibody-mediated (panitumumab) killing of NCI-H747 cells (colon, KRAS G13D) with rested human NK cells (DELFIA assay). FIG. 41 shows TriNKET-mediated (A49-F3'-TriNKET-EGFR-necitumumabLH (neciLH)) and monoclonal antibody-mediated (necitumumab) killing of NCI-H747 cells (colon, KRAS G13D) with rested human NK cells (DELFIA assay). FIG. 42 shows TriNKET-mediated (A49-F3'-TriNKET-EGFR-necitumumabLH (neciLH)) and monoclonal antibody-mediated (necitumumab) killing of NCI-H2172 cells (lung, EGFR L858R T790M) with KHYG1-CD16V cells (DELFIA assay). FIG. 43 shows TriNKET-mediated (A49-F3'-TriNKET-EGFR-necitumumabLH (neciLH)) and monoclonal antibody-mediated (necitumumab) killing of NCI-H1975 cells (lung, EGFR L858R) with KHYG1-CD16V cells (DELFIA assay). FIG. 44 shows TriNKET-mediated (A49-F3'-TriNKET-EGFR-necitumumabLH (neciLH)) and monoclonal antibody-mediated (necitumumab) killing of NCI-N87 cells (gastric) with KHYG1-CD16V cells (DELFIA assay). FIG. 45 shows TriNKET-mediated (A49-F3'-TriNKET-EGFR-necitumumabLH (neciLH)) and monoclonal antibody-mediated (necitumumab) killing of HCT116 cells (colon, KRAS G13D) with KHYG1-CD16V cells (DELFIA assay). FIG. 46 shows TriNKET-mediated (A49-F3'-TriNKET-EGFR-necitumumabLH (neciLH)) and monoclonal antibody-mediated (necitumumab) killing of A549 cells (lung, KRAS G12S) with KHYG1-CD16V cells (DELFIA assay).

In all experiments, TriNKETs killed target cells more effectively than their parental mAbs. These results demonstrate the improved efficacy of the disclosed TriNKETs in facilitating targeted cell death as compared to mAbs targeting the same antigens.

### INCORPORATION BY REFERENCE

The entire disclosure of each of the patent documents and scientific articles referred to herein is incorporated by reference for all purposes.

### EQUIVALENTS

The invention may be embodied in other specific forms without departing from the spirit or essential characteristics thereof. The foregoing embodiments are therefore to be considered in all respects illustrative rather than limiting the invention described herein. Scope of the invention is thus indicated by the appended claims rather than by the foregoing description, and all changes that come within the meaning and range of equivalency of the claims are intended to be embraced therein.

### Embodiments of the Invention

1. A protein comprising:
   (a) a first antigen-binding site that binds NKG2D;
   (b) a second antigen-binding site that binds EGFR, HLA-E, CCR4, or PD-L1; and
   (c) an antibody Fc domain or a portion thereof sufficient to bind CD16, or a third antigen-binding site that binds CD16.
2. A protein comprising:
   (a) a first antigen-binding site that binds NKG2D;
   (b) a second antigen-binding site that binds EGFR; and
   (c) an antibody Fc domain or a portion thereof sufficient to bind CD16, or a third antigen-binding site that binds CD16.
3. A protein comprising:
   (a) a first antigen-binding site that binds NKG2D;
   (b) a second antigen-binding site that binds HLA-E; and
   (c) an antibody Fc domain or a portion thereof sufficient to bind CD16, or a third antigen-binding site that binds CD16.
4. A protein comprising:
   (a) a first antigen-binding site that binds NKG2D;
   (b) a second antigen-binding site that binds CCR4; and
   (c) an antibody Fc domain or a portion thereof sufficient to bind CD16, or a third antigen-binding site that binds CD16.
5. A protein comprising:
   (a) a first antigen-binding site that binds NKG2D;
   (b) a second antigen-binding site that binds PD-L1; and
   (c) an antibody Fc domain or a portion thereof sufficient to bind CD16, or a third antigen-binding site that binds CD16.
6. The protein of any one of embodiments 1-5, wherein the first antigen-binding site binds to human NKG2D.
7. The protein of any one of embodiments 1-5 or 6, wherein the first antigen-binding site comprises a heavy chain variable domain and a light chain variable domain.
8. The protein of embodiment 7, wherein the heavy chain variable domain and the light chain variable domain are present on the same polypeptide.
9. A protein according to embodiment 7 or 8, wherein the second antigen-binding site comprises a heavy chain variable domain and a light chain variable domain.
10. The protein of embodiment 9, wherein the heavy chain variable domain and the light chain variable domain of the second antigen-binding site are present on the same polypeptide.
11. A protein according to embodiment 9 or 10, wherein the light chain variable domain of the first antigen-binding site has an amino acid sequence identical to the amino acid sequence of the light chain variable domain of the second antigen-binding site.
12. A protein comprising:
   (a) a first antigen-binding site comprising an Fab fragment that binds NKG2D;
   (b) a second antigen-binding site comprising a single-chain variable fragment (scFv) that binds EGFR; and
   (c) an antibody Fc domain or a portion thereof sufficient to bind CD16, or a third antigen-binding site that binds CD16.
13. The protein of embodiment 12, wherein the scFv is linked to the antibody Fc domain or a portion thereof sufficient to bind CD 16, or the third antigen-binding site that binds CD16, via a hinge comprising Ala-Ser, wherein the scFv comprises a heavy chain variable domain and a light chain variable domain.
14. The protein of embodiment 13 wherein the scFv is linked to the antibody Fc domain.
15. The protein of embodiment 13 or 14, wherein the heavy chain variable domain of the scFv forms a disulfide bridge with the light chain variable domain of the scFv.
16. The protein of embodiment 15, wherein the disulfide bridge is formed between C44 from the heavy chain variable domain and C100 from the light chain variable domain.
17. The protein of embodiment 16, wherein the scFv is linked to the antibody Fc domain, wherein the light chain variable domain of the scFv is positioned at the N-terminus of the heavy chain variable domain of the scFv, and is linked to the heavy chain variable domain of the scFv via a flexible linker (GlyGlyGlyGlySer)₄ ((G4S)₄), and the Fab is linked to the antibody Fc domain.
18. A protein according to any one of embodiments 13-17, wherein the heavy chain variable domain of the scFv is linked to the light chain variable domain of the scFv via a flexible linker.
19. The protein of embodiment 18, wherein the flexible linker comprises (GlyGlyGlyGlySer)₄ ((G4S)₄).
20. A protein according to any one of embodiments 13-19, wherein the heavy chain variable domain of the scFv is positioned at the N-terminus or the C-terminus of the light chain variable domain of the scFv.
21. The protein of embodiment 20, wherein the light chain variable domain of the scFv is positioned at the N-terminus of the heavy chain variable domain of the scFv.
22. A protein according to any one of embodiments 12 to 21, wherein the Fab fragment is linked to the antibody Fc domain or a portion thereof sufficient to bind CD 16 or the third antigen-binding site that binds CD16.
23. The protein of embodiment 22, wherein the heavy chain portion of the Fab fragment comprises a heavy chain variable domain and a CH1 domain, and wherein the heavy chain variable domain is linked to the CH1 domain.
24. A protein according to embodiment 22 or 23, wherein the Fab is linked to the antibody Fc domain.
25. A protein according to any one of embodiments 12 to 24 comprising a sequence selected from SEQ ID NO:264, SEQ IS NO:265, and SEQ ID NO:266.
26. A protein according to any one of embodiments 13-25 comprising an scFv linked to an antibody Fc domain, wherein the scFv linked to the antibody Fc domain is represented by a sequence selected from SEQ ID NO:267, SEQ ID NO:268, and SEQ ID NO:269.
27. A protein according to any one of embodiments 13-25 comprising a sequence selected from SEQ ID NO:267, SEQ ID NO:268, and SEQ ID NO:269.
28. A protein according to any one of embodiments 13-24 comprising a sequence at least 90% identical to an amino acid sequence selected from SEQ ID NO:264, SEQ IS NO:265, and SEQ ID NO:266.
29. A protein according to any one of embodiments 13-24 comprising a sequence at least 95% identical to an amino acid sequence selected from SEQ ID NO:264, SEQ IS NO:265, and SEQ ID NO:266.
30. A protein according to any one of embodiments 13-24 comprising a sequence at least 99% identical to an amino acid sequence selected from SEQ ID NO:264, SEQ IS NO:265, and SEQ ID NO:266.
31. A protein according to any one of embodiments 13-26 comprising a sequence at least 90% identical to an amino acid sequence selected from SEQ ID NO:267, SEQ ID NO:268, and SEQ ID NO:269.
32. A protein according to any one of embodiments 13-29 comprising a sequence at least 95% identical to an amino acid sequence selected from SEQ ID NO:267, SEQ ID NO:268, and SEQ ID NO:269.
33. A protein according to any one of embodiments 13-29 comprising a sequence at least 99% identical to an amino acid sequence selected from SEQ ID NO:267, SEQ ID NO:268, and SEQ ID NO:269.
34. A protein according to any one of embodiments 1-32, wherein the first antigen-binding site comprises a heavy chain variable domain at least 90% identical to an amino acid sequence selected from: SEQ ID NO:1, SEQ ID NO:41, SEQ ID NO:49, SEQ ID NO:57, SEQ ID NO:59, SEQ ID NO:61, SEQ ID NO:69, SEQ ID NO:77, SEQ ID NO:85, and SEQ ID NO:93.
35. A protein according to any one of embodiments 1-32, wherein the first antigen-binding site comprises a heavy chain variable domain at least 90% identical to SEQ ID NO:41 and a light chain variable domain at least 90% identical to SEQ ID NO:42.
36. A protein according to any one of embodiments 1-32, wherein the first antigen-binding site comprises a heavy chain variable domain at least 90% identical to SEQ ID NO:49 and a light chain variable domain at least 90% identical to SEQ ID NO:50.
37. A protein according to any one of embodiments 1-32, wherein the first antigen-binding site comprises a heavy chain variable domain at least 90% identical to SEQ ID NO:57 and a light chain variable domain at least 90% identical to SEQ ID NO:58.
38. A protein according to any one of embodiments 1-32, wherein the first antigen-binding site comprises a heavy chain variable domain at least 90% identical to SEQ ID NO:59 and a light chain variable domain at least 90% identical to SEQ ID NO:60.
39. A protein according to any one of embodiments 1-32, wherein the first antigen-binding site comprises a heavy chain variable domain at least 90% identical to SEQ ID NO:61 and a light chain variable domain at least 90% identical to SEQ ID NO:62.
40. A protein according to any one of embodiments 1-32, wherein the first antigen-binding site comprises a heavy chain variable domain at least 90% identical to SEQ ID NO:69 and a light chain variable domain at least 90% identical to SEQ ID NO:70.
41. A protein according to any one of embodiments 1-32, wherein the first antigen-binding site comprises a heavy chain variable domain at least 90% identical to SEQ ID NO:77 and a light chain variable domain at least 90% identical to SEQ ID NO:78.
42. A protein according to any one of embodiments 1-32, wherein the first antigen-binding site comprises a heavy chain variable domain at least 90% identical to SEQ ID NO:85 and a light chain variable domain at least 90% identical to SEQ ID NO:86.
43. A protein according to any one of embodiments 1-32, wherein the first antigen-binding site comprises a heavy chain variable domain at least 90% identical to SEQ ID NO:93 and a light chain variable domain at least 90% identical to SEQ ID NO:94.
44. A protein according to any one of embodiments 1-32, wherein the first antigen-binding site comprises a heavy chain variable domain at least 90% identical to SEQ ID NO: 101 and a light chain variable domain at least 90% identical to SEQ ID NO: 102.
45. A protein according to any one of embodiments 1-32, wherein the first antigen-binding site comprises a heavy chain variable domain at least 90% identical to SEQ ID NO: 103 and a light chain variable domain at least 90% identical to SEQ ID NO: 104.
46. The protein of any one of embodiments 1-6, wherein the first antigen-binding site comprises a single-domain antibody.
47. The protein of embodiment 46, wherein the single-domain antibody comprises a V_{H}H fragment or a V_{NAR} fragment.
48. A protein according to any one of embodiments 1-6, 46, or 47, wherein the second antigen-binding site comprises a heavy chain variable domain and a light chain variable domain.
49. The protein of embodiment 47, wherein the heavy chain variable domain and the light chain variable domain of the second antigen-binding site are present on the same polypeptide.
50. A protein according to any one of embodiments 1-24, wherein the second antigen-binding site binds EGFR, the heavy chain variable domain of the second antigen-binding site comprises an amino acid sequence at least 90% identical to SEQ ID NO: 151 and the light chain variable domain of the second antigen-binding site comprises an amino acid sequence at least 90% identical to SEQ ID NO:152.
51. A protein according to any one of embodiments 1-24, wherein the second antigen-binding site binds EGFR, the heavy chain variable domain of the second antigen-binding site comprises an amino acid sequence at least 90% identical to SEQ ID NO: 153 and the light chain variable domain of the second antigen-binding site comprises an amino acid sequence at least 90% identical to SEQ ID NO:154.
52. A protein according to any one of embodiments 1-24, wherein the second antigen-binding site binds EGFR, the heavy chain variable domain of the second antigen-binding site comprises an amino acid sequence at least 90% identical to SEQ ID NO: 155 and the light chain variable domain of the second antigen-binding site comprises an amino acid sequence at least 90% identical to SEQ ID NO:156.
53. A protein according to any one of embodiments 1-24, wherein the second antigen-binding site binds EGFR, the heavy chain variable domain of the second antigen-binding site comprises an amino acid sequence at least 90% identical to SEQ ID NO: 157 and the light chain variable domain of the second antigen-binding site comprises an amino acid sequence at least 90% identical to SEQ ID NO:158.
54. A protein according to any one of embodiments 1-24, wherein the second antigen-binding site binds EGFR, the heavy chain variable domain of the second antigen-binding site comprises an amino acid sequence at least 90% identical to SEQ ID NO: 159 and the light chain variable domain of the second antigen-binding site comprises an amino acid sequence at least 90% identical to SEQ ID NO:160.
55. A protein according to any one of embodiments 1-24, wherein the second antigen-binding site binds EGFR, the heavy chain variable domain of the second antigen-binding site comprises an amino acid sequence at least 90% identical to SEQ ID NO: 161 and the light chain variable domain of the second antigen-binding site comprises an amino acid sequence at least 90% identical to SEQ ID NO:162.
56. A protein according to any one of embodiments 1-24, wherein the second antigen-binding site binds EGFR, the heavy chain variable domain of the second antigen-binding site comprises an amino acid sequence at least 90% identical to SEQ ID NO: 163 and the light chain variable domain of the second antigen-binding site comprises an amino acid sequence at least 90% identical to SEQ ID NO:164.
57. A protein according to any one of embodiments 1-11 and 33-48, wherein the second antigen-binding site binds PD-L1, the heavy chain variable domain of the second antigen-binding site comprises an amino acid sequence at least 90% identical to SEQ ID NO: 167 and the light chain variable domain of the second antigen-binding site comprises an amino acid sequence at least 90% identical to SEQ ID NO: 171.
58. A protein according to any one of embodiments 1-11 and 33-48, wherein the second antigen-binding site binds PD-L1, the heavy chain variable domain of the second antigen-binding site comprises an amino acid sequence at least 90% identical to SEQ ID NO: 175 and the light chain variable domain of the second antigen-binding site comprises an amino acid sequence at least 90% identical to SEQ ID NO: 179.
59. A protein according to any one of embodiments 1-11 and 33-48, wherein the second antigen-binding site binds PD-L1, the heavy chain variable domain of the second antigen-binding site comprises an amino acid sequence at least 90% identical to SEQ ID NO:183 and the light chain variable domain of the second antigen-binding site comprises an amino acid sequence at least 90% identical to SEQ ID NO:187.
60. A protein according to any one of embodiments 1-11 and 33-48, wherein the second antigen-binding site binds CCR4, the heavy chain variable domain of the second antigen-binding site comprises an amino acid sequence at least 90% identical to SEQ ID NO:192 and the light chain variable domain of the second antigen-binding site comprises an amino acid sequence at least 90% identical to SEQ ID NO:196.
61. A protein according to any one of embodiments 1-11 and 33-48, wherein the second antigen-binding site binds CCR4, the heavy chain variable domain of the second antigen-binding site comprises an amino acid sequence at least 90% identical to SEQ ID NO:200 and the light chain variable domain of the second antigen-binding site comprises an amino acid sequence at least 90% identical to SEQ ID NO:204.
62. A protein according to any one of embodiments 1-11 and 33-48, wherein the second antigen-binding site binds CCR4, the heavy chain variable domain of the second antigen-binding site comprises an amino acid sequence at least 90% identical to SEQ ID NO:208 and the light chain variable domain of the second antigen-binding site comprises an amino acid sequence at least 90% identical to SEQ ID NO:212.
63. A protein according to any one of embodiments 1-11, wherein the second antigen-binding site comprises a single-domain antibody.
64. The protein of embodiment 62, wherein the single-domain antibody of the second antigen-binding site comprises a V_{H}H fragment or a V_{NAR} fragment.
65. A protein according to any one of embodiments 1-63, wherein the antibody Fc domain comprises a hinge and a CH2 domain.
66. The protein of embodiment 64, wherein the antibody Fc domain comprises a hinge and a CH2 domain of a human IgG1 antibody.
67. The protein of embodiment 65 or 66, wherein the Fc domain comprises an amino acid sequence at least 90% identical to amino acids 234-332 of a human IgG1 antibody.
68. The protein of embodiment 67, wherein the Fc domain comprises an amino acid sequence at least 90% identical to the Fc domain of human IgG1 and differs at one or more positions selected from the group consisting of: Q347, Y349, L351, S354, E356, E357, K360, Q362, S364, T366, L368, K370, N390, K392, T394, D399, S400, D401, F405, Y407, K409, T411, and K439.
69. A protein according to any one of embodiments 1-67, wherein the protein binds to NKG2D with a K_{D} of 10 nM or weaker affinity.
70. A formulation comprising a protein according to any one of the preceding embodiments and a pharmaceutically acceptable carrier.
71. A cell comprising one or more nucleic acids expressing a protein according to any one of embodiments 1-68.
72. A method of enhancing tumor cell death, the method comprising exposing a tumor cell and a natural killer cell to an effective amount of the protein according to any one of embodiments 1-69, wherein the tumor cell expresses at least one of EGFR, HLA-E, CCR4, or PD-L1.
73. A method of treating cancer, wherein the method comprises administering an effective amount of the protein according to any one of embodiments 1-69 or the formulation according to embodiment 70 to a patient.
74. The method of embodiment 73, wherein the second antigen binding site of the protein binds EGFR, and wherein the cancer is selected from the group consisting of head and neck cancer, colorectal cancer, non-small cell lung cancer, glioma, renal cell carcinoma, bladder cancer, cervical cancer, ovarian cancer, pancreatic cancer, and liver cancer.
75. The method of embodiment 73, wherein the second antigen binding site of the protein binds HLA-E, and wherein the cancer is selected from the group consisting of lymphoma, head and neck cancer, bladder cancer, cervical cancer, lung cancer, renal cancer, melanoma, colorectal cancer, ovarian cancer, glioblastoma, and a sarcoma.
76. The method of embodiment 73, wherein the second antigen binding site of the protein binds PD-L1, and wherein the cancer is selected from the group consisting of lymphoma, leukemia, multiple myeloma, head and neck cancer, bladder cancer, cervical cancer, lung cancer, renal cancer, melanoma, colorectal cancer, ovarian cancer, glioblastoma, a sarcoma, and gastric cancer.
77. The method of embodiment 73, wherein the second antigen binding site of the protein binds CCR4, and wherein the cancer is selected from the group consisting of adult T-cell lymphoma/leukemia, peripheral T cell lymphoma, cutaneous T cell lymphoma, chronic lymphocytic leukemia, a B cell malignancy, non-Hodgkin's lymphoma, Hodgkin's lymphoma, anaplastic large cell lymphoma, mature T/natural killer (NK) cell neoplasms, thymoma, gastric cancer, and renal cell carcinoma.

## Claims

1. A protein comprising:
(a) a first antigen-binding site that binds NKG2D;
(b) a second antigen-binding site that binds EGFR, CCR4, or PD-L1; and
(c) an antibody Fc domain or a portion thereof sufficient to bind CD16, or a third antigen-binding site that binds CD16.

2. A protein comprising:
i)
(a) a first antigen-binding site that binds NKG2D;
(b) a second antigen-binding site that binds EGFR; and
(c) an antibody Fc domain or a portion thereof sufficient to bind CD16, or a third antigen-binding site that binds CD16, or
ii)
(a) a first antigen-binding site that binds NKG2D;
(b) a second antigen-binding site that binds CCR4; and
(c) an antibody Fc domain or a portion thereof sufficient to bind CD16, or a third antigen-binding site that binds CD16, or
iii)
(a) a first antigen-binding site that binds NKG2D;
(b) a second antigen-binding site that binds PD-L1; and
(c) an antibody Fc domain or a portion thereof sufficient to bind CD16, or a third antigen-binding site that binds CD16.

3. The protein of claims 1 or 2, wherein the first antigen-binding site binds to human and cynomolgus monkey NKG2D.

4. The protein of any one of claims 1-3, wherein the first antigen-binding site comprises a heavy chain variable domain and a light chain variable domain, optionally wherein the heavy chain variable domain and the light chain variable domain are present on the same polypeptide.

5. A protein according to claim 4, wherein the second antigen-binding site comprises a heavy chain variable domain and a light chain variable domain, optionally wherein the heavy chain variable domain and the light chain variable domain of the second antigen-binding site are present on the same polypeptide.

6. A protein according to claim 5, wherein the light chain variable domain of the first antigen-binding site has an amino acid sequence identical to the amino acid sequence of the light chain variable domain of the second antigen-binding site.

7. A protein comprising:
(a) a first antigen-binding site comprising an Fab fragment that binds NKG2D;
(b) a second antigen-binding site comprising a single-chain variable fragment (scFv) that binds EGFR; and
(c) an antibody Fc domain or a portion thereof sufficient to bind CD16, or a third antigen-binding site that binds CD16.

8. The protein of claim 7, wherein the scFv is linked to the antibody Fc domain or a portion thereof sufficient to bind CD 16, or the third antigen-binding site that binds CD 16, via a hinge comprising Ala-Ser, wherein the scFv comprises a heavy chain variable domain and a light chain variable domain, optionally wherein the scFv is linked to the antibody Fc domain.

9. The protein of claim 8, wherein the heavy chain variable domain of the scFv forms a disulfide bridge with the light chain variable domain of the scFv,
optionally wherein the disulfide bridge is formed between C44 from the heavy chain variable domain and C100 from the light chain variable domain,
further optionally wherein the scFv is linked to the antibody Fc domain, wherein the light chain variable domain of the scFv is positioned at the N-terminus of the heavy chain variable domain of the scFv, and is linked to the heavy chain variable domain of the scFv via a flexible linker (GlyGlyGlyGlySer)₄ ((G4S)₄), and the Fab is linked to the antibody Fc domain.

10. A protein according to any one of claims 8-9, wherein the heavy chain variable domain of the scFv is linked to the light chain variable domain of the scFv via a flexible linker, optionally wherein the flexible linker comprises (GlyGlyGlyGlySer)₄ ((G4S)₄).

11. A protein according to any one of claims 8-10, wherein the heavy chain variable domain of the scFv is positioned at the N-terminus or the C-terminus of the light chain variable domain of the scFv, optionally wherein the light chain variable domain of the scFv is positioned at the N-terminus of the heavy chain variable domain of the scFv.

12. A protein according to any one of claims 8-11, wherein the Fab fragment is linked to the antibody Fc domain or a portion thereof sufficient to bind CD16 or the third antigen-binding site that binds CD 16,
optionally wherein the heavy chain portion of the Fab fragment comprises a heavy chain variable domain and a CH1 domain, and wherein the heavy chain variable domain is linked to the CH1 domain,
further optionally wherein the Fab is linked to the antibody Fc domain.

13. A protein according to any one of claims 8-12, comprising:
a) a sequence selected from SEQ ID NO:264, SEQ ID NO:265, and SEQ ID NO:266, or
b) a sequence at least 90%, 95% or 99% identical to an amino acid sequence selected from SEQ ID NO:264, SEQ ID NO:265, and SEQ ID NO:266.

14. A protein according to any one of claims 8-12, comprising:
a) an scFv linked to an antibody Fc domain, wherein the scFv linked to the antibody Fc domain is represented by a sequence selected from SEQ ID NO:267, SEQ ID NO:268, and SEQ ID NO:269,
b) a sequence selected from SEQ ID NO:267, SEQ ID NO:268, and SEQ ID NO:269, or
c) a sequence at least 90%, 95% or 99% identical to an amino acid sequence selected from SEQ ID NO:267, SEQ ID NO:268, and SEQ ID NO:269.

15. A protein according to any one of claims 1-14, wherein the first antigen-binding site comprises a heavy chain variable domain amino acid sequence at least 90% identical to an amino acid sequence selected from: SEQ ID NO:85, SEQ ID NO:1, SEQ ID NO:41, SEQ ID NO:49, SEQ ID NO:57, SEQ ID NO:59, SEQ ID NO:61, SEQ ID NO:69, SEQ ID NO:77, and SEQ ID NO:93.

16. A protein according to any one of claims 1-14, wherein
a) the first antigen-binding site comprises a heavy chain variable domain amino acid sequence at least 90% identical to SEQ ID NO:85 and a light chain variable domain amino acid sequence at least 90% identical to SEQ ID NO:86,
b) the first antigen-binding site comprises a heavy chain variable domain amino acid sequence at least 90% identical to SEQ ID NO:41 and a light chain variable domain amino acid sequence at least 90% identical to SEQ ID NO:42,
c) the first antigen-binding site comprises a heavy chain variable domain amino acid sequence at least 90% identical to SEQ ID NO:49 and a light chain variable domain amino acid sequence at least 90% identical to SEQ ID NO:50,
d) the first antigen-binding site comprises a heavy chain variable domain amino acid sequence at least 90% identical to SEQ ID NO:57 and a light chain variable domain amino acid sequence at least 90% identical to SEQ ID NO:58,
e) the first antigen-binding site comprises a heavy chain variable domain amino acid sequence at least 90% identical to SEQ ID NO:59 and a light chain variable domain amino acid sequence at least 90% identical to SEQ ID NO:60,
f) the first antigen-binding site comprises a heavy chain variable domain amino acid sequence at least 90% identical to SEQ ID NO:61 and a light chain variable domain amino acid sequence at least 90% identical to SEQ ID NO:62,
g) the first antigen-binding site comprises a heavy chain variable domain amino acid sequence at least 90% identical to SEQ ID NO:69 and a light chain variable domain amino acid sequence at least 90% identical to SEQ ID NO:70,
h) the first antigen-binding site comprises a heavy chain variable domain amino acid sequence at least 90% identical to SEQ ID NO:77 and a light chain variable domain amino acid sequence at least 90% identical to SEQ ID NO:78,
i) the first antigen-binding site comprises a heavy chain variable domain amino acid sequence at least 90% identical to SEQ ID NO:93 and a light chain variable domain amino acid sequence at least 90% identical to SEQ ID NO:94,
j) the first antigen-binding site comprises a heavy chain variable domain amino acid sequence at least 90% identical to SEQ ID NO: 101 and a light chain variable domain amino acid sequence at least 90% identical to SEQ ID NO: 102, or
k) the first antigen-binding site comprises a heavy chain variable domain amino acid sequence at least 90% identical to SEQ ID NO: 103 and a light chain variable domain amino acid sequence at least 90% identical to SEQ ID NO: 104.

17. The protein of any one of claims 1-3, wherein the first antigen-binding site comprises a single-domain antibody.

18. The protein of claim 17, wherein the single-domain antibody comprises a V_{H}H fragment or a V_{NAR} fragment.

19. A protein according to any one of claims 1-3, 17 or 18, wherein the second antigen-binding site comprises a heavy chain variable domain and a light chain variable domain.

20. The protein of claim 18, wherein the heavy chain variable domain and the light chain variable domain of the second antigen-binding site are present on the same polypeptide.

21. A protein according to any one of claims 1-12, wherein:
a) the second antigen-binding site binds EGFR and comprises a heavy chain variable domain comprising an amino acid sequence at least 90% identical to SEQ ID NO: 151 and a light chain variable comprising an amino acid sequence at least 90% identical to SEQ ID NO: 152,
b) the second antigen-binding site binds EGFR and comprises a heavy chain variable domain comprising an amino acid sequence at least 90% identical to SEQ ID NO: 153 and a light chain variable domain comprising an amino acid sequence at least 90% identical to SEQ ID NO:154,
c) the second antigen-binding site binds EGFR and comprises a heavy chain variable domain comprising an amino acid sequence at least 90% identical to SEQ ID NO: 155 and a light chain variable domain comprising an amino acid sequence at least 90% identical to SEQ ID NO:156,
d) the second antigen-binding site binds EGFR and comprises a heavy chain variable domain comprising an amino acid sequence at least 90% identical to SEQ ID NO: 157 and a light chain variable domain comprising an amino acid sequence at least 90% identical to SEQ ID NO:158,
e) the second antigen-binding site binds EGFR and comprises a heavy chain variable domain comprising an amino acid sequence at least 90% identical to SEQ ID NO: 159 and a light chain variable domain comprising an amino acid sequence at least 90% identical to SEQ ID NO:160,
f) the second antigen-binding site binds EGFR and comprises a heavy chain variable domain comprising an amino acid sequence at least 90% identical to SEQ ID NO: 161 and a light chain variable domain comprising an amino acid sequence at least 90% identical to SEQ ID NO:162, or
g) the second antigen-binding site binds EGFR and comprises a heavy chain variable domain comprising an amino acid sequence at least 90% identical to SEQ ID NO: 163 and a light chain variable domain comprising an amino acid sequence at least 90% identical to SEQ ID NO:164.

22. A protein according to any one of claims 1-6 and 15-20, wherein:
a) the second antigen-binding site binds PD-L1 and comprises a heavy chain variable domain comprising an amino acid sequence at least 90% identical to SEQ ID NO: 167 and a light chain variable domain comprising an amino acid sequence at least 90% identical to SEQ ID NO:171,
b) the second antigen-binding site binds PD-L1 and comprises a heavy chain variable domain comprising an amino acid sequence at least 90% identical to SEQ ID NO: 175 and a light chain variable domain comprising an amino acid sequence at least 90% identical to SEQ ID NO:179,
c) the second antigen-binding site binds PD-L1 and comprises a heavy chain variable domain comprising an amino acid sequence at least 90% identical to SEQ ID NO: 183 and a light chain variable domain comprising an amino acid sequence at least 90% identical to SEQ ID NO:187,
d) the second antigen-binding site binds CCR4 and comprises a heavy chain variable domain comprising an amino acid sequence at least 90% identical to SEQ ID NO: 192 and a light chain variable domain comprising an amino acid sequence at least 90% identical to SEQ ID NO:196,
e) the second antigen-binding site binds CCR4 and comprises a heavy chain variable domain comprising an amino acid sequence at least 90% identical to SEQ ID NO:200 and a light chain variable domain comprising an amino acid sequence at least 90% identical to SEQ ID NO:204, or
f) the second antigen-binding site binds CCR4 and comprises a heavy chain variable domain of the comprising an amino acid sequence at least 90% identical to SEQ ID NO:208 and a light chain variable domain comprising an amino acid sequence at least 90% identical to SEQ ID NO:212.

23. A protein according to any one of claims 1-6, wherein the second antigen-binding site comprises a single-domain antibody, optionally wherein the single-domain antibody of the second antigen-binding site comprises a V_{H}H fragment or a V_{NAR} fragment.

24. A protein according to any one of claims 1-23, wherein the antibody Fc domain comprises a hinge and a CH2 domain, optionally wherein the antibody Fc domain comprises a hinge and a CH2 domain of a human IgG1 antibody.

25. The protein of claim 24, wherein the Fc domain comprises an amino acid sequence at least 90% identical to amino acids 234-332 of a human IgG1 antibody, optionally wherein the Fc domain comprises an amino acid sequence at least 90% identical to the Fc domain of human IgG1 and differs at one or more positions selected from the group consisting of: Q347, Y349, L351, S354, E356, E357, K360, Q362, S364, T366, L368, K370, N390, K392, T394, D399, S400, D401, F405, Y407, K409, T411, and K439.

26. A protein according to any one of claims 1-25, wherein the protein binds to NKG2D with a K_{D} of 10 nM or weaker affinity.

27. A formulation comprising a protein according to any one of the preceding claims and a pharmaceutically acceptable carrier.

28. A cell comprising one or more nucleic acids encoding a protein according to any one of claims 1-26.

29. A protein according to any one of claims 1-26 for use in a method of enhancing tumor cell death, the method comprising exposing a tumor cell and a natural killer cell to an effective amount of the protein, wherein the tumor cell expresses at least one of EGFR, CCR4, or PD-L1.

30. A protein according to any one of claims 1-26, or the formulation according to claim 27, for use in a method of treating cancer, wherein the method comprises administering an effective amount of the protein or the formulation to a patient.

31. The protein or the formulation for the use of claim 30, wherein:
a) the second antigen binding site of the protein binds EGFR, and the cancer is selected from the group consisting of head and neck cancer, colorectal cancer, non-small cell lung cancer, glioma, renal cell carcinoma, bladder cancer, cervical cancer, ovarian cancer, pancreatic cancer, and liver cancer,
b) the second antigen binding site of the protein binds PD-L1, and the cancer is selected from the group consisting of lymphoma, leukemia, multiple myeloma, head and neck cancer, bladder cancer, cervical cancer, lung cancer, renal cancer, melanoma, colorectal cancer, ovarian cancer, glioblastoma, a sarcoma, and gastric cancer, or
c) the second antigen binding site of the protein binds CCR4, and the cancer is selected from the group consisting of adult T-cell lymphoma/leukemia, peripheral T cell lymphoma, cutaneous T cell lymphoma, chronic lymphocytic leukemia, a B cell malignancy, non-Hodgkin's lymphoma, Hodgkin's lymphoma, anaplastic large cell lymphoma, mature T/natural killer (NK) cell neoplasms, thymoma, gastric cancer, and renal cell carcinoma.
